# EUROPEAN PATENT APPLICATION

(11) **EP 4 810 026 A1**
(43) Date of publication of application: **23.09.2026**
(21) Application number: 26165599.7
(22) Date of filing: 17.03.2026
(51) Int. Cl.: A61K 9/20, A61K 38/26, A61K 47/28, A61P 3/10, A61K 47/14

(54) **PHARMACEUTICAL COMPOSITIONS COMPRISING BILE ACID COMPOUNDS FOR ORAL DELIVERY OF THERAPEUTIC PEPTIDES**

(30) Priority: 18.03.2025 WO PCT/CN2025/083058
(71) Applicant: Waterstone Pharmaceuticals (Wuhan) Co., Ltd., Wuhan, Hubei 430074 (CN)
(72) Inventor: HU, Yimin, Wuhan, Hubei, 430074 (CN); WANG, Xiaolong, Wuhan, Hubei, 430074 (CN); JIN, Jing, Wuhan, Hubei, 430074 (CN); JIA, Tian, Wuhan, Hubei, 430074 (CN); ZHOU, Yuan, Wuhan, Hubei, 430074 (CN); YE, Zelin, Wuhan, Hubei, 430074 (CN); HU, Minglong, Wuhan, Hubei, 430074 (CN); ZHANG, Faming, Wuhan, Hubei, 430074 (CN)
(74) Representative: Maiwald GmbH

(57) **Abstract**

Provided herein are oral pharmaceutical compositions comprising a therapeutic polypeptide, a bile acid compound, e.g., a compound of Formula (I), a CYP450 inhibitor, and one or more pharmaceutically acceptable excipients. Also provided herein are methods of their therapeutic use.

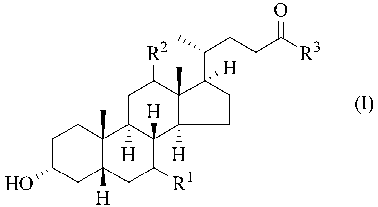

## Description

### FIELD

Provided herein are oral pharmaceutical compositions comprising a therapeutic polypeptide, a bile acid compound, a CYP450 inhibitor, and one or more pharmaceutically acceptable excipients. Also provided herein are methods of their therapeutic use.

### BACKGROUND

Therapeutic peptides offer several advantages over small molecule drugs, including remarkable potency and selectivity as well as low toxicity. Fosgerau and Hoffmann, Drug Discov. Today 2015, 20, 122-8; Lau and Dunn, Bioorg. Med. Chem. 2018, 26, 2700-7; Muttenthaler et al., Nat. Rev. Drug Discov. 2021, 20, 309-25; Zhu et al., Acta Pharm. Sin. B. 2021, 11, 2416-48; Wang et al., Signal Transduct. Target. Ther. 2022, 7, 48. However, their physicochemical properties and gastrointestinal tract (GIT) barriers, such as enzymatic degradation and low permeability, significantly hinder oral delivery. Verma et al., Drug Dev. Res. 2021, 82, 927-44; Wang et al., Signal Transduct. Target. Ther. 2022, 7, 48. As a result, most therapeutic peptides are delivered by injection, which comes with several drawbacks, including poor patient adherence, administration challenges, and risk of infection. Ganesh et al., Med. Drug Discov. 2021, 9, 100079; Muttenthaler et al., Nat. Rev. Drug Discov. 2021, 20, 309-25; Zhu et al., Acta Pharm. Sin. B. 2021, 11, 2416-48; Wang et al., Signal Transduct. Target. Ther. 2022, 7, 48. Developing effective oral delivery systems for therapeutic peptides remains a longstanding challenge due to their biological and physicochemical limitations. Ganesh et al., Med. Drug Discov. 2021, 9, 100079; Muttenthaler et al., Nat. Rev. Drug Discov. 2021, 20, 309-25; Chen et al., Theranostics 2022, 12, 1419-39. Hence, there is an urgent need for a pharmaceutical formulation capable of orally delivering a therapeutic peptide effectively. Dubey et al., Drug Discov. Today 2021, 26, 931-50.

### SUMMARY OF THE DISCLOSURE

Provided herein is an oral pharmaceutical composition comprising a therapeutic polypeptide, a bile acid compound, a CYP450 inhibitor, and one or more pharmaceutically acceptable excipients.

Also provided herein is an oral pharmaceutical composition comprising a therapeutic polypeptide, in one embodiment, a glucagon-like peptide-1 receptor agonist (GLP-1RA), a bile acid compound, a CYP450 inhibitor, and one or more pharmaceutically acceptable excipients; wherein the bile acid compound is a compound having the structure of Formula (I): 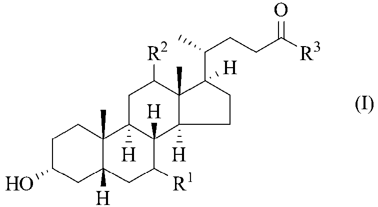 or an enantiomer, a mixture of enantiomers, a diastereomer, a mixture of two or more diastereomers, a tautomer, a mixture of two or more tautomers thereof; or a pharmaceutically acceptable salt, solvate, or hydrate thereof; wherein:
R¹ and R² are each independently hydrogen or -OH;
R³ is -N(H)-X-Z-R⁴, heterocyclyl, or -OH;
R⁴ is C₁₋₆ alkyl, C₁₋₆ heteroalkyl, C₇₋₁₅ aralkyl, heterocyclyl, -OH, or -N(H)R^{4a};
R^{4a} is C₁₋₆ alkyl, C₁₋₆ heteroalkyl, or C₇₋₁₅ aralkyl;
X is a bond, C₁₋₆ alkylene, or C₁₋₆ heteroalkylene; and
Z is a bond, -C(O)-, or -S(O₂)-;
wherein each alkyl, alkylene, heteroalkyl, heteroalkylene, aralkyl, and heterocyclyl is optionally substituted with one or more, in one embodiment, one, two, three, or four, substituents Q, wherein each Q is independently selected from: (a) deuterium, cyano, halo, imino, nitro, and oxo; (b) C₁₋₆ alkyl, C₁₋₆ heteroalkyl, C₂₋₆ alkenyl, C₂₋₆ alkynyl, C₃₋₁₀ cycloalkyl, C₆₋₁₄ aryl, C₇₋₁₅ aralkyl, heteroaryl, and heterocyclyl, each of which is further optionally substituted with one or more, in one embodiment, one, two, three, or four, substituents Q^{a}; and (c) -C(O)R^{a}, -C(O)OR^{a}, -C(O)NR^{b}R^{c}, -C(O)SR^{a}, -C(NR^{a})NR^{b}R^{c}, -C(S)R^{a}, -C(S)OR^{a}, -C(S)NR^{b}R^{c}, -OR^{a}, -OC(O)R^{a}, -OC(O)OR^{a}, -OC(O)NR^{b}R^{c}, -OC(O)SR^{a}, -OC(NR^{a})NR^{b}R^{c}, -OC(S)R^{a}, -OC(S)OR^{a}, -OC(S)NR^{b}R^{c}, -OS(O)R^{a}, -OS(O)₂R^{a}, -OS(O)NR^{b}R^{c}, -OS(O)₂NR^{b}R^{c}, -NR^{b}R^{c}, -NR^{a}C(O)R^{d}, -NR^{a}C(O)OR^{d}, -NR^{a}C(O)NR^{b}R^{c}, -NR^{a}C(O)SR^{d}, -NR^{a}C(NR^{d})NR^{b}R^{c}, -NR^{a}C(S)R^{d}, -NR^{a}C(S)OR^{d}, -NR^{a}C(S)NR^{b}R^{c}, -NR^{a}S(O)R^{d}, -NR^{a}S(O)₂R^{d}, -NR^{a}S(O)NR^{b}R^{c}, -NR^{a}S(O)₂NR^{b}R^{c}, -SR^{a}, -S(O)R^{a}, -S(O)₂R^{a}, -S(O)NR^{b}R^{c}, and -S(O)₂NR^{b}R^{c}, wherein each R^{a}, R^{b}, R^{c}, and R^{d} is independently (i) hydrogen or deuterium; (ii) C₁₋₆ alkyl, C₁₋₆ heteroalkyl, C₂₋₆ alkenyl, C₂₋₆ alkynyl, C₃₋₁₀ cycloalkyl, C₆₋₁₄ aryl, C₇₋₁₅ aralkyl, heteroaryl, or heterocyclyl, each of which is optionally substituted with one or more, in one embodiment, one, two, three, or four, substituents Q^{a}; or (iii) R^{b} and R^{c} together with the N atom to which they are attached form heterocyclyl, optionally substituted with one or more, in one embodiment, one, two, three, or four, substituents Q^{a};
wherein each Q^{a} is independently selected from: (a) deuterium, cyano, halo, nitro, imino, and oxo; (b) C₁₋₆ alkyl, C₂₋₆ alkenyl, C₂₋₆ alkynyl, C₃₋₁₀ cycloalkyl, C₆₋₁₄ aryl, C₇₋₁₅ aralkyl, heteroaryl, and heterocyclyl; and (c) -C(O)R^{e}, -C(O)OR^{e}, -C(O)NR^{f}R^{g}, -C(O)SR^{e}, -C(NR^{e})NR^{f}R^{g}, -C(S)R^{e}, -C(S)OR^{e}, -C(S)NR^{f}R^{g}, -OR^{e}, -OC(O)R^{e}, -OC(O)OR^{e}, -OC(O)NR^{f}R^{g}, -OC(O)SR^{e}, -OC(NR^{e})NR^{f}R^{g}, -OC(S)R^{e}, -OC(S)OR^{e}, -OC(S)NR^{f}R^{g}, -OS(O)R^{e}, -OS(O)₂R^{e}, -OS(O)NR^{f}R^{g}, -OS(O)₂NR^{f}R^{g}, -NR^{f}R^{g}, -NR^{e}C(O)R^{h}, -NR^{e}C(O)OR^{f}, -NR^{e}C(O)NR^{f}R^{g}, -NR^{e}C(O)SR^{f}, -NR^{e}C(NR^{h})NR^{f}R^{g}, -NR^{e}C(S)R^{h}, -NR^{e}C(S)OR^{f}, -NR^{e}C(S)NR^{f}R^{g}, -NR^{e}S(O)R^{h}, -NR^{e}S(O)₂R^{h}, -NR^{e}S(O)NR^{f}R^{g}, -NR^{e}S(O)₂NR^{f}R^{g}, -SR^{e}, -S(O)R^{e}, -S(O)₂R^{e}, -S(O)NR^{f}R^{g}, and -S(O)₂NR^{f}R^{g}; wherein each R^{e}, R^{f}, R^{g}, and R^{h} is independently (i) hydrogen or deuterium; (ii) C₁₋₆ alkyl, C₂₋₆ alkenyl, C₂₋₆ alkynyl, C₃₋₁₀ cycloalkyl, C₆₋₁₄ aryl, C₇₋₁₅ aralkyl, heteroaryl, or heterocyclyl; or (iii) R^{f} and R^{g} together with the N atom to which they are attached form heterocyclyl.

Additionally provided herein is a method of use in treating diabetes in a subject, comprising administering to the subject in need thereof a therapeutically effective amount of an oral pharmaceutical composition provided herein.

Furthermore, provided herein is a method of use in treating obesity in a subject, comprising administering to the subject in need thereof a therapeutically effective amount of an oral pharmaceutical composition provided herein.

### DETAILED DESCRIPTION

To facilitate understanding of the disclosure set forth herein, a number of terms are defined below.

Generally, the nomenclature used herein and the laboratory procedures in organic chemistry, medicinal chemistry, biochemistry, biology, and pharmacology described herein are those well-known and commonly employed in the art. Unless defined otherwise, all technical and scientific terms used herein generally have the same meaning as commonly understood by one of ordinary skill in the art to which this disclosure belongs.

The term "subject" refers to an animal, including, but not limited to, a primate (*e.g*., human), cow, pig, sheep, goat, horse, dog, cat, rabbit, rat, or mouse. The terms "subject" and "patient" are used interchangeably herein in reference, for example, to a mammalian subject, such as a human subject. In one embodiment, the subject is a human.

The terms "treat," "treating," and "treatment" are meant to include alleviating or abrogating a disorder, disease, or condition, or one or more of the symptoms associated with the disorder, disease, or condition; or alleviating or eradicating the cause(s) of the disorder, disease, or condition itself.

The term "therapeutically effective amount" or "effective amount" is meant to include the amount of a compound that, when administered, is sufficient to alleviate to some extent, one or more of the symptoms of the disorder, disease, or condition being treated.

The term "pharmaceutically acceptable carrier," "pharmaceutically acceptable excipient," "physiologically acceptable carrier," or "physiologically acceptable excipient" refers to a pharmaceutically acceptable material, composition, or vehicle, such as a liquid or solid filler, diluent, solvent, or encapsulating material. In one embodiment, each component is "pharmaceutically acceptable" in the sense of being compatible with the other ingredients of a pharmaceutical formulation, and suitable for use in contact with the tissue or organ of a subject (*e.g.*, a human) without excessive toxicity, irritation, allergic response, immunogenicity, or other problems or complications, and commensurate with a reasonable benefit/risk ratio. *See*, *e.g.*, Remington: The Science and Practice of Pharmacy, 23rd ed.; Adejare Ed.; Academic Press, 2020; Handbook of Pharmaceutical Excipients, 9th ed.; Sheskey et al., Eds.; Pharmaceutical Press, 2020; Handbook of Pharmaceutical Additives, 3rd ed.; Ash and Ash Eds.; Synapse Information Resources, 2007; Pharmaceutical Preformulation and Formulation, 2nd ed.; Gibson Ed.; CRC Press, 2009.

The term "about" or "approximately" means an acceptable error for a particular value as determined by one of ordinary skill in the art, which depends in part on how the value is measured or determined. In certain embodiments, the term "about" or "approximately" means within 1, 2, or 3 standard deviations. In certain embodiments, the term "about" or "approximately" means within 25%, 20%, 15%, 10%, 9%, 8%, 7%, 6%, 5%, 4%, 3%, 2%, 1%, 0.5%, or 0.05% of a given value or range.

The term "lipidated peptide" refers to a peptide molecule that has a lipid moiety (*e.g.*, a fatty acid chain) attached to it.

The term "alkyl" refers to a linear or branched saturated monovalent hydrocarbon radical, wherein the alkyl is optionally substituted with one or more substituents Q as described herein. For example, C₁₋₆ alkyl refers to a linear saturated monovalent hydrocarbon radical of 1 to 6 carbon atoms or a branched saturated monovalent hydrocarbon radical of 3 to 6 carbon atoms. In certain embodiments, the alkyl is a linear saturated monovalent hydrocarbon radical that has 1 to 20 (C₁₋₂₀), 1 to 15 (C₁₋₁₅), 1 to 10 (C₁₋₁₀), or 1 to 6 (C₁₋₆) carbon atoms, or branched saturated monovalent hydrocarbon radical of 3 to 20 (C₃₋₂₀), 3 to 15 (C₃₋₁₅), 3 to 10 (C₃₋₁₀), or 3 to 6 (C₃₋₆) carbon atoms. As used herein, linear C₁₋₆ and branched C₃₋₆ alkyl groups are also referred as "lower alkyl." Examples of alkyl groups include, but are not limited to, methyl, ethyl, propyl (including all isomeric forms, *e.g., n*-propyl and isopropyl), butyl (including all isomeric forms, *e.g*., *n*-butyl, isobutyl, *sec*-butyl, and *t*-butyl), pentyl (including all isomeric forms, *e.g*., *n*-pentyl, isopentyl, *sec*-pentyl, neopentyl, and *tert*-pentyl), and hexyl (including all isomeric forms, *e.g*., *n*-hexyl, isohexyl, and *sec*-hexyl).

The terms "alkylene" and "alkanediyl" are used interchangeably herein in reference to a linear or branched saturated divalent hydrocarbon radical, wherein the alkanediyl is optionally substituted with one or more substituents Q as described herein. For example, C₁₋₆ alkanediyl refers to a linear saturated divalent hydrocarbon radical of 1 to 6 carbon atoms or a branched saturated divalent hydrocarbon radical of 3 to 6 carbon atoms. In certain embodiments, the alkanediyl is a linear saturated divalent hydrocarbon radical that has 1 to 30 (C₁₋₃₀), 1 to 20 (C₁₋₂₀), 1 to 15 (C₁₋₁₅), 1 to 10 (C₁₋₁₀), or 1 to 6 (C₁₋₆) carbon atoms, or branched saturated divalent hydrocarbon radical of 3 to 30 (C₃₋₃₀), 3 to 20 (C₃₋₂₀), 3 to 15 (C₃₋₁₅), 3 to 10 (C₃₋₁₀), or 3 to 6 (C₃₋₆) carbon atoms. As used herein, linear C₁₋₆ and branched C₃₋₆ alkanediyl groups are also referred as "lower alkanediyl." Examples of alkanediyl groups include, but are not limited to, methanediyl, ethanediyl (including all isomeric forms, *e.g*., ethane-1,1-diyl and ethane-1,2-diyl), propanediyl (including all isomeric forms, *e.g*., propane-1,1-diyl, propane-1,2-diyl, and propane-1,3-diyl), butanediyl (including all isomeric forms, *e.g*., butane-1,1-diyl, butane-1,2-diyl, butane-1,3-diyl, and butane-1,4-diyl), pentanediyl (including all isomeric forms, *e.g.*, pentane-1,1-diyl, pentane-1,2-diyl, pentane-1,3-diyl, and pentane-1,5-diyl), and hexanediyl (including all isomeric forms, *e.g.*, hexane-1,1-diyl, hexane-1,2-diyl, hexane-1,3-diyl, and hexane-1,6-diyl). Examples of substituted alkanediyl groups include, but are not limited to, -C(O)CH₂-, -C(O)(CH₂)₂-, -C(O)(CH₂)₃-, -C(O)(CH₂)₄-, -C(O)(CH₂)₅-, -C(O)(CH₂)₆-, -C(O)(CH₂)₇-, -C(O)(CH₂)₈-, -C(O)(CH₂)₉-, -C(O)(CH₂)₁₀-, -C(O)CH₂C(O)-, -C(O)(CH₂)₂C(O)-, -C(O)(CH₂)₃C(O)-, -C(O)(CH₂)₄C(O)-, or -C(O)(CH₂)₅C(O)-.

The term "heteroalkyl" refers to a linear or branched saturated monovalent hydrocarbon radical that contains one or more heteroatoms on its main chain, each independently selected from O, S, and N. The heteroalkyl is optionally substituted with one or more substituents Q as described herein. For example, C₁₋₆ heteroalkyl refers to a linear saturated monovalent hydrocarbon radical of 1 to 6 carbon atoms or a branched saturated monovalent hydrocarbon radical of 3 to 6 carbon atoms. In certain embodiments, the heteroalkyl is a linear saturated monovalent hydrocarbon radical that has 1 to 20 (C₁₋₂₀), 1 to 15 (C₁₋₁₅), 1 to 10 (C₁₋₁₀), or 1 to 6 (C₁₋₆) carbon atoms, or branched saturated monovalent hydrocarbon radical of 3 to 20 (C₃₋₂₀), 3 to 15 (C₃₋₁₅), 3 to 10 (C₃₋₁₀), or 3 to 6 (C₃₋₆) carbon atoms. As used herein, linear C₁₋₆ and branched C₃₋₆ heteroalkyl groups are also referred as "lower heteroalkyl." Examples of heteroalkyl groups include, but are not limited to, -OCH₃, -OCH₂CH₃, -CH₂OCH₃, -NHCH₃, -ONHCH₃, -NHOCH₃, -SCH₃, -CH₂NHCH₂CH₃, and -NHCH₂CH₂CH₃. Examples of substituted heteroalkyl groups include, but are not limited to, -CH₂NHC(O)CH₃ and -NHC(O)CH₂CH₃.

The terms "heteroalkylene" and "heteroalkanediyl" are used interchangeably herein in reference to a linear or branched saturated divalent hydrocarbon radical that contains one or more heteroatoms in its main chain, each independently selected from O, S, and N. The heteroalkylene is optionally substituted with one or more substituents Q as described herein. For example, C₁₋₆ heteroalkylene refers to a linear saturated divalent hydrocarbon radical of 1 to 6 carbon atoms or a branched saturated divalent hydrocarbon radical of 3 to 6 carbon atoms. In certain embodiments, the heteroalkylene is a linear saturated divalent hydrocarbon radical that has 1 to 20 (C₁₋₂₀), 1 to 15 (C₁₋₁₅), 1 to 10 (C₁₋₁₀), or 1 to 6 (C₁₋₆) carbon atoms, or branched saturated divalent hydrocarbon radical of 3 to 20 (C₃₋₂₀), 3 to 15 (C₃₋₁₅), 3 to 10 (C₃₋₁₀), or 3 to 6 (C₃₋₆) carbon atoms. As used herein, linear C₁₋₆ and branched C₃₋₆ heteroalkylene groups are also referred as "lower heteroalkylene." Examples of heteroalkylene groups include, but are not limited to, -CH₂O-, -CH₂CH₂O-, -CH₂CH₂CH₂O-, -(CH₂)₄O-, -(CH₂)₅O-, -(CH₂)₆O-, -(CH₂)₇O-, -(CH₂)₈O-, -(CH₂)₉O-, -(CH₂)₁₀O-, -CH₂OCH₂-, -CH₂CH₂O-, -(CH₂CH₂O)₂-, -(CH₂CH₂O)₃-, -(CH₂CH₂O)₄-, -(CH₂CH₂O)₅-, -CH₂NH-, -CH₂NHCH₂-, -CH₂CH₂NH-, - CH₂CH₂CH₂NH-, -(CH₂)₄NH-, -CH₂S-, -CH₂SCH₂-, and -CH₂CH₂S-. Examples of substituted heteroalkylene groups include, but are not limited to, -C(O)CH₂O-, -C(O)(CH₂)₂O-, -C(O)CH₂CH₂CH₂O-, -C(O)CH₂CH₂CH₂CH₂O-, -C(O)(CH₂)₅O-, -C(O)(CH₂)₆O-, -C(O)(CH₂)₇O-, -C(O)(CH₂)₈O-, -C(O)(CH₂)₉O-, -C(O)(CH₂)₁₀O-, -C(O)CH₂OCH₂CH₂O-, -C(O)CH₂O(CH₂CH₂O)₂-, -C(O)CH₂O(CH₂CH₂O)₃-, -C(O)CH₂O(CH₂CH₂O)₄, -C(O)CH₂O(CH₂CH₂O)₅-, -CH₂NHC(O)CH₂-, -CH₂CH₂C(O)NH-, -CH₂N(CH₃)-, -(CH₂)₂N(CH₃)-, -(CH₂)₃N(CH₃)-, or -(CH₂)₄N(CH₃)-.

The term "alkenyl" refers to a linear or branched monovalent hydrocarbon radical, which contains one or more, in one embodiment, one, two, three, or four, in another embodiment, one, carbon-carbon double bond(s). The alkenyl is optionally substituted with one or more substituents Q as described herein. The term "alkenyl" embraces radicals having a "*cis*" or "*trans*" configuration or a mixture thereof, or alternatively, a "*Z*" or "*E*" configuration or a mixture thereof, as appreciated by those of ordinary skill in the art. For example, C₂₋₆ alkenyl refers to a linear unsaturated monovalent hydrocarbon radical of 2 to 6 carbon atoms or a branched unsaturated monovalent hydrocarbon radical of 3 to 6 carbon atoms. In certain embodiments, the alkenyl is a linear monovalent hydrocarbon radical of 2 to 20 (C₂₋₂₀), 2 to 15 (C₂₋₁₅), 2 to 10 (C₂₋₁₀), or 2 to 6 (C₂₋₆) carbon atoms, or a branched monovalent hydrocarbon radical of 3 to 20 (C₃₋₂₀), 3 to 15 (C₃₋₁₅), 3 to 10 (C₃₋₁₀), or 3 to 6 (C₃₋₆) carbon atoms. Examples of alkenyl groups include, but are not limited to, ethenyl, propenyl (including all isomeric forms, *e.g.,* propen-1-yl, propen-2-yl, and allyl), and butenyl (including all isomeric forms, *e.g.,* buten-1-yl, buten-2-yl, buten-3-yl, and 2-buten-1-yl).

The term "alkynyl" refers to a linear or branched monovalent hydrocarbon radical, which contains one or more, in one embodiment, one, two, three, or four, in another embodiment, one, carbon-carbon triple bond(s). An alkynyl group does not contain a carbon-carbon double bond. The alkynyl is optionally substituted with one or more substituents Q as described herein. For example, C₂₋₆ alkynyl refers to a linear unsaturated monovalent hydrocarbon radical of 2 to 6 carbon atoms or a branched unsaturated monovalent hydrocarbon radical of 4 to 6 carbon atoms. In certain embodiments, the alkynyl is a linear monovalent hydrocarbon radical of 2 to 20 (C₂₋₂₀), 2 to 15 (C₂₋₁₅), 2 to 10 (C₂₋₁₀), or 2 to 6 (C₂₋₆) carbon atoms, or a branched monovalent hydrocarbon radical of 4 to 20 (C₄₋₂₀), 4 to 15 (C₄₋₁₅), 4 to 10 (C₄₋₁₀), or 4 to 6 (C₄₋₆) carbon atoms. Examples of alkynyl groups include, but are not limited to, ethynyl (-C≡CH), propynyl (including all isomeric forms, *e.g.*, 1-propynyl (-C≡CCH₃) and propargyl (-CH₂C≡CH)), butynyl (including all isomeric forms, *e.g*., 1-butyn-1-yl and 2-butyn-1-yl), pentynyl (including all isomeric forms, *e.g*., 1-pentyn-1-yl and 1-methyl-2-butyn-1-yl), and hexynyl (including all isomeric forms, *e.g*., 1-hexyn-1-yl and 2-hexyn-1-yl).

The term "cycloalkyl" refers to a cyclic monovalent hydrocarbon radical, which is optionally substituted with one or more substituents Q as described herein. In one embodiment, the cycloalkyl is a saturated or unsaturated but non-aromatic, and/or bridged or non-bridged, and/or fused bicyclic group. In certain embodiments, the cycloalkyl has from 3 to 20 (C₃₋₂₀), from 3 to 15 (C₃₋₁₅), from 3 to 10 (C₃₋₁₀), or from 3 to 7 (C₃₋₇) carbon atoms. In one embodiment, the cycloalkyl is monocyclic. In another embodiment, the cycloalkyl is bicyclic. In yet another embodiment, the cycloalkyl is tricyclic. In still another embodiment, the cycloalkyl is polycyclic. Examples of cycloalkyl groups include, but are not limited to, cyclopropyl, cyclobutyl, cyclopentyl, cyclopentenyl, cyclohexyl, cyclohexenyl, cyclohexadienyl, cycloheptyl, cyclo-heptenyl, bicyclo[1.1.1]pentyl, bicyclo[2.1.1]hexyl, bicyclo[2.2.1]heptyl, bicyclo[2.2.2]octyl, decalinyl, and adamantyl.

The term "aryl" refers to a monovalent monocyclic aromatic hydrocarbon radical and/or monovalent polycyclic aromatic hydrocarbon radical that contain at least one aromatic carbon ring. In certain embodiments, the aryl has from 6 to 20 (C₆₋₂₀), from 6 to 15 (C₆₋₁₅), or from 6 to 10 (C₆₋₁₀) ring carbon atoms. Examples of aryl groups include, but are not limited to, phenyl, naphthyl, fluorenyl, azulenyl, anthryl, phenanthryl, pyrenyl, biphenyl, and terphenyl. The aryl also refers to bicyclic or tricyclic carbon rings, where one of the rings is aromatic and the others of which may be saturated, partially unsaturated, or aromatic, for example, dihydronaphthyl, indenyl, indanyl, or tetrahydronaphthyl (tetralinyl). In one embodiment, the aryl is monocyclic. In another embodiment, the aryl is bicyclic. In yet another embodiment, the aryl is tricyclic. In still another embodiment, the aryl is polycyclic. In certain embodiments, the aryl is optionally substituted with one or more substituents Q as described herein.

The term "aralkyl" or "arylalkyl" refers to a monovalent alkyl group substituted with one or more aryl groups. In certain embodiments, the aralkyl has from 7 to 30 (C₇₋₃₀), from 7 to 20 (C₇₋₂₀), or from 7 to 16 (C₇₋₁₆) carbon atoms. Examples of aralkyl groups include, but are not limited to, benzyl, phenylethyl (including all isomeric forms, *e.g.*, 1-phenylethyl and 2-phenylethyl), and phenylpropyl (including all isomeric forms, *e.g.*, 1-phenylpropyl, 2-phenylpropyl, and 3-phenylpropyl). In certain embodiments, the aralkyl is optionally substituted with one or more substituents Q as described herein.

The term "heteroaryl" refers to a monovalent monocyclic aromatic group or monovalent polycyclic aromatic group that contain at least one aromatic ring, wherein at least one aromatic ring contains one or more heteroatoms, each independently selected from O, S, and N, in the ring. For a heteroaryl group containing a heteroaromatic ring and a nonaromatic heterocyclic ring, the heteroaryl group is not bonded to the rest of a molecule through its nonaromatic heterocyclic ring. Each ring of a heteroaryl group can contain one or two O atoms, one or two S atoms, and/or one to four N atoms; provided that the total number of heteroatoms in each ring is four or less and each ring contains at least one carbon atom. In certain embodiments, the heteroaryl has from 5 to 20, from 5 to 15, or from 5 to 10 ring atoms. In one embodiment, the heteroaryl is monocyclic. Examples of monocyclic heteroaryl groups include, but are not limited to, furanyl, imidazolyl, isothiazolyl, isoxazolyl, oxadiazolyl, oxazolyl, pyrazinyl, pyrazolyl, pyridazinyl, pyridyl, pyrimidinyl, pyrrolyl, thiadiazolyl, thiazolyl, thienyl, tetrazolyl, triazinyl, and triazolyl. In another embodiment, the heteroaryl is bicyclic. Examples of bicyclic heteroaryl groups include, but are not limited to, benzofuranyl, benzimidazolyl, benzoisoxazolyl, benzopyranyl, benzothiadiazolyl, benzothiazolyl, benzothienyl, benzotriazolyl, benzoxazolyl, furopyrindyl (including all isomeric forms, *e.g*., furo[2,3-*b*]pyridinyl, furo[2,3-*c*]pyridinyl, furo[3,2-*b*]pyridinyl, furo[3,2-*c*]pyridinyl, furo[3,4-*b*]pyridinyl, and furo[3,4-*c*]pyridinyl), imidazopyridinyl (including all isomeric forms, *e.g*., imidazo[1,2-*a*]pyridinyl, imidazo[4,5-*b*]pyridinyl, and imidazo[4,5-*c*]pyridinyl), imidazothiazolyl (including all isomeric forms, *e.g*., imidazo[2,1-*b*]thiazolyl and imidazo[4,5-*d*]thiazolyl), indazolyl, indolizinyl, indolyl, isobenzofuranyl, isobenzothienyl (*i.e.*, benzo[*c*]thienyl), isoindolyl, isoquinolinyl, naphthyridinyl (including all isomeric forms, *e.g.*, 1,5-naphthyridinyl, 1,6-naphthyridinyl, 1,7-naphthyridinyl, and 1,8-naphthyridinyl), oxazolopyridinyl (including all isomeric forms, *e.g.*, oxazolo[4,5-*b*]pyridinyl, oxazolo[4,5-*c*]pyridinyl, oxazolo[5,4-*b*]pyridinyl, and oxazolo[5,4-*c*]pyridinyl), phthalazinyl, pteridinyl, purinyl, pyrrolopyridyl (including all isomeric forms, *e.g.*, pyrrolo[2,3-*b*]pyridinyl, pyrrolo[2,3-*c*]pyridinyl, pyrrolo[3,2-*b*]pyridinyl, and pyrrolo[3,2-c]pyridinyl), quinolinyl, quinoxalinyl, quinazolinyl, thiadiazolopyrimidyl (including all isomeric forms, *e.g.*, [1,2,5]thiadiazolo[3,4-*d*]pyrimidinyl and [1,2,3]thiadiazolo[4,5-*d*]pyrimidinyl), and thienopyridyl (including all isomeric forms, *e.g*., thieno[2,3-*b*]pyridinyl, thieno[2,3-*c*]pyridinyl, thieno[3,2-*b*]pyridinyl, and thieno[3,2-*c*]pyridinyl). In yet another embodiment, the heteroaryl is tricyclic. Examples of tricyclic heteroaryl groups include, but are not limited to, acridinyl, benzindolyl, carbazolyl, dibenzofuranyl, perimidinyl, phenanthrolinyl, phenanthridinyl (including all isomeric forms, *e.g.*, 1,5-phenanthrolinyl, 1,6-phenanthrolinyl, 1,7-phenanthrolinyl, 1,9-phenanthrolinyl, and 2,10-phenanthrolinyl), phenarsazinyl, phenazinyl, phenothiazinyl, phenoxazinyl, and xanthenyl. In certain embodiments, the heteroaryl is optionally substituted with one or more substituents Q as described herein.

The term "heterocyclyl" or "heterocyclic" refers to a monovalent monocyclic non-aromatic ring system or monovalent polycyclic ring system that contains at least one non-aromatic ring, wherein one or more of the non-aromatic ring atoms are heteroatoms, each independently selected from O, S, and N; and the remaining ring atoms are carbon atoms. For a heterocyclyl group containing a heteroaromatic ring and a nonaromatic heterocyclic ring, the heterocyclyl group is not bonded to the rest of a molecule through the heteroaromatic ring. In certain embodiments, the heterocyclyl or heterocyclic group has from 3 to 20, from 3 to 15, from 3 to 10, from 3 to 8, from 4 to 7, or from 5 to 6 ring atoms. In certain embodiments, the heterocyclyl is a monocyclic, bicyclic, tricyclic, or tetracyclic ring system, which may be fused or bridged, and in which nitrogen or sulfur atoms may be optionally oxidized, nitrogen atoms may be optionally quaternized, and some rings may be partially or fully saturated, or aromatic. The heterocyclyl may be attached to the main structure at any heteroatom or carbon atom which results in the creation of a stable compound. Examples of heterocyclyls and heterocyclic groups include, but are not limited to, azepinyl, benzodioxanyl, benzodioxolyl, benzofuranonyl, chromanyl, decahydroisoquinolinyl, dihydrobenzofuranyl, dihydrobenzisothiazolyl, dihydro-benzisoxazinyl (including all isomeric forms, *e.g.*, 1,4-dihydrobenzo[*d*][1,3]oxazinyl, 3,4-dihydrobenzo[*c*][1,2]-oxazinyl, and 3,4-dihydrobenzo[*d*][1,2]oxazinyl), dihydrobenzothienyl, dihydroisobenzofuranyl, dihydrobenzo[*c*]thienyl, dihydrofuryl, dihydroisoindolyl, dihydro-pyranyl, dihydropyrazolyl, dihydropyrazinyl, dihydropyridinyl, dihydropyrimidinyl, dihydro-pyrrolyl, dioxolanyl, 1,4-dithianyl, furanonyl, imidazolidinyl, imidazolinyl, indolinyl, isochromanyl, isoindolinyl, isothiazolidinyl, isoxazolidinyl, morpholinyl, octahydroindolyl, octahydroisoindolyl, oxazolidinonyl, oxazolidinyl, oxiranyl, piperazinyl, piperidinyl, 4-piperidonyl, pyrazolidinyl, pyrazolinyl, pyrrolidinyl, pyrrolinyl, quinuclidinyl, tetrahydrofuryl, tetrahydroisoquinolinyl, tetrahydropyranyl, tetrahydrothienyl, thiamorpholinyl, thiazolidinyl, thiochromanyl, tetrahydroquinolinyl, and 1,3,5-trithianyl. In certain embodiments, the heterocyclyl is optionally substituted with one or more substituents Q as described herein.

The term "halogen," "halide," or "halo" refers to fluoro, chloro, bromo, and/or iodo.

The term "optionally substituted" is intended to mean that a group or substituent, such as an alkyl, alkylene, heteroalkyl, heteroalkylene, alkenyl, alkynyl, cycloalkyl, aryl, aralkyl, heteroaryl, or heterocyclyl group, may be substituted with one or more, in one embodiment, one, two, three, or four, substituents Q, each of which is independently selected from, *e.g.,* (a) deuterium (-D), cyano (-CN), halo, imino (=NH), nitro (-NO₂), and oxo (=O); (b) C₁₋₆ alkyl, C₁₋₆ heteroalkyl, C₂₋₆ alkenyl, C₂₋₆ alkynyl, C₃₋₁₀ cycloalkyl, C₆₋₁₄ aryl, C₇₋₁₅ aralkyl, heteroaryl, and heterocyclyl, each of which is further optionally substituted with one or more, in one embodiment, one, two, three, or four, substituents Q^{a}; and (c) -C(O)R^{a}, -C(O)OR^{a}, -C(O)NR^{b}R^{c}, -C(O)SR^{a}, -C(NR^{a})NR^{b}R^{c}, -C(S)R^{a}, -C(S)OR^{a}, -C(S)NR^{b}R^{c}, -OR^{a}, -OC(O)R^{a}, -OC(O)OR^{a}, -OC(O)NR^{b}R^{c}, -OC(O)SR^{a}, -OC(NR^{a})NR^{b}R^{c}, -OC(S)R^{a}, -OC(S)OR^{a}, -OC(S)NR^{b}R^{c}, -OP(O)(OR^{b})OR^{c}, -OS(O)R^{a}, -OS(O)₂R^{a}, -OS(O)NR^{b}R^{c}, -OS(O)₂NR^{b}R^{c}, -NR^{b}R^{c}, -NR^{a}C(O)R^{d}, -NR^{a}C(O)OR^{d}, -NR^{a}C(O)NR^{b}R^{c}, -NR^{a}C(O)SR^{d}, -NR^{a}C(NR^{d})NR^{b}R^{c}, -NR^{a}C(S)R^{d}, -NR^{a}C(S)OR^{d}, -NR^{a}C(S)NR^{b}R^{c}, -NR^{a}S(O)R^{d}, -NR^{a}S(O)₂R^{d}, -NR^{a}S(O)NR^{b}R^{c}, -NR^{a}S(O)₂NR^{b}R^{c}, -SR^{a}, -S(O)R^{a}, -S(O)₂R^{a}, -S(O)NR^{b}R^{c}, and -S(O)₂NR^{b}R^{c}, wherein each R^{a}, R^{b}, R^{c}, and R^{d} is independently (i) hydrogen or deuterium; (ii) C₁₋₆ alkyl, C₁₋₆ heteroalkyl, C₂₋₆ alkenyl, C₂₋₆ alkynyl, C₃₋₁₀ cycloalkyl, C₆₋₁₄ aryl, C₇₋₁₅ aralkyl, heteroaryl, or heterocyclyl, each of which is optionally substituted with one or more, in one embodiment, one, two, three, or four, substituents Q^{a}; or (iii) R^{b} and R^{c} together with the N atom to which they are attached form heterocyclyl optionally substituted with one or more, in one embodiment, one, two, three, or four, substituents Q^{a}. As used herein, all groups that can be substituted are "optionally substituted."

In one embodiment, each Q^{a} is independently selected from: (a) deuterium, cyano, halo, imino, nitro, and oxo; (b) C₁₋₆ alkyl, C₁₋₆ heteroalkyl, C₂₋₆ alkenyl, C₂₋₆ alkynyl, C₃₋₁₀ cycloalkyl, C₆₋₁₄ aryl, C₇₋₁₅ aralkyl, heteroaryl, and heterocyclyl; and (c) -C(O)R^{e}, -C(O)OR^{e}, -C(O)NR^{f}R^{g}, -C(O)SR^{e}, -C(NR^{e})NR^{f}R^{g}, -C(S)R^{e}, -C(S)OR^{e}, -C(S)NR^{f}R^{g}, -OR^{e}, -OC(O)R^{e}, -OC(O)OR^{e}, -OC(O)NR^{f}R^{g}, -OC(O)SR^{e}, -OC(NR^{e})NR^{f}R^{g}, -OC(S)R^{e}, -OC(S)OR^{e}, -OC(S)NR^{f}R^{g}, -OP(O)(OR^{f})OR^{g}, -OS(O)R^{e}, -OS(O)₂R^{e}, -OS(O)NR^{f}R^{g}, -OS(O)₂NR^{f}R^{g}, -NR^{f}R^{g}, -NR^{e}C(O)R^{h}, -NR^{e}C(O)OR^{f}, -NR^{e}C(O)NR^{f}R^{g}, -NR^{e}C(O)SR^{f}, -NR^{e}C(NR^{h})NR^{f}R^{g}, -NR^{e}C(S)R^{h}, -NR^{e}C(S)OR^{f}, -NR^{e}C(S)NR^{f}R^{g}, -NR^{e}S(O)R^{h}, -NR^{e}S(O)₂R^{h}, -NR^{e}S(O)NR^{f}R^{g}, -NR^{e}S(O)₂NR^{f}R^{g}, -SR^{e}, -S(O)R^{e}, -S(O)₂R^{e}, -S(O)NR^{f}R^{g}, and -S(O)₂NR^{f}R^{g}; wherein each R^{e}, R^{f}, R^{g}, and R^{h} is independently (i) hydrogen or deuterium; (ii) C₁₋₆ alkyl, C₂₋₆ alkenyl, C₂₋₆ alkynyl, C₃₋₁₀ cycloalkyl, C₆₋₁₄ aryl, C₇₋₁₅ aralkyl, heteroaryl, or heterocyclyl; or (iii) R^{f} and R^{g} together with the N atom to which they are attached form heterocyclyl.

The terms "optically active," "enantiomerically active," and "enantiomerically pure" refer to a collection of molecules, which has an enantiomeric excess of no less than about 80%, no less than about 90%, no less than about 91%, no less than about 92%, no less than about 93%, no less than about 94%, no less than about 95%, no less than about 96%, no less than about 97%, no less than about 98%, no less than about 99%, no less than about 99.5%, or no less than about 99.8%. In certain embodiments, an optically active compound comprises about 95% or more of one enantiomer and about 5% or less of the other enantiomer based on the total weight of the enantiomeric mixture in question. In certain embodiments, an optically active compound comprises about 98% or more of one enantiomer and about 2% or less of the other enantiomer based on the total weight of the enantiomeric mixture in question. In certain embodiments, an optically active compound comprises about 99% or more of one enantiomer and about 1% or less of the other enantiomer based on the total weight of the enantiomeric mixture in question.

In describing an optically active compound, the prefixes R and S are used to denote the absolute configuration of the compound about its chiral center(s). The (+) and (-) are used to denote the optical rotation of the compound, that is, the direction in which a plane of polarized light is rotated by the optically active compound. The (-) prefix indicates that the compound is levorotatory, that is, the compound rotates the plane of polarized light to the left or counterclockwise. The (+) prefix indicates that the compound is dextrorotatory, that is, the compound rotates the plane of polarized light to the right or clockwise. However, the sign of optical rotation, (+) and (-), is not related to the absolute configuration of the compound, R and S.

The terms "substantially pure" and "substantially homogeneous" mean, when referred to a substance, sufficiently homogeneous to appear free of readily detectable impurities as determined by a standard analytical method used by one of ordinary skill in the art, including, but not limited to, thin layer chromatography (TLC), gel electrophoresis, high performance liquid chromatography (HPLC), gas chromatography (GC), nuclear magnetic resonance (NMR), and mass spectrometry (MS); or sufficiently pure such that further purification would not detectably alter the physical, chemical, biological, and/or pharmacological properties, such as enzymatic and biological activities, of the substance. In certain embodiments, "substantially pure" or "substantially homogeneous" refers to a collection of molecules, wherein at least about 95%, at least about 96%, at least about 97%, at least about 98%, at least about 99%, or at least about 99.5% by weight of the molecules are a single compound as determined by standard analytical methods.

The term "solvate" refers to a complex or aggregate formed by one or more molecules of a solute, *e.g.,* a compound provided herein, and one or more molecules of a solvent, which are present in a stoichiometric or non-stoichiometric amount. Suitable solvents include, but are not limited to, water, methanol, ethanol, n-propanol, isopropanol, and acetic acid. In certain embodiments, the solvent is pharmaceutically acceptable. In one embodiment, the complex or aggregate is in a crystalline form. In another embodiment, the complex or aggregate is in a noncrystalline form. Where the solvent is water, the solvate is a hydrate. Examples of hydrates include, but are not limited to, a hemihydrate, monohydrate, dihydrate, trihydrate, tetrahydrate, and pentahydrate.

For a divalent group described herein, no orientation is implied by the direction in which the divalent group is presented. For example, unless a particular orientation is specified, the formula -C(O)NH- represents both -C(O)NH- and -NHC(O)-.

The phrase "an enantiomer, a mixture of enantiomers, a diastereomer, a mixture of two or more diastereomers, a tautomer, a mixture of two or more tautomers thereof; or a pharmaceutically acceptable salt, solvate, or hydrate thereof" has the same meaning as the phrase "(i) an enantiomer, a mixture of enantiomers, a diastereomer, a mixture of two or more diastereomers, a tautomer, or a mixture of two or more tautomers of the compound referenced therein; (ii) a pharmaceutically acceptable salt, solvate, or hydrate of the compound referenced therein; or (iii) a pharmaceutically acceptable salt, solvate, or hydrate of an enantiomer, a mixture of enantiomers, a diastereomer, a mixture of two or more diastereomers, a tautomer, or a mixture of two or more tautomers of the compound referenced therein."

The phrase "a diastereomer, a mixture of two or more diastereomers, a tautomer, a mixture of two or more tautomers thereof; or a pharmaceutically acceptable salt, solvate, or hydrate thereof" has the same meaning as the phrase "(i) a diastereomer, a mixture of two or more diastereomers, a tautomer, or a mixture of two or more tautomers of the compound referenced therein; (ii) a pharmaceutically acceptable salt, solvate, or hydrate of the compound referenced therein; or (iii) a pharmaceutically acceptable salt, solvate, or hydrate of a diastereomer, a mixture of two or more diastereomers, a tautomer, or a mixture of two or more tautomers of the compound referenced therein."

### Oral Pharmaceutical Compositions

In one embodiment, provided herein is an oral pharmaceutical composition comprising a therapeutic polypeptide, a bile acid compound, a CYP450 inhibitor, and one or more pharmaceutically acceptable excipients.

In certain embodiments, a pharmaceutical composition provided herein further comprises a gastrointestinal enzyme inhibitor and/or an absorption enhancer.

In another embodiment, provided herein is an oral pharmaceutical composition comprising a therapeutic polypeptide, a bile acid compound, a CYP450 inhibitor, a gastrointestinal enzyme inhibitor, and one or more pharmaceutically acceptable excipients.

In yet another embodiment, provided herein is an oral pharmaceutical composition comprising a therapeutic polypeptide, a bile acid compound, a CYP450 inhibitor, an absorption enhancer, and one or more pharmaceutically acceptable excipients.

In still another embodiment, provided herein is an oral pharmaceutical composition comprising a therapeutic polypeptide, a bile acid compound, a CYP450 inhibitor, a gastrointestinal enzyme inhibitor, an absorption enhancer, and one or more pharmaceutically acceptable excipients.

In certain embodiments, a pharmaceutical composition provided herein comprises from about 1 to about 50%, from about 2 to about 50%, from about 5 to about 50%, or from about 5 to about 40% by weight of the one or more pharmaceutically acceptable excipients. In certain embodiments, a pharmaceutical composition provided herein comprises from about 1 to about 50% by weight of the one or more pharmaceutically acceptable excipients. In certain embodiments, a pharmaceutical composition provided herein comprises from about 2 to about 50% by weight of the one or more pharmaceutically acceptable excipients. In certain embodiments, a pharmaceutical composition provided herein comprises from about 5 to about 50% by weight of the one or more pharmaceutically acceptable excipients. In certain embodiments, a pharmaceutical composition provided herein comprises from about 5 to about 40% by weight of the one or more pharmaceutically acceptable excipients.

In certain embodiments, the one or more pharmaceutically acceptable excipients comprise a disintegrant, filler, lubricant, or glidant, or a mixture of two or more thereof. In certain embodiments, the one or more pharmaceutically acceptable excipients comprise a disintegrant. In certain embodiments, the one or more pharmaceutically acceptable excipients comprise a filler. In certain embodiments, the one or more pharmaceutically acceptable excipients comprise a lubricant. In certain embodiments, the one or more pharmaceutically acceptable excipients comprise a glidant. In certain embodiments, the one or more pharmaceutically acceptable excipients comprise a disintegrant, filler, lubricant, and glidant.

In one embodiment, provided herein is an oral pharmaceutical composition comprising a therapeutic polypeptide, a bile acid compound, a CYP450 inhibitor, a disintegrant, a filler, a lubricant, and a glidant.

In another embodiment, provided herein is an oral pharmaceutical composition comprising a therapeutic polypeptide, a bile acid compound, a CYP450 inhibitor, a gastrointestinal enzyme inhibitor, a disintegrant, a filler, a lubricant, and a glidant.

In yet another embodiment, provided herein is an oral pharmaceutical composition comprising a therapeutic polypeptide, a bile acid compound, a CYP450 inhibitor, an absorption enhancer, a disintegrant, a filler, a lubricant, and a glidant.

In still another embodiment, provided herein is an oral pharmaceutical composition comprising a therapeutic polypeptide, a bile acid compound, a CYP450 inhibitor, a gastrointestinal enzyme inhibitor, an absorption enhancer, a disintegrant, a filler, a lubricant, and a glidant.

In certain embodiments, a pharmaceutical composition provided herein comprises from about 0.1 to about 10%, from about 0.2 to about 7.5%, from about 0.2 to about 5%, from about 0.5 to about 5%, from about 1 to about 5%, or from about 1 to about 2.5% by weight of the therapeutic polypeptide. In certain embodiments, a pharmaceutical composition provided herein comprises from about 0.1 to about 10% by weight of the therapeutic polypeptide. In certain embodiments, a pharmaceutical composition provided herein comprises from about 0.2 to about 7.5% by weight of the therapeutic polypeptide. In certain embodiments, a pharmaceutical composition provided herein comprises from about 0.2 to about 5% by weight of the therapeutic polypeptide. In certain embodiments, a pharmaceutical composition provided herein comprises from about 0.5 to about 5% by weight of the therapeutic polypeptide. In certain embodiments, a pharmaceutical composition provided herein comprises from about 1 to about 5% by weight of the therapeutic polypeptide. In certain embodiments, a pharmaceutical composition provided herein comprises from about 1 to about 2.5% by weight of the therapeutic polypeptide.

In certain embodiments, a pharmaceutical composition provided herein comprises from about 0.1 to about 50 mg, from about 0.2 to about 25 mg, from about 0.5 to about 20 mg, or from about 1 to about 15 mg of the therapeutic polypeptide. In certain embodiments, a pharmaceutical composition provided herein comprises from about 0.1 to about 50 mg of the therapeutic polypeptide. In certain embodiments, a pharmaceutical composition provided herein comprises from about 0.2 to about 25 mg of the therapeutic polypeptide. In certain embodiments, a pharmaceutical composition provided herein comprises from about 0.5 to about 20 mg of the therapeutic polypeptide. In certain embodiments, a pharmaceutical composition provided herein comprises from about 1 to about 15 mg of the therapeutic polypeptide. In certain embodiments, a pharmaceutical composition provided herein comprises about 1, about 2, about 3, about 4, about 5, about 6, about 7, about 8, about 9, about 10, about 11, about 12, about 13, about 14, or about 15 mg of the therapeutic polypeptide.

In certain embodiments, the therapeutic peptide is a glucagon-like peptide-1 receptor agonist (GLP-1RA), a κ-opioid receptor (KOR) agonist, or an insulin receptor (IR) agonist. In certain embodiments, the therapeutic peptide is a GLP-1RA. In certain embodiments, the therapeutic peptide is beinaglutide, exenatide, liraglutide, lixisenatide, mazdutide, PEG-loxenatide, PEGylated exenatide, retatrutide, semaglutide, or tirzepatide. In certain embodiments, the therapeutic peptide is beinaglutide. In certain embodiments, the therapeutic peptide is exenatide. In certain embodiments, the therapeutic peptide is liraglutide. In certain embodiments, the therapeutic peptide is lixisenatide. In certain embodiments, the therapeutic peptide is mazdutide. In certain embodiments, the therapeutic peptide is PEG-loxenatide. In certain embodiments, the therapeutic peptide is PEGylated exenatide. In certain embodiments, the therapeutic peptide is retatrutide. In certain embodiments, the therapeutic peptide is semaglutide. In certain embodiments, the therapeutic peptide is tirzepatide. In certain embodiments, the therapeutic peptide is a GLP-1RA disclosed in U.S. 9,993,430 B2, the disclosure of which is incorporated herein by reference in its entirety.

In certain embodiments, the therapeutic peptide is a KOR agonist. In certain embodiments, the therapeutic peptide is CR665 ((2*R*)-2-[[(2*R*)-2-[[(2*R*)-2-amino-3-phenylpropanoyl]amino]-3-phenylpropanoyl]amino]-*N*-[(2*R*)-5-(diaminomethylideneamino)-1-oxo-1-(pyridin-4-ylmethylamino)pentan-2-yl]hexanamide), difelikefalin, or FE200041 ((2*R*)*-N-*[(2*R*)-1-amino-5-(diaminomethylideneamino)-1-oxopentan-2-yl]-2-[[(2*R*)-2-[[(2*R*)-2-amino-3-phenylpropanoyl]amino]-3-phenylpropanoyl]amino]hexanamide). In certain embodiments, the therapeutic peptide is difelikefalin.

In certain embodiments, the therapeutic peptide is an IR agonist. In certain embodiments, the therapeutic peptide is an insulin. In certain embodiments, the therapeutic peptide is insulin aspart, insulin degludec, insulin detemir, insulin glargine, insulin icodec, insulin lispro, or ORMD-0801. In certain embodiments, the therapeutic peptide is insulin aspart. In certain embodiments, the therapeutic peptide is insulin degludec. In certain embodiments, the therapeutic peptide is insulin detemir. In certain embodiments, the therapeutic peptide is insulin glargine. In certain embodiments, the therapeutic peptide is insulin icodec. In certain embodiments, the therapeutic peptide is insulin lispro.

In certain embodiments, the therapeutic peptide is a lipidated peptide. In certain embodiments, the therapeutic peptide is a lipidated GLP-1RA. In certain embodiments, the lipidated GLP-1RA is lixisenatide, semaglutide, or tirzepatide. In certain embodiments, the therapeutic peptide is a lipidated KOP agonist. In certain embodiments, the therapeutic peptide is a lipidated IR agonist. In certain embodiments, the lipidated IR agonist is insulin degludec or insulin detemir.

In one embodiment, provided herein is an oral pharmaceutical composition comprising a GLP-1RA, a bile acid compound, a CYP450 inhibitor, and one or more pharmaceutically acceptable excipients.

In another embodiment, provided herein is an oral pharmaceutical composition comprising a GLP-1RA, a bile acid compound, a CYP450 inhibitor, a gastrointestinal enzyme inhibitor, and one or more pharmaceutically acceptable excipients.

In yet another embodiment, provided herein is an oral pharmaceutical composition comprising a GLP-1RA, a bile acid compound, a CYP450 inhibitor, an absorption enhancer, and one or more pharmaceutically acceptable excipients.

In still another embodiment, provided herein is an oral pharmaceutical composition comprising a GLP-1RA, a bile acid compound, a CYP450 inhibitor, a gastrointestinal enzyme inhibitor, an absorption enhancer, and one or more pharmaceutically acceptable excipients.

In one embodiment, provided herein is an oral pharmaceutical composition comprising a GLP-1RA, a bile acid compound, a CYP450 inhibitor, a disintegrant, a filler, a lubricant, and a glidant.

In another embodiment, provided herein is an oral pharmaceutical composition comprising a GLP-1RA, a bile acid compound, a CYP450 inhibitor, a gastrointestinal enzyme inhibitor, a disintegrant, a filler, a lubricant, and a glidant.

In yet another embodiment, provided herein is an oral pharmaceutical composition comprising a GLP-1RA, a bile acid compound, a CYP450 inhibitor, an absorption enhancer, a disintegrant, a filler, a lubricant, and a glidant.

In still another embodiment, provided herein is an oral pharmaceutical composition comprising a GLP-1RA, a bile acid compound, a CYP450 inhibitor, a gastrointestinal enzyme inhibitor, an absorption enhancer, a disintegrant, a filler, a lubricant, and a glidant.

In one embodiment, provided herein is an oral pharmaceutical composition comprising a lipidated GLP-1RA, a bile acid compound, a CYP450 inhibitor, and one or more pharmaceutically acceptable excipients.

In another embodiment, provided herein is an oral pharmaceutical composition comprising a lipidated GLP-1RA, a bile acid compound, a CYP450 inhibitor, a gastrointestinal enzyme inhibitor, and one or more pharmaceutically acceptable excipients.

In yet another embodiment, provided herein is an oral pharmaceutical composition comprising a lipidated GLP-1RA, a bile acid compound, a CYP450 inhibitor, an absorption enhancer, and one or more pharmaceutically acceptable excipients.

In still another embodiment, provided herein is an oral pharmaceutical composition comprising a lipidated GLP-1RA, a bile acid compound, a CYP450 inhibitor, a gastrointestinal enzyme inhibitor, an absorption enhancer, and one or more pharmaceutically acceptable excipients.

In one embodiment, provided herein is an oral pharmaceutical composition comprising a lipidated GLP-1RA, a bile acid compound, a CYP450 inhibitor, a disintegrant, a filler, a lubricant, and a glidant.

In another embodiment, provided herein is an oral pharmaceutical composition comprising a lipidated GLP-1RA, a bile acid compound, a CYP450 inhibitor, a gastrointestinal enzyme inhibitor, a disintegrant, a filler, a lubricant, and a glidant.

In yet another embodiment, provided herein is an oral pharmaceutical composition comprising a lipidated GLP-1RA, a bile acid compound, a CYP450 inhibitor, an absorption enhancer, a disintegrant, a filler, a lubricant, and a glidant.

In still another embodiment, provided herein is an oral pharmaceutical composition comprising a lipidated GLP-1RA, a bile acid compound, a CYP450 inhibitor, a gastrointestinal enzyme inhibitor, an absorption enhancer, a disintegrant, a filler, a lubricant, and a glidant.

In one embodiment, provided herein is an oral pharmaceutical composition comprising semaglutide, a bile acid compound, a CYP450 inhibitor, and one or more pharmaceutically acceptable excipients.

In another embodiment, provided herein is an oral pharmaceutical composition comprising semaglutide, a bile acid compound, a CYP450 inhibitor, a gastrointestinal enzyme inhibitor, and one or more pharmaceutically acceptable excipients.

In yet another embodiment, provided herein is an oral pharmaceutical composition comprising semaglutide, a bile acid compound, a CYP450 inhibitor, an absorption enhancer, and one or more pharmaceutically acceptable excipients.

In still another embodiment, provided herein is an oral pharmaceutical composition comprising semaglutide, a bile acid compound, a CYP450 inhibitor, a gastrointestinal enzyme inhibitor, an absorption enhancer, and one or more pharmaceutically acceptable excipients.

In one embodiment, provided herein is an oral pharmaceutical composition comprising semaglutide, a bile acid compound, a CYP450 inhibitor, a disintegrant, a filler, a lubricant, and a glidant.

In another embodiment, provided herein is an oral pharmaceutical composition comprising semaglutide, a bile acid compound, a CYP450 inhibitor, a gastrointestinal enzyme inhibitor, a disintegrant, a filler, a lubricant, and a glidant.

In yet another embodiment, provided herein is an oral pharmaceutical composition comprising semaglutide, a bile acid compound, a CYP450 inhibitor, an absorption enhancer, a disintegrant, a filler, a lubricant, and a glidant.

In still another embodiment, provided herein is an oral pharmaceutical composition comprising semaglutide, a bile acid compound, a CYP450 inhibitor, a gastrointestinal enzyme inhibitor, an absorption enhancer, a disintegrant, a filler, a lubricant, and a glidant.

In one embodiment, provided herein is an oral pharmaceutical composition comprising tirzepatide, a bile acid compound, a CYP450 inhibitor, and one or more pharmaceutically acceptable excipients.

In another embodiment, provided herein is an oral pharmaceutical composition comprising tirzepatide, a bile acid compound, a CYP450 inhibitor, a gastrointestinal enzyme inhibitor, and one or more pharmaceutically acceptable excipients.

In yet another embodiment, provided herein is an oral pharmaceutical composition comprising tirzepatide, a bile acid compound, a CYP450 inhibitor, an absorption enhancer, and one or more pharmaceutically acceptable excipients.

In still another embodiment, provided herein is an oral pharmaceutical composition comprising tirzepatide, a bile acid compound, a CYP450 inhibitor, a gastrointestinal enzyme inhibitor, an absorption enhancer, and one or more pharmaceutically acceptable excipients.

In one embodiment, provided herein is an oral pharmaceutical composition comprising tirzepatide, a bile acid compound, a CYP450 inhibitor, a disintegrant, a filler, a lubricant, and a glidant.

In another embodiment, provided herein is an oral pharmaceutical composition comprising tirzepatide, a bile acid compound, a CYP450 inhibitor, a gastrointestinal enzyme inhibitor, a disintegrant, a filler, a lubricant, and a glidant.

In yet another embodiment, provided herein is an oral pharmaceutical composition comprising tirzepatide, a bile acid compound, a CYP450 inhibitor, an absorption enhancer, a disintegrant, a filler, a lubricant, and a glidant.

In still another embodiment, provided herein is an oral pharmaceutical composition comprising tirzepatide, a bile acid compound, a CYP450 inhibitor, a gastrointestinal enzyme inhibitor, an absorption enhancer, a disintegrant, a filler, a lubricant, and a glidant.

In certain embodiments, a pharmaceutical composition provided herein comprises from about 5 to about 80%, from about 5 to about 75%, from about 5 to about 65%, from about 10 to about 65%, from about 20 to about 65%, from about 30 to about 65%, from about 40 to about 65%, from about 10 to about 50%, or from about 15 to about 50% by weight of the bile acid compound. In certain embodiments, a pharmaceutical composition provided herein comprises from about 5 to about 80% by weight of the bile acid compound. In certain embodiments, a pharmaceutical composition provided herein comprises from about 5 to about 75% by weight of the bile acid compound. In certain embodiments, a pharmaceutical composition provided herein comprises from about 5 to about 65% by weight of the bile acid compound. In certain embodiments, a pharmaceutical composition provided herein comprises from about 10 to about 65% by weight of the bile acid compound. In certain embodiments, a pharmaceutical composition provided herein comprises from about 20 to about 60% by weight of the bile acid compound. In certain embodiments, a pharmaceutical composition provided herein comprises from about 30 to about 65% by weight of the bile acid compound. In certain embodiments, a pharmaceutical composition provided herein comprises from about 40 to about 65% by weight of the bile acid compound. In certain embodiments, a pharmaceutical composition provided herein comprises from about 10 to about 50% by weight of the bile acid compound. In certain embodiments, a pharmaceutical composition provided herein comprises from about 15 to about 50% by weight of the bile acid compound.

In certain embodiments, a pharmaceutical composition provided herein comprises from about 10 to about 500 mg, from about 25 to about 250 mg, from about 50 to about 250 mg, or from about 100 to about 250 mg of the bile acid compound. In certain embodiments, a pharmaceutical composition provided herein comprises from about 10 to about 500 mg of the bile acid compound. In certain embodiments, a pharmaceutical composition provided herein comprises from about 25 to about 250 mg of the bile acid compound. In certain embodiments, a pharmaceutical composition provided herein comprises from about 50 to about 250 mg of the bile acid compound. In certain embodiments, a pharmaceutical composition provided herein comprises from about 100 to about 250 mg of the bile acid compound.

In one embodiment, the bile acid compound is a compound having the structure of Formula (I): 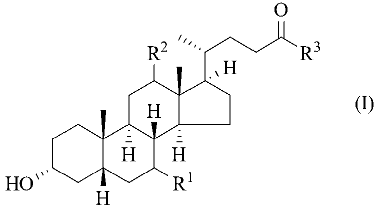 or an enantiomer, a mixture of enantiomers, a diastereomer, a mixture of two or more diastereomers, a tautomer, a mixture of two or more tautomers thereof; or a pharmaceutically acceptable salt, solvate, or hydrate thereof; wherein:
R¹ and R² are each independently hydrogen or -OH;
R³ is -N(H)-X-Z-R⁴, heterocyclyl, or -OH;
R⁴ is C₁₋₆ alkyl, C₁₋₆ heteroalkyl, C₇₋₁₅ aralkyl, heterocyclyl, -OH, or -N(H)R^{4a};
R^{4a} is C₁₋₆ alkyl, C₁₋₆ heteroalkyl, or C₇₋₁₅ aralkyl;
X is a bond, C₁₋₆ alkylene, or C₁₋₆ heteroalkylene; and
Z is a bond, -C(O)-, or -S(O₂)-;
wherein each alkyl, alkylene, heteroalkyl, heteroalkylene, aralkyl, and heterocyclyl is optionally substituted with one or more, in one embodiment, one, two, three, or four, substituents Q, wherein each Q is independently selected from: (a) deuterium, cyano, halo, imino, nitro, and oxo; (b) C₁₋₆ alkyl, C₁₋₆ heteroalkyl, C₂₋₆ alkenyl, C₂₋₆ alkynyl, C₃₋₁₀ cycloalkyl, C₆₋₁₄ aryl, C₇₋₁₅ aralkyl, heteroaryl, and heterocyclyl, each of which is further optionally substituted with one or more, in one embodiment, one, two, three, or four, substituents Q^{a}; and (c) -C(O)R^{a}, -C(O)OR^{a}, -C(O)NR^{b}R^{c}, -C(O)SR^{a}, -C(NR^{a})NR^{b}R^{c}, -C(S)R^{a}, -C(S)OR^{a}, -C(S)NR^{b}R^{c}, -OR^{a}, -OC(O)R^{a}, -OC(O)OR^{a}, -OC(O)NR^{b}R^{c}, -OC(O)SR^{a}, -OC(NR^{a})NR^{b}R^{c}, -OC(S)R^{a}, -OC(S)OR^{a}, -OC(S)NR^{b}R^{c}, -OS(O)R^{a}, -OS(O)₂R^{a}, -OS(O)NR^{b}R^{c}, -OS(O)₂NR^{b}R^{c}, -NR^{b}R^{c}, -NR^{a}C(O)R^{d}, -NR^{a}C(O)OR^{d}, -NR^{a}C(O)NR^{b}R^{c}, -NR^{a}C(O)SR^{d}, -NR^{a}C(NR^{d})NR^{b}R^{c}, -NR^{a}C(S)R^{d}, -NR^{a}C(S)OR^{d}, -NR^{a}C(S)NR^{b}R^{c}, -NR^{a}S(O)R^{d}, -NR^{a}S(O)₂R^{d}, -NR^{a}S(O)NR^{b}R^{c}, -NR^{a}S(O)₂NR^{b}R^{c}, -SR^{a}, -S(O)R^{a}, -S(O)₂R^{a}, -S(O)NR^{b}R^{c}, and -S(O)₂NR^{b}R^{c}, wherein each R^{a}, R^{b}, R^{c}, and R^{d} is independently (i) hydrogen or deuterium; (ii) C₁₋₆ alkyl, C₁₋₆ heteroalkyl, C₂₋₆ alkenyl, C₂₋₆ alkynyl, C₃₋₁₀ cycloalkyl, C₆₋₁₄ aryl, C₇₋₁₅ aralkyl, heteroaryl, or heterocyclyl, each of which is optionally substituted with one or more, in one embodiment, one, two, three, or four, substituents Q^{a}; or (iii) R^{b} and R^{c} together with the N atom to which they are attached form heterocyclyl, optionally substituted with one or more, in one embodiment, one, two, three, or four, substituents Q^{a};
wherein each Q^{a} is independently selected from: (a) deuterium, cyano, halo, nitro, imino, and oxo; (b) C₁₋₆ alkyl, C₂₋₆ alkenyl, C₂₋₆ alkynyl, C₃₋₁₀ cycloalkyl, C₆₋₁₄ aryl, C₇₋₁₅ aralkyl, heteroaryl, and heterocyclyl; and (c) -C(O)R^{e}, -C(O)OR^{e}, -C(O)NR^{f}R^{g}, -C(O)SR^{e}, -C(NR^{e})NR^{f}R^{g}, -C(S)R^{e}, -C(S)OR^{e}, -C(S)NR^{f}R^{g}, -OR^{e}, -OC(O)R^{e}, -OC(O)OR^{e}, -OC(O)NR^{f}R^{g}, -OC(O)SR^{e}, -OC(NR^{e})NR^{f}R^{g}, -OC(S)R^{e}, -OC(S)OR^{e}, -OC(S)NR^{f}R^{g}, -OS(O)R^{e}, -OS(O)₂R^{e}, -OS(O)NR^{f}R^{g}, -OS(O)₂NR^{f}R³, -NR^{f}R^{g}, -NR^{e}C(O)R^{h}, -NR^{e}C(O)OR^{f}, -NR^{e}C(O)NR^{f}R^{g}, -NR^{e}C(O)SR^{f}, -NR^{e}C(NR^{h})NR^{f}R^{g}, -NR^{e}C(S)R^{h}, -NR^{e}C(S)OR^{f}, -NR^{e}C(S)NR^{f}R^{g}, -NR^{e}S(O)R^{h}, -NR^{e}S(O)₂R^{h}, -NR^{e}S(O)NR^{f}R^{g}, -NR^{e}S(O)₂NR^{f}R^{g}, -SR^{e}, -S(O)R^{e}, -S(O)₂R^{e}, -S(O)NR^{f}R^{g}, and -S(O)₂NR^{f}R^{g}; wherein each R^{e}, R^{f}, R^{g}, and R^{h} is independently (i) hydrogen or deuterium; (ii) C₁₋₆ alkyl, C₂₋₆ alkenyl, C₂₋₆ alkynyl, C₃₋₁₀ cycloalkyl, C₆₋₁₄ aryl, C₇₋₁₅ aralkyl, heteroaryl, or heterocyclyl; or (iii) R^{f} and R^{g} together with the N atom to which they are attached form heterocyclyl.

In another embodiment, the bile acid compound is a compound having the structure of Formula (II): 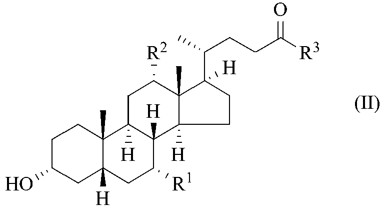 or an enantiomer, a mixture of enantiomers, a diastereomer, a mixture of two or more diastereomers, a tautomer, a mixture of two or more tautomers thereof; or a pharmaceutically acceptable salt, solvate, or hydrate thereof; wherein R¹, R², and R³ are each as defined herein.

In certain embodiments, in Formula (I) or (II), R³ is heterocyclyl, optionally substituted with one or more substituents Q. In certain embodiments, in Formula (I) or (II), R³ is monocyclic heterocyclyl, optionally substituted with one or more substituents Q. In certain embodiments, in Formula (I) or (II), R³ is 3-, 4-, 5-, 6-, or 7-membered heterocyclyl, each optionally substituted with one or more substituents Q. In certain embodiments, in Formula (I) or (II), R³ is 3-membered heterocyclyl, optionally substituted with one or more substituents Q. In certain embodiments, in Formula (I) or (II), R³ is 4-membered heterocyclyl, optionally substituted with one or more substituents Q. In certain embodiments, in Formula (I) or (II), R³ is 5-membered heterocyclyl, optionally substituted with one or more substituents Q. In certain embodiments, in Formula (I) or (II), R³ is pyrrolidinyl, optionally substituted with one or more substituents Q. In certain embodiments, in Formula (I) or (II), R³ is pyrrolidin-1-yl, optionally substituted with one or more substituents Q. In certain embodiments, in Formula (I) or (II), R³ is 2-carboxypyrrolidin-1-yl. In certain embodiments, in Formula (I) or (II), R³ is 2(R)-carboxy-pyrrolidin-1-yl. In certain embodiments, in Formula (I) or (II), R³ is 2(S)-carboxypyrrolidin-1-yl. In certain embodiments, in Formula (I) or (II), R³ is 6-membered heterocyclyl, optionally substituted with one or more substituents Q. In certain embodiments, in Formula (I) or (II), R³ is 7-membered heterocyclyl, optionally substituted with one or more substituents Q.

In certain embodiments, in Formula (I) or (II), R³ is bicyclic heterocyclyl, optionally substituted with one or more substituents Q. In certain embodiments, in Formula (I) or (II), R³ is bridged, fused, or spiro heterocyclyl, each optionally substituted with one or more substituents Q. In certain embodiments, in Formula (I) or (II), R³ is bridged heterocyclyl, optionally substituted with one or more substituents Q. In certain embodiments, in Formula (I) or (II), R³ is fused heterocyclyl, optionally substituted with one or more substituents Q. In certain embodiments, in Formula (I) or (II), R³ is spiro heterocyclyl, optionally substituted with one or more substituents Q.

In certain embodiments, in Formula (I) or (II), R³ is -OH.

In yet another embodiment, the bile acid compound is a compound having the structure of Formula (III): 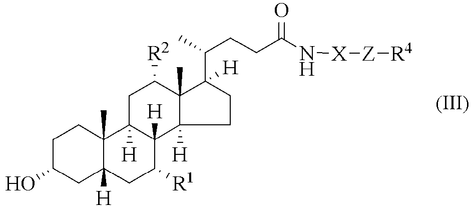 or an enantiomer, a mixture of enantiomers, a diastereomer, a mixture of two or more diastereomers, a tautomer, a mixture of two or more tautomers thereof; or a pharmaceutically acceptable salt, solvate, or hydrate thereof; wherein R¹, R², R⁴, X, and Z are each as defined herein.

In certain embodiments, in any one of Formulae (I) to (III), R⁴ is C₁₋₆ alkyl, optionally substituted with one or more substituents Q. In certain embodiments, in any one of Formulae (I) to (III), R⁴ is C₁₋₆ heteroalkyl, optionally substituted with one or more substituents Q. In certain embodiments, in any one of Formulae (I) to (III), R⁴ is C₇₋₁₅ aralkyl, optionally substituted with one or more substituents Q.

In certain embodiments, in any one of Formulae (I) to (III), R⁴ is heterocyclyl, optionally substituted with one or more substituents Q. In certain embodiments, in any one of Formulae (I) to (III), R⁴ is monocyclic heterocyclyl, optionally substituted with one or more substituents Q. In certain embodiments, in any one of Formulae (I) to (III), R⁴ is 3-, 4-, 5-, 6-, or 7-membered heterocyclyl, each optionally substituted with one or more substituents Q. In certain embodiments, in any one of Formulae (I) to (III), R⁴ is 3-membered heterocyclyl, optionally substituted with one or more substituents Q. In certain embodiments, in any one of Formulae (I) to (III), R⁴ is 4-membered heterocyclyl, optionally substituted with one or more substituents Q. In certain embodiments, in any one of Formulae (I) to (III), R⁴ is 5-membered heterocyclyl, optionally substituted with one or more substituents Q. In certain embodiments, in any one of Formulae (I) to (III), R⁴ is pyrrolidinyl, optionally substituted with one or more substituents Q. In certain embodiments, in any one of Formulae (I) to (III), R⁴ is pyrrolidin-1-yl, optionally substituted with one or more substituents Q. In certain embodiments, in any one of Formulae (I) to (III), R⁴ is 2-carboxypyrrolidin-1-yl. In certain embodiments, in any one of Formulae (I) to (III), R⁴ is 2(*R*)-carboxypyrrolidin-1-yl. In certain embodiments, in any one of Formulae (I) to (III), R⁴ is 2(*S*)-carboxy-pyrrolidin-1-yl. In certain embodiments, in any one of Formulae (I) to (III), R⁴ is 6-membered heterocyclyl, optionally substituted with one or more substituents Q. In certain embodiments, in any one of Formulae (I) to (III), R⁴ is 7-membered heterocyclyl, optionally substituted with one or more substituents Q.

In certain embodiments, in any one of Formulae (I) to (III), R⁴ is -OH.

In certain embodiments, in any one of Formulae (I) to (III), R⁴ is C₁₋₆ alkyl or C₇₋₁₅ aralkyl, each optionally substituted with one or more substituents Q. In certain embodiments, in any one of Formulae (I) to (III), R⁴ is C₁₋₆ alkyl or C₇₋₁₅ aralkyl, each optionally substituted with heteroaryl, -C(O)OR^{a}, -C(O)NR^{b}R^{c}, -OR^{a}, -NR^{b}R^{c}, -NR^{a}C(NR^{d})NR^{b}R^{c}, or -SR^{a}; wherein each R^{a}, R^{b}, R^{c}, and R^{d} is as defined herein. In certain embodiments, in any one of Formulae (I) to (III), R⁴ is (i) hydrogen; or (ii) methyl, ethyl, propyl, butyl, or benzyl, each optionally substituted with imidazolyl, indolyl, carboxy, aminocarbonyl, hydroxyl, amino, guanidino, mercapto, or methylthio. In certain embodiments, in any one of Formulae (I) to (III), R⁴ is hydrogen, methyl, isopropyl, isobutyl, but-2-yl, benzyl, 4-hydroxybenzyl, imidazol-4-ylmethyl, indol-3-ylmethyl, carboxymethyl, 2-carboxyethyl, aminocarbonylmethyl, 2-aminocarbonylethyl, hydroxymethyl, 1-hydroxyethyl, 3-aminopropyl, 4-aminobutyl, 3-guanidinopropyl, mercaptomethyl, or 2-methylthioethyl.

In certain embodiments, in any one of Formulae (I) to (III), X is a bond. In certain embodiments, in any one of Formulae (I) to (III), X is C₁₋₆ alkylene, optionally substituted with one or more substituents Q. In certain embodiments, in any one of Formulae (I) to (III), X is C₁₋₆ alkylene, optionally substituted with (i) C₁₋₆ alkyl, C₆₋₁₄ aryl, or heteroaryl, each optionally substituted with one or more substituents Q; or (ii) -C(O)OR^{a}, -C(O)NR^{b}R^{c}, -OR^{a}, -NR^{b}R^{c}, -NR^{a}C(NR^{d})NR^{b}R^{c}, or -SR^{a}; wherein each R^{a}, R^{b}, R^{c}, and R^{d} is as defined herein. In certain embodiments, in any one of Formulae (I) to (III), X is methanediyl, ethanediyl, propanediyl, butanediyl, or pentanediyl, each optionally substituted with (i) C₁₋₆ alkyl, C₆₋₁₄ aryl, or heteroaryl, each optionally substituted with one or more substituents Q; or (ii) -C(O)OR^{a}, -C(O)NR^{b}R^{c}, -OR^{a}, -NR^{b}R^{c}, -NR^{a}C(NR^{d})NR^{b}R^{c}, or -SR^{a}; wherein each R^{a}, R^{b}, R^{c}, and R^{d} is as defined herein. In certain embodiments, in any one of Formulae (I) to (III), X is methanediyl, ethanediyl, propanediyl, butanediyl, or pentanediyl, each optionally substituted with (i) methyl, phenyl, imidazolyl, or indolyl, each optionally substituted with one or more substituents Q; or (ii) carboxy, aminocarbonyl, hydroxyl, amino, guanidino, mercapto, or methylthio. In certain embodiments, in any one of Formulae (I) to (III), X is methanediyl, ethane-1,1-diyl, propane-1,1-diyl, butane-1,1-diyl, or pentane-1,1-diyl, each optionally substituted with methyl, phenyl, 4-hydroxyphenyl, imidazol-4-yl, indol-3-yl, carboxy, aminocarbonyl, hydroxyl, amino, guanidino, mercapto, or methylthio. In certain embodiments, in any one of Formulae (I) to (III), X is methanediyl, ethane-1,1-diyl, 2-phenylethane-1,1-diyl, 2-(4-hydroxyphenyl)ethane-1,1-diyl, 2-imidazol-4-ylethane-1,1-diyl, 2-(indol-3-yl)ethane-1,1-diyl, 2-carboxyethane-1,1-diyl, 2-hydroxyethane-1,1-diyl, 2-aminocarbonylethane-1,1-diyl, 2-mercaptoethane-1,1-diyl, 2-methylpropane-1,1-diyl, 2-carboxypropane-1,1-diyl, 2-hydroxypropane-1,1-diyl, 3-aminocarbonyl-propane-1,1-diyl, 3-methylthiopropane-1,1-diyl, 2-methylbutane-1,1-diyl, 3-methylbutane-1,1-diyl, 4-aminobutane-1,1-diyl, 4-guanidinobutane-1,1-diyl, or 5-aminopentyane-1,1-diyl.

In certain embodiments, in any one of Formulae (I) to (III), X is C₁₋₆ heteroalkylene, optionally substituted with one or more substituents Q.

In certain embodiments, in any one of Formulae (I) to (III), Z is a bond. In certain embodiments, in any one of Formulae (I) to (III), Z is -C(O)-. In certain embodiments, in any one of Formulae (I) to (III), Z is -S(O₂)-.

In certain embodiments, in any one of Formulae (I) to (III), X is C₁₋₆ alkylene, optionally substituted with one or more substituents Q; and Z is -C(O)-. In certain embodiments, in any one of Formulae (I) to (III), X is C₁₋₆ alkylene, optionally substituted with one or more substituents Q; and Z is -S(O₂)-.

In yet another embodiment, the bile acid compound is a compound having the structure of Formula (IV): 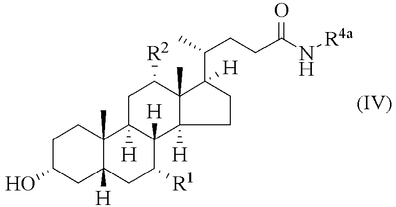 or an enantiomer, a mixture of enantiomers, a diastereomer, a mixture of two or more diastereomers, a tautomer, a mixture of two or more tautomers thereof; or a pharmaceutically acceptable salt, solvate, or hydrate thereof; wherein R¹, R², and R^{4a} are each as defined herein.

In certain embodiments, in Formula (IV), R^{4a} is C₁₋₆ alkyl, optionally substituted with one or more substituents Q. In certain embodiments, in Formula (IV), R^{4a} is C₁₋₆ heteroalkyl, optionally substituted with one or more substituents Q. In certain embodiments, in Formula (IV), R^{4a} is C₇₋₁₅ aralkyl, optionally substituted with one or more substituents Q.

In certain embodiments, in Formula (IV), R^{4a} is C₁₋₆ alkyl, optionally substituted one or two substituents, each of which is independently (i) C₁₋₆ alkyl, C₆₋₁₄ aryl, or heteroaryl, each optionally substituted with one or more substituents Q; or (ii) -C(O)OR^{a}, -C(O)NR^{b}R^{c}, -OR^{a}, -NR^{b}R^{c}, -NR^{a}C(NR^{d})NR^{b}R^{c}, or -SR^{a}; wherein each R^{a}, R^{b}, R^{c}, and R^{d} is as defined herein. In certain embodiments, in Formula (IV), R^{4a} is methyl, ethyl, propyl, butyl, or pentyl, each optionally substituted one or two substituents, each of which is independently (i) C₁₋₆ alkyl, C₆₋₁₄ aryl, or heteroaryl, each optionally substituted with one or more substituents Q; or (ii) -C(O)OR^{a}, -C(O)NR^{b}R^{c}, -OR^{a}, -NR^{b}R^{c}, -NR^{a}C(NR^{d})NR^{b}R^{c}, or -SR^{a}; wherein each R^{a}, R^{b}, R^{c}, and R^{d} is as defined herein. In certain embodiments, in Formula (IV), R^{4a} is carboxymethyl, carboxyethyl, carboxypropyl, carboxybutyl, or carboxypentyl, each optionally substituted with (i) methyl, phenyl, imidazolyl, or indolyl, each optionally substituted with one or more substituents Q; or (ii) carboxy, aminocarbonyl, hydroxyl, amino, guanidino, mercapto, or methylthio. In certain embodiments, in Formula (IV), R^{4a} is carboxymethyl, 1-carboxyethyl, 1-carboxypropyl, 1-carboxybutyl, or 1-carboxypentyl, each optionally substituted with methyl, phenyl, 4-hydroxy-phenyl, imidazol-4-yl, indol-3-yl, carboxy, aminocarbonyl, hydroxyl, amino, guanidino, mercapto, or methylthio. In certain embodiments, in Formula (IV), R^{4a} is carboxymethyl, 1-carboxyethyl, 1-carboxy-2-phenylethyl, 1-carboxy-2-(4-hydroxyphenyl)ethyl, 1-carboxy-2-imidazol-4-ylethyl, 1-carboxy-2-(indol-3-yl)ethyl, 1-carboxy-2-carboxyethyl, 1-carboxy-2-hydroxyethyl, 1-carboxy-2-aminocarbonylethyl, 1-carboxy-2-mercaptoethyl, 1-carboxy-2-methylpropyl, 1-carboxy-2-carboxypropyl, 1-carboxy-2-hydroxypropyl, 1-carboxy-3-aminocarbonylpropyl, 1-carboxy-3-methylthiopropyl, 1-carboxy-2-methylbutyl, 1-carboxy-3-methylbutyl, 1-carboxy-4-aminobutyl, 1-carboxy-4-guanidinobutyl, or 1-carboxy-5-aminopentyl.

In yet another embodiment, the bile acid compound is a compound having the structure of Formula (V): 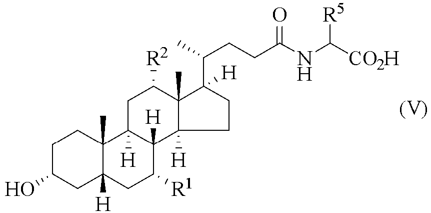 or an enantiomer, a mixture of enantiomers, a diastereomer, a mixture of two or more diastereomers, a tautomer, a mixture of two or more tautomers thereof; or a pharmaceutically acceptable salt, solvate, or hydrate thereof; wherein R⁵ is (i) hydrogen; or (ii) C₁₋₆ alkyl, C₁₋₆ heteroalkyl, C₁₋₆ heteroalkyl, or C₇₋₁₅ aralkyl, each optionally substituted with one or more substituents Q; and R¹ and R² are each as defined herein.

In yet another embodiment, the bile acid compound is a compound having the structure of Formula (VI): 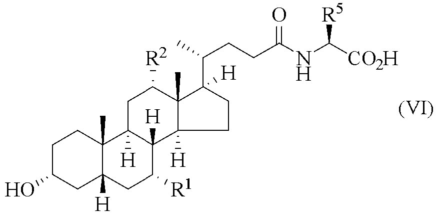 or a diastereomer, a mixture of two or more diastereomers, a tautomer, a mixture of two or more tautomers thereof; or a pharmaceutically acceptable salt, solvate, or hydrate thereof; wherein R¹, R², and R⁵ are each as defined herein.

In certain embodiments, in Formula (V) or (VI), R⁵ is hydrogen. In certain embodiments, in Formula (V) or (VI), R⁵ is C₁₋₆ alkyl, optionally substituted with one or more substituents Q. In certain embodiments, in Formula (V) or (VI), R⁵ is C₁₋₆ heteroalkyl, optionally substituted with one or more substituents Q. In certain embodiments, in Formula (V) or (VI), R⁵ is C₇₋₁₅ aralkyl, optionally substituted with one or more substituents Q.

In certain embodiments, in Formula (V) or (VI), R⁵ is (i) hydrogen; or (ii) C₁₋₆ alkyl or C₇₋₁₅ aralkyl, each optionally substituted with heteroaryl, -C(O)OR^{a}, -C(O)NR^{b}R^{c}, -OR^{a}, -NR^{b}R^{c}, -NR^{a}C(NR^{d})NR^{b}R^{c}, or -SR^{a}; wherein each R^{a}, R^{b}, R^{c}, and R^{d} is as defined herein. In certain embodiments, in Formula (V) or (VI), R⁵ is (i) hydrogen; or (ii) methyl, ethyl, propyl, butyl, or benzyl, each optionally substituted with heteroaryl, -C(O)OR^{a}, -C(O)NR^{b}R^{c}, -OR^{a}, -NR^{b}R^{c}, -NR^{a}C(NR^{d})NR^{b}R^{c}, or -SR^{a}; wherein each R^{a}, R^{b}, R^{c}, and R^{d} is as defined herein. In certain embodiments, in Formula (V) or (VI), R⁵ is (i) hydrogen; or (ii) methyl, ethyl, propyl, butyl, or benzyl, each optionally substituted with imidazolyl, indolyl, carboxy, aminocarbonyl, hydroxyl, amino, guanidino, mercapto, or methylthio. In certain embodiments, in Formula (V) or (VI), R⁵ is hydrogen, methyl, isopropyl, isobutyl, but-2-yl, benzyl, 4-hydroxybenzyl, imidazol-4-ylmethyl, indol-3-ylmethyl, carboxymethyl, 2-carboxyethyl, aminocarbonylmethyl, 2-aminocarbonylethyl, hydroxymethyl, 1-hydroxyethyl, 3-aminopropyl, 4-aminobutyl, 3-guanidinopropyl, mercaptomethyl, or 2-methylthioethyl.

In yet another embodiment, the bile acid compound is a compound having the structure of Formula (VII): 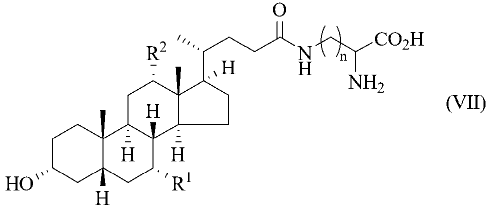 or an enantiomer, a mixture of enantiomers, a diastereomer, a mixture of two or more diastereomers, a tautomer, a mixture of two or more tautomers thereof; or a pharmaceutically acceptable salt, solvate, or hydrate thereof; wherein n is an integer of 1, 2, 3, 4, or 5; and R¹ and R² are each as defined herein.

In yet another embodiment, the bile acid compound is a compound having the structure of Formula (VIII): 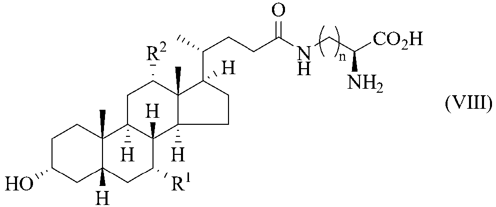 or a diastereomer, a mixture of two or more diastereomers, a tautomer, a mixture of two or more tautomers thereof; or a pharmaceutically acceptable salt, solvate, or hydrate thereof; wherein R¹, R², and n are each as defined herein.

In certain embodiments, in Formula (VII) or (VIII), n is an integer of 1. In certain embodiments, in Formula (VII) or (VIII), n is an integer of 2. In certain embodiments, in Formula (VII) or (VIII), n is an integer of 3. In certain embodiments, in Formula (VII) or (VIII), n is an integer of 4. In certain embodiments, in Formula (VII) or (VIII), n is an integer of 5.

In yet another embodiment, the bile acid compound is a compound having the structure of Formula (IX): 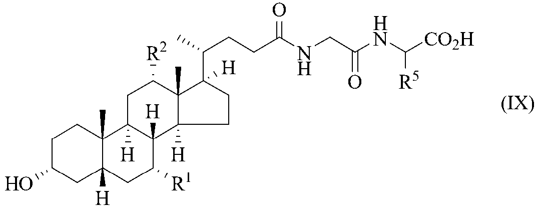 or an enantiomer, a mixture of enantiomers, a diastereomer, a mixture of two or more diastereomers, a tautomer, a mixture of two or more tautomers thereof; or a pharmaceutically acceptable salt, solvate, or hydrate thereof; wherein R¹, R², and R⁵ are each as defined herein.

In yet another embodiment, the bile acid compound is a compound having the structure of Formula (X): 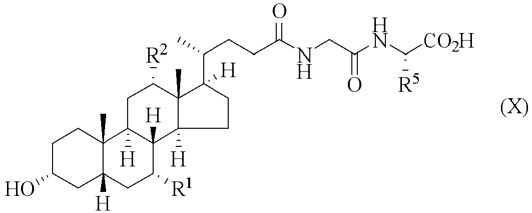 or a diastereomer, a mixture of two or more diastereomers, a tautomer, a mixture of two or more tautomers thereof; or a pharmaceutically acceptable salt, solvate, or hydrate thereof; wherein R¹, R², and R⁵ are each as defined herein.

In certain embodiments, in Formula (IX) or (X), R⁵ is hydrogen. In certain embodiments, in Formula (VII), R⁵ is amino. In certain embodiments, in Formula (IX) or (X), R⁵ is C₁₋₆ alkyl, optionally substituted with one or more substituents Q. In certain embodiments, in Formula (IX) or (X), R⁵ is C₁₋₆ heteroalkyl, optionally substituted with one or more substituents Q. In certain embodiments, in Formula (IX) or (X), R⁵ is C₇₋₁₅ aralkyl, optionally substituted with one or more substituents Q.

In certain embodiments, in Formula (IX) or (X), R⁵ is (i) hydrogen; or (ii) C₁₋₆ alkyl or C₇₋₁₅ aralkyl, each optionally substituted with heteroaryl, -C(O)OR^{a}, -C(O)NR^{b}R^{c}, -OR^{a}, -NR^{b}R^{c}, -NR^{a}C(NR^{d})NR^{b}R^{c}, or -SR^{a}; wherein each R^{a}, R^{b}, R^{c}, and R^{d} is as defined herein. In certain embodiments, in Formula (IX) or (X), R⁵ is (i) hydrogen; or (ii) methyl, ethyl, propyl, butyl, or benzyl, each optionally substituted with heteroaryl, -C(O)OR^{a}, -C(O)NR^{b}R^{c}, -OR^{a}, -NR^{b}R^{c}, -NR^{a}C(NR^{d})NR^{b}R^{c}, or -SR^{a}; wherein each R^{a}, R^{b}, R^{c}, and R^{d} is as defined herein. In certain embodiments, in Formula (IX) or (X), R⁵ is (i) hydrogen; or (ii) methyl, ethyl, propyl, butyl, or benzyl, each optionally substituted with imidazolyl, indolyl, carboxy, aminocarbonyl, hydroxyl, amino, guanidino, mercapto, or methylthio. In certain embodiments, in Formula (IX) or (X), R⁵ is hydrogen, methyl, isopropyl, isobutyl, but-2-yl, benzyl, 4-hydroxybenzyl, imidazol-4-ylmethyl, indol-3-ylmethyl, carboxymethyl, 2-carboxyethyl, aminocarbonylmethyl, 2-aminocarbonylethyl, hydroxymethyl, 1-hydroxyethyl, 3-aminopropyl, 4-aminobutyl, 3-guanidinopropyl, mercaptomethyl, or 2-methylthioethyl.

In yet another embodiment, the bile acid compound described herein is a compound having the structure of Formula (XI): 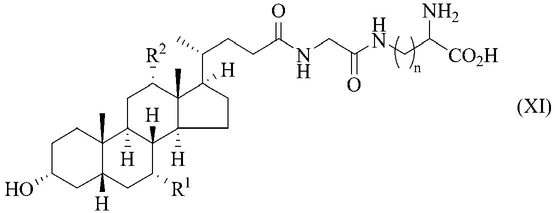 or an enantiomer, a mixture of enantiomers, a diastereomer, a mixture of two or more diastereomers, a tautomer, a mixture of two or more tautomers thereof; or a pharmaceutically acceptable salt, solvate, or hydrate thereof; wherein R¹, R², and n are each as defined herein.

In still another embodiment, the bile acid compound described herein is a compound having the structure of Formula (XII): 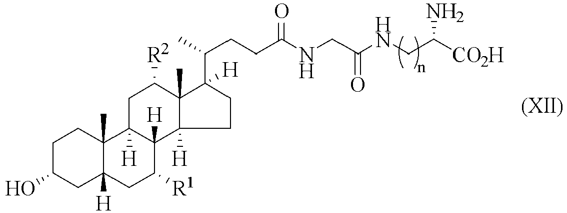 or a diastereomer, a mixture of two or more diastereomers, a tautomer, a mixture of two or more tautomers thereof; or a pharmaceutically acceptable salt, solvate, or hydrate thereof; wherein R¹, R², and n are each as defined herein.

In certain embodiments, in Formula (XI) or (XII), n is an integer of 1. In certain embodiments, in Formula (XI) or (XII), n is an integer of 2. In certain embodiments, in Formula (XI) or (XII), n is an integer of 3. In certain embodiments, in Formula (XI) or (XII), n is an integer of 4. In certain embodiments, in Formula (XI) or (XII), n is an integer of 5.

In certain embodiments, in any one of Formulae (I) to (XII), R¹ is hydrogen. In certain embodiments, in any one of Formulae (I) to (XII), R¹ is -OH.

In certain embodiments, in any one of Formulae (I) to (IX), R² is hydrogen. In certain embodiments, in any one of Formulae (I) to (XII), R² is -OH.

In certain embodiments, the bile acid compound is:
*N*⁶-(((*R*)-4-((3*R*,5*S*,7*R*,8*R*,9*S*,10*S*,12*S*,13*R*,14*S*,17*R*)-3,7,12-trihydroxy-10,13-dimethyl-hexadecahydro-1*H*-cyclopenta[*a*]phenanthren-17-yl)pentanoyl)glycyl)-*L*-lysine (KGCA);
*N*⁶-((*R*)-4-((3*R*,5*S*,7*R*,8*R*,9*S*,10*S*,13*R*,14*S*,17*R*)-3,7-dihydroxy-10,13-dimethylhexadecahydro-1*H*-cyclopenta[*a*]phenanthren-17-yl)pentanoyl)-*L*-lysine (KCDCA);
*N*⁶-((*R*)-4-((3*R*,5*S*,7*S*,8*R*,9*S*,10*S*,13*R*,14*S*,17*R*)-3,7-dihydroxy-10,13-dimethylhexadecahydro-1*H*-cyclopenta[*a*]phenanthren-17-yl)pentanoyl)-*L*-lysine (KUDCA);
*N*⁶-((*R*)-4-((3*R*,5*R*,8*R*,9*S*,10*S*,12*S*,13*R*,14*S*,17*R*)-3,12-dihydroxy-10,13-dimethylhexadecahydro-1*H*-cyclopenta[*a*]phenanthren-17-yl)pentanoyl)-*L*-lysine (KDCA);
*N*⁶-((*R*)-4-((3*R*,5*S*,7*R*,8*R*,9*S*,10*S*,12*S*,13*R*,14*S*,17*R*)-3,7,12-trihydroxy-10,13-dimethylhexadecahydro-1*H*-cyclopenta[*a*]phenanthren-17-yl)pentanoyl)-*L*-lysine (KCA);
*N*⁶-(((*R*)-4-((3*R*,5*S*,7*R*,8*R*,9*S*,10*S*,12*S*,13*R*,14*S*,17*R*)-3,7,12-trihydroxy-10,13-dimethylhexadecahydro-1*H*-cyclopenta[*a*]phenanthren-17-yl)pentanoyl)glycyl)-*L*-arginine (RGCA);
*N*⁶-(((*R*)-4-((3*R*,5*S*,7*R*,8*R*,9*S*,10*S*,12*S*,13*R*,14*S*,17*R*)-3,7,12-trihydroxy-10,13-dimethylhexadecahydro-1*H*-cyclopenta[*a*]phenanthren-17-yl)pentanoyl)glycyl)-*L*-histidine (HGCA); or
*N*⁶-(((*R*)-4-((3*R*,5*S*,7*R*,8*R*,9*S*,10*S*,12*S*,13*R*,14*S*,17*R*)-3,7,12-trihydroxy-10,13-dimethylhexadecahydro-1*H*-cyclopenta[*a*]phenanthren-17-yl)pentanoyl)glycyl)-*L*-aspartic acid (DGCA); or a diastereomer, a mixture of two or more diastereomers, a tautomer, a mixture of two or more tautomers thereof; or a pharmaceutically acceptable salt, solvate, or hydrate thereof.

In certain embodiments, the bile acid compound is KGCA, or a diastereomer, a mixture of two or more diastereomers, a tautomer, a mixture of two or more tautomers thereof; or a pharmaceutically acceptable salt, solvate, or hydrate thereof. In certain embodiments, the bile acid compound is KCDCA, or a diastereomer, a mixture of two or more diastereomers, a tautomer, a mixture of two or more tautomers thereof; or a pharmaceutically acceptable salt, solvate, or hydrate thereof. In certain embodiments, the bile acid compound is KUDCA, or a diastereomer, a mixture of two or more diastereomers, a tautomer, a mixture of two or more tautomers thereof; or a pharmaceutically acceptable salt, solvate, or hydrate thereof. In certain embodiments, the bile acid compound is KDCA, or a diastereomer, a mixture of two or more diastereomers, a tautomer, a mixture of two or more tautomers thereof; or a pharmaceutically acceptable salt, solvate, or hydrate thereof. In certain embodiments, the bile acid compound is KCA, or a diastereomer, a mixture of two or more diastereomers, a tautomer, a mixture of two or more tautomers thereof; or a pharmaceutically acceptable salt, solvate, or hydrate thereof. In certain embodiments, the bile acid compound is RGCA, or a diastereomer, a mixture of two or more diastereomers, a tautomer, a mixture of two or more tautomers thereof; or a pharmaceutically acceptable salt, solvate, or hydrate thereof. In certain embodiments, the bile acid compound is HGCA, or a diastereomer, a mixture of two or more diastereomers, a tautomer, a mixture of two or more tautomers thereof; or a pharmaceutically acceptable salt, solvate, or hydrate thereof. In certain embodiments, the bile acid compound is DGCA, or a diastereomer, a mixture of two or more diastereomers, a tautomer, a mixture of two or more tautomers thereof; or a pharmaceutically acceptable salt, solvate, or hydrate thereof.

In certain embodiments, the bile acid compound is chenodeoxycholic acid (CDCA), cholic acid (CA), dehydrocholic acid (DHCA), deoxycholic acid (DCA), glycochenodeoxycholic acid (GCDCA), glycocholic acid (GCA), lithocholic acid (LCA), taurocholic acid (TCA), taurodeoxycholic acid (TDCA), or ursodeoxycholic acid (UDCA), or a diastereomer, a mixture of two or more diastereomers, a tautomer, a mixture of two or more tautomers thereof; or a pharmaceutically acceptable salt, solvate, or hydrate thereof. In certain embodiments, the bile acid compound is CDCA, CA, DCA, GCA, or UDCA, or a diastereomer, a mixture of two or more diastereomers, a tautomer, a mixture of two or more tautomers thereof; or a pharmaceutically acceptable salt, solvate, or hydrate thereof. In certain embodiments, the bile acid compound is DCA or GCA, or a diastereomer, a mixture of two or more diastereomers, a tautomer, a mixture of two or more tautomers thereof; or a pharmaceutically acceptable salt, solvate, or hydrate thereof.

In certain embodiments, the bile acid compound is CDCA, CA, DHCA, DCA, GCDCA, GCA, LCA, TCA, TDCA, or UDCA, or a combination of two or more thereof. In certain embodiments, the bile acid compound is CDCA, CA, DCA, GCA, or UDCA, or a combination of two or more thereof. In certain embodiments, the bile acid compound is DCA or GCA, or a combination thereof.

In certain embodiments, the bile acid compound is CDCA, or a diastereomer, a mixture of two or more diastereomers, a tautomer, a mixture of two or more tautomers thereof; or a pharmaceutically acceptable salt, solvate, or hydrate thereof. In certain embodiments, the bile acid compound is CA, or a diastereomer, a mixture of two or more diastereomers, a tautomer, a mixture of two or more tautomers thereof; or a pharmaceutically acceptable salt, solvate, or hydrate thereof. In certain embodiments, the bile acid compound is DHCA, or a diastereomer, a mixture of two or more diastereomers, a tautomer, a mixture of two or more tautomers thereof; or a pharmaceutically acceptable salt, solvate, or hydrate thereof. In certain embodiments, the bile acid compound is DCA, or a diastereomer, a mixture of two or more diastereomers, a tautomer, a mixture of two or more tautomers thereof; or a pharmaceutically acceptable salt, solvate, or hydrate thereof. In certain embodiments, the bile acid compound is GCDCA, or a diastereomer, a mixture of two or more diastereomers, a tautomer, a mixture of two or more tautomers thereof; or a pharmaceutically acceptable salt, solvate, or hydrate thereof. In certain embodiments, the bile acid compound is GCA, or a diastereomer, a mixture of two or more diastereomers, a tautomer, a mixture of two or more tautomers thereof; or a pharmaceutically acceptable salt, solvate, or hydrate thereof. In certain embodiments, the bile acid compound is LCA, or a diastereomer, a mixture of two or more diastereomers, a tautomer, a mixture of two or more tautomers thereof; or a pharmaceutically acceptable salt, solvate, or hydrate thereof. In certain embodiments, the bile acid compound is TCA, or a diastereomer, a mixture of two or more diastereomers, a tautomer, a mixture of two or more tautomers thereof; or a pharmaceutically acceptable salt, solvate, or hydrate thereof. In certain embodiments, the bile acid compound is TDCA, or a diastereomer, a mixture of two or more diastereomers, a tautomer, a mixture of two or more tautomers thereof; or a pharmaceutically acceptable salt, solvate, or hydrate thereof. In certain embodiments, the bile acid compound is UDCA, or a diastereomer, a mixture of two or more diastereomers, a tautomer, a mixture of two or more tautomers thereof; or a pharmaceutically acceptable salt, solvate, or hydrate thereof.

In certain embodiments, a pharmaceutical composition provided herein comprises from about 5 to about 50%, from about 10 to about 50%, from about 10 to about 40%, from about 20 to about 40%, from about 15 to about 35%, or from about 20 to about 35% by weight of the CYP450 inhibitor. In certain embodiments, a pharmaceutical composition provided herein comprises from about 5 to about 50% by weight of the CYP450 inhibitor. In certain embodiments, a pharmaceutical composition provided herein comprises from about 10 to about 50% by weight of the CYP450 inhibitor. In certain embodiments, a pharmaceutical composition provided herein comprises from about 10 to about 40% by weight of the CYP450 inhibitor. In certain embodiments, a pharmaceutical composition provided herein comprises from about 20 to about 40% by weight of the CYP450 inhibitor. In certain embodiments, a pharmaceutical composition provided herein comprises from about 15 to about 35% by weight of the CYP450 inhibitor. In certain embodiments, a pharmaceutical composition provided herein comprises from about 20 to about 35% by weight of the CYP450 inhibitor.

In certain embodiments, a pharmaceutical composition provided herein comprises from about 10 to about 500 mg, from about 25 to about 250 mg, or from about 50 to about 100 mg of the CYP450 inhibitor. In certain embodiments, a pharmaceutical composition provided herein comprises from about 10 to about 500 mg of the CYP450 inhibitor. In certain embodiments, a pharmaceutical composition provided herein comprises from about 25 to about 250 mg of the CYP450 inhibitor. In certain embodiments, a pharmaceutical composition provided herein comprises from about 50 to about 100 mg of the CYP450 inhibitor.

In certain embodiments, the CYP450 inhibitor is a gallate ester. In certain embodiments, the CYP450 inhibitor is dodecyl gallate (DG), ethyl gallate (EG), octyl gallate (OG), or propyl gallate (PG). In certain embodiments, the CYP450 inhibitor is DG, OG, or PG. In certain embodiments, the CYP450 inhibitor is DG. In certain embodiments, the CYP450 inhibitor is OG. In certain embodiments, the CYP450 inhibitor is PG.

In certain embodiments, a pharmaceutical composition provided herein has a weight ratio of the bile acid compound over the CYP450 inhibitor in a range from about 0.1 to about 50, from about 0.1 to about 25, from about 0.2 to about 10, from about 0.5 to about 5, or from about 0.5 to about 3. In certain embodiments, a pharmaceutical composition provided herein has a weight ratio of the bile acid compound over the CYP450 inhibitor in a range from about 0.1 to about 50. In certain embodiments, a pharmaceutical composition provided herein has a weight ratio of the bile acid compound over the CYP450 inhibitor in a range from about 0.1 to about 25. In certain embodiments, a pharmaceutical composition provided herein has a weight ratio of the bile acid compound over the CYP450 inhibitor in a range from about 0.2 to about 10. In certain embodiments, a pharmaceutical composition provided herein has a weight ratio of the bile acid compound over the CYP450 inhibitor in a range from about 0.5 to about 5. In certain embodiments, a pharmaceutical composition provided herein has a weight ratio of the bile acid compound over the CYP450 inhibitor in a range from about 0.5 to about 3.

In certain embodiments, a pharmaceutical composition provided herein comprises from about 0.1 to about 20%, from about 0.2 to about 15%, from about 0.5 to about 15%, from about 1 to about 15%, from about 0.5 to about 10%, from about 1 to about 10%, or from about 0.5 to about 5% by weight of the gastrointestinal enzyme inhibitor. In certain embodiments, a pharmaceutical composition provided herein comprises from about 0.1 to about 20% by weight of the gastrointestinal enzyme inhibitor. In certain embodiments, a pharmaceutical composition provided herein comprises from about 0.2 to about 15% by weight of the gastrointestinal enzyme inhibitor. In certain embodiments, a pharmaceutical composition provided herein comprises from about 0.5 to about 15% by weight of the gastrointestinal enzyme inhibitor. In certain embodiments, a pharmaceutical composition provided herein comprises from about 1 to about 15% by weight of the gastrointestinal enzyme inhibitor. In certain embodiments, a pharmaceutical composition provided herein comprises from about 0.5 to about 10% by weight of the gastrointestinal enzyme inhibitor. In certain embodiments, a pharmaceutical composition provided herein comprises from about 1 to about 10% by weight of the gastrointestinal enzyme inhibitor. In certain embodiments, a pharmaceutical composition provided herein comprises from about 0.5 to about 5% by weight of the gastrointestinal enzyme inhibitor.

In certain embodiments, a pharmaceutical composition provided herein comprises from about 1 to about 100 mg, from about 1 to about 50 mg, from about 1 to about 30 mg, from about 1 to about 20 mg, from about 2 to about 20 mg, or from about 2 to about 10 mg of the gastrointestinal enzyme inhibitor. In certain embodiments, a pharmaceutical composition provided herein comprises from about 1 to about 100 mg. of the gastrointestinal enzyme inhibitor. In certain embodiments, a pharmaceutical composition provided herein comprises from about 1 to about 50 mg. of the gastrointestinal enzyme inhibitor. In certain embodiments, a pharmaceutical composition provided herein comprises from about 1 to about 30 mg. of the gastrointestinal enzyme inhibitor. In certain embodiments, a pharmaceutical composition provided herein comprises from about 1 to about 20 mg. of the gastrointestinal enzyme inhibitor. In certain embodiments, a pharmaceutical composition provided herein comprises from about 2 to about 20 mg of the gastrointestinal enzyme inhibitor. In certain embodiments, a pharmaceutical composition provided herein comprises from about 2 to about 10 mg of the gastrointestinal enzyme inhibitor.

In certain embodiments, the gastrointestinal enzyme inhibitor is a protease inhibitor. In certain embodiments, the gastrointestinal enzyme inhibitor is a trypsin inhibitor. In certain embodiments, the gastrointestinal enzyme inhibitor is amastatin, aprotinin, betastatin, captopril, chemostatin, chymostatin, pepstatin, phosphoramidon, or soybean trypsin inhibitor (SBTI). In certain embodiments, the gastrointestinal enzyme inhibitor is soybean trypsin inhibitor.

In certain embodiments, a pharmaceutical composition provided herein comprises from about 5 to about 50%, from about 10 to about 50%, from about 10 to about 40%, or from about 15 to about 30% by weight of the absorption enhancer. In certain embodiments, a pharmaceutical composition provided herein comprises from about 5 to about 50% by weight of the absorption enhancer. In certain embodiments, a pharmaceutical composition provided herein comprises from about 10 to about 50% by weight of the absorption enhancer. In certain embodiments, a pharmaceutical composition provided herein comprises from about 10 to about 40% by weight of the absorption enhancer. In certain embodiments, a pharmaceutical composition provided herein comprises from about 15 to about 30% by weight of the absorption enhancer.

In certain embodiments, a pharmaceutical composition provided herein comprises from about 10 to about 500 mg, from about 25 to about 250 mg, from about 50 to about 250 mg, or from about 100 to about 250 mg of the absorption enhancer. In certain embodiments, a pharmaceutical composition provided herein comprises from about 10 to about 500 mg of the absorption enhancer. In certain embodiments, a pharmaceutical composition provided herein comprises from about 25 to about 250 mg of the absorption enhancer. In certain embodiments, a pharmaceutical composition provided herein comprises from about 50 to about 250 mg of the absorption enhancer. In certain embodiments, a pharmaceutical composition provided herein comprises from about 100 to about 250 mg of the absorption enhancer.

In certain embodiments, the absorption enhancer is *N*-(4-chlorosalicyloyl)-4-aminobutyric acid, *N*-(8-[2-hydroxybenzoyl]-amino)caprylic acid, 8-(*N*-2-hydroxy-5-chlorobenzoyl)aminocaprylic acid, salcaprozic acid, or a pharmaceutically acceptable salt thereof. In certain embodiments, the absorption enhancer is sodium *N*-(4-chlorosalicyloyl)-4-aminobutyrate (4-CNAB), sodium *N*-(8-[2-hydroxybenzoyl]amino)caprylate (SNAC), sodium 8-(*N*-2-hydroxy-5-chlorobenzoyl)aminocaprylate (5-CNAC), or salcaprozate sodium. In certain embodiments, the absorption enhancer is 4-CNAB. In certain embodiments, the absorption enhancer is SNAC. In certain embodiments, the absorption enhancer is 5-CNAC. In certain embodiments, the absorption enhancer is salcaprozate sodium.

In certain embodiments, the absorption enhancer is a medium-chain fatty acid or a pharmaceutically acceptable salt thereof. In certain embodiments, the absorption enhancer is caprylate or caprylic acid, or a pharmaceutically acceptable salt thereof. In certain embodiments, the absorption enhancer is sodium caprylate (C8) or sodium caprate (C10). In certain embodiments, the absorption enhancer is C8. In certain embodiments, the absorption enhancer is C10.

In certain embodiments, a pharmaceutical composition provided herein comprises from about 0.1 to about 10%, from about 0.2 to about 5%, from about 0.5 to about 5%, from about 1 to about 5%, or from about 2 to about 5% by weight of the disintegrant. In certain embodiments, a pharmaceutical composition provided herein comprises from about 0.1 to about 10% by weight of the disintegrant. In certain embodiments, a pharmaceutical composition provided herein comprises from about 0.2 to about 5% by weight of the disintegrant. In certain embodiments, a pharmaceutical composition provided herein comprises from about 0.5 to about 5% by weight of the disintegrant. In certain embodiments, a pharmaceutical composition provided herein comprises from about 1 to about 5% by weight of the disintegrant. In certain embodiments, a pharmaceutical composition provided herein comprises the disintegrant in amount ranging from about 2 to about 5% by weight.

In certain embodiments, a pharmaceutical composition provided herein comprises from about 1 to about 100 mg, from about 2 to about 50 mg, from about 5 to about 25 mg, or from about 10 to about 20 mg of the disintegrant. In certain embodiments, a pharmaceutical composition provided herein comprises from about 1 to about 100 mg of the disintegrant. In certain embodiments, a pharmaceutical composition provided herein comprises from about 2 to about 50 mg of the disintegrant. In certain embodiments, a pharmaceutical composition provided herein comprises from about 5 to about 25 mg of the disintegrant. In certain embodiments, a pharmaceutical composition provided herein comprises from about 10 to about 20 mg of the disintegrant.

In certain embodiments, the disintegrant is alginic acid, calcium silicate, croscarmellose sodium, crospovidone, low-substituted hydroxypropyl cellulose, microcrystalline cellulose (MCC), polacrilin potassium, pregelatinized starch, sodium starch glycolate, or soy polysaccharide. In certain embodiments, the disintegrant is a cross-linked polyvinylpyrrolidone. In certain embodiments, the disintegrant is crospovidone.

In certain embodiments, a pharmaceutical composition provided herein comprises from about 1 to about 50%, from about 1 to about 40%, from about 1 to about 35%, from about 2 to about 30%, or from about 5 to about 15% by weight of the filler. In certain embodiments, a pharmaceutical composition provided herein comprises from about 1 to about 50% by weight of the filler. In certain embodiments, a pharmaceutical composition provided herein comprises from about 1 to about 40% by weight of the filler. In certain embodiments, a pharmaceutical composition provided herein comprises from about 1 to about 35% by weight of the filler. In certain embodiments, a pharmaceutical composition provided herein comprises from about 2 to about 30% by weight of the filler. In certain embodiments, a pharmaceutical composition provided herein comprises from about 5 to about 15% by weight of the filler.

In certain embodiments, a pharmaceutical composition provided herein comprises from about 1 to about 500 mg, from about 2 to about 250 mg, from about 5 to about 200 mg, or from about 5 to about 150 mg of the filler. In certain embodiments, a pharmaceutical composition provided herein comprises from about 1 to about 500 mg of the filler. In certain embodiments, a pharmaceutical composition provided herein comprises from about 2 to about 250 mg of the filler. In certain embodiments, a pharmaceutical composition provided herein comprises from about 5 to about 200 mg of the filler. In certain embodiments, a pharmaceutical composition provided herein comprises from about 5 to about 150 mg of the filler.

In certain embodiments, the filler is a sugar. In certain embodiments, the filler is dextrose, fructose, glucose, lactose, maltose, starch, sucrose, trehalose, or a mixture thereof. In certain embodiments, the filler is a sugar alcohol. In certain embodiments, the filler is arabitol, erythritol, fucitol, galactitol, iditol, inositol, isomalt, lactitol, maltitol, maltotritol, mannitol, ribitol, sorbitol, threitol, volemitol, xylitol, or a mixture thereof. In certain embodiments, the filler is erythritol, lactitol, maltitol, mannitol, sorbitol, xylitol, or a mixture thereof. In certain embodiments, the filler is arabitol. In certain embodiments, the filler is erythritol. In certain embodiments, the filler is fucitol. In certain embodiments, the filler is galactitol. In certain embodiments, the filler is iditol. In certain embodiments, the filler is inositol. In certain embodiments, the filler is isomalt. In certain embodiments, the filler is lactitol. In certain embodiments, the filler is maltitol. In certain embodiments, the filler is maltotritol. In certain embodiments, the filler is mannitol. In certain embodiments, the filler is D-mannitol. In certain embodiments, the filler is ribitol. In certain embodiments, the filler is sorbitol. In certain embodiments, the filler is threitol. In certain embodiments, the filler is volemitol. In certain embodiments, the filler is xylitol.

In certain embodiments, a pharmaceutical composition provided herein comprises from about 0.1 to about 5%, from about 0.1 to about 2%, from about 0.2 to about 1%, or from about 0.5 to about 1% by weight of the lubricant. In certain embodiments, a pharmaceutical composition provided herein comprises from about 0.1 to about 5% by weight of the lubricant. In certain embodiments, a pharmaceutical composition provided herein comprises from about 0.1 to about 2% by weight of the lubricant. In certain embodiments, a pharmaceutical composition provided herein comprises from about 0.2 to about 1% by weight of the lubricant. In certain embodiments, a pharmaceutical composition provided herein comprises from about 0.5 to about 1% by weight of the lubricant.

In certain embodiments, a pharmaceutical composition provided herein comprises from about 1 to about 20 mg, from about 1 to about 10 mg, or from about 1 to about 5 mg of the lubricant. In certain embodiments, a pharmaceutical composition provided herein comprises from about 1 to about 20 mg of the lubricant. In certain embodiments, a pharmaceutical composition provided herein comprises from about 1 to about 10 mg of the lubricant. In certain embodiments, a pharmaceutical composition provided herein comprises from about 1 to about 5 mg of the lubricant.

In certain embodiments, the lubricant is magnesium stearate, stearic acid, calcium stearate, sodium stearyl fumarate, polyethylene glycols, colloidal silicon dioxide, talc, beeswax, or hydrogenated vegetable oil. In certain embodiments, the lubricant is magnesium stearate.

In certain embodiments, a pharmaceutical composition provided herein comprises from about 0.1 to about 5%, from about 0.1 to about 2%, from about 0.2 to about 1%, or from about 0.5 to about 1% by weight of the glidant. In certain embodiments, a pharmaceutical composition provided herein comprises from about 0.1 to about 5% by weight of the glidant. In certain embodiments, a pharmaceutical composition provided herein comprises from about 0.1 to about 2% by weight of the glidant. In certain embodiments, a pharmaceutical composition provided herein comprises from about 0.2 to about 1% by weight of the glidant. In certain embodiments, a pharmaceutical composition provided herein comprises from about 0.5 to about 1% by weight of the glidant.

In certain embodiments, a pharmaceutical composition provided herein comprises from about 1 to about 20 mg, from about 1 to about 10 mg, or from about 1 to about 5 mg of the glidant. In certain embodiments, a pharmaceutical composition provided herein comprises from about 1 to about 20 mg of the glidant. In certain embodiments, a pharmaceutical composition provided herein comprises from about 1 to about 10 mg of the glidant. In certain embodiments, a pharmaceutical composition provided herein comprises from about 1 to about 5 mg of the glidant.

In certain embodiments, the glidant is silicon dioxide, CAB-O-SIL^{®}, or asbestos-free talc. In certain embodiments, the glidant is silicon dioxide.

In one embodiment, provided herein is an oral pharmaceutical composition comprising from about 0.1 to about 10% by weight of the therapeutic polypeptide, from about 5 to about 65% by weight of the bile acid compound, from about 10 to about 50% by weight of the CYP450 inhibitor, from about 0.2 to about 15% by weight of the gastrointestinal enzyme inhibitor, from about 1 to about 5% by weight of the disintegrant, from about 1 to about 50% by weight of the filler, from about 0.1 to about 5% by weight of the lubricant, and from about 0.1 to about 5% by weight of the glidant.

In another embodiment, provided herein is an oral pharmaceutical composition comprising from about 0.2 to about 5% by weight of the therapeutic polypeptide, from about 10 to about 65% by weight of the bile acid compound, from about 10 to about 50% by weight of the CYP450 inhibitor, from about 0.5 to about 15% by weight of the gastrointestinal enzyme inhibitor, from about 2 to about 5% by weight of the disintegrant, from about 1 to about 50% by weight of the filler, from about 0.1 to about 2% by weight of the lubricant, and from about 0.1 to about 2% by weight of the glidant.

In yet another embodiment, provided herein is an oral pharmaceutical composition comprising from about 0.5 to about 5% by weight of the therapeutic polypeptide, from about 10 to about 50% by weight of the bile acid compound, from about 10 to about 40% by weight of the CYP450 inhibitor, from about 1 to about 15% by weight of the gastrointestinal enzyme inhibitor, from about 2 to about 5% by weight of the disintegrant, from about 1 to about 40% by weight of the filler, from about 0.2 to about 1% by weight of the lubricant, and from about 0.2 to about 1% by weight of the glidant.

In yet another embodiment, provided herein is an oral pharmaceutical composition comprising from about 1 to about 2.5% by weight of the therapeutic polypeptide, from about 15 to about 50% by weight of the bile acid compound, from about 15 to about 35% by weight of the CYP450 inhibitor, from about 1 to about 10% by weight of the gastrointestinal enzyme inhibitor, from about 2 to about 5% by weight of the disintegrant, from about 1 to about 35% by weight of the filler, from about 0.5 to about 1% by weight of the lubricant, and from about 0.5 to about 1% by weight of the glidant.

In yet another embodiment, provided herein is an oral pharmaceutical composition comprising from about 0.5 to about 5% by weight of the therapeutic polypeptide, from about 30 to about 65% by weight of the bile acid compound, from about 20 to about 40% by weight of the CYP450 inhibitor, from about 0.5 to about 10% by weight of the gastrointestinal enzyme inhibitor, from about 2 to about 5% by weight of the disintegrant, from about 2 to about 30% by weight of the filler, from about 0.2 to about 1% by weight of the lubricant, and from about 0.2 to about 1% by weight of the glidant.

In still another embodiment, provided herein is an oral pharmaceutical composition comprising from about 1 to about 2.5% by weight of the therapeutic polypeptide, from about 40 to about 65% by weight of the bile acid compound, from about 20 to about 35% by weight of the CYP450 inhibitor, from about 0.5 to about 5% by weight of the gastrointestinal enzyme inhibitor, from about 2 to about 5% by weight of the disintegrant, from about 5 to about 15% by weight of the filler, from about 0.5 to about 1% by weight of the lubricant, and from about 0.5 to about 1% by weight of the glidant.

In one embodiment, provided herein is an oral pharmaceutical composition comprising from about 0.1 to about 10% by weight of the therapeutic polypeptide, from about 5 to about 65% by weight of the bile acid compound, from about 5 to about 50% by weight of the absorption enhancer, from about 10 to about 50% by weight of the CYP450 inhibitor, from about 0.2 to about 15% by weight of the gastrointestinal enzyme inhibitor, from about 1 to about 5% by weight of the disintegrant, from about 1 to about 50% by weight of the filler, from about 0.1 to about 5% by weight of the lubricant, and from about 0.1 to about 5% by weight of the glidant.

In another embodiment, provided herein is an oral pharmaceutical composition comprising from about 0.2 to about 5% by weight of the therapeutic polypeptide, from about 10 to about 50% by weight of the bile acid compound, from about 10 to about 50% by weight of the absorption enhancer, from about 10 to about 50% by weight of the CYP450 inhibitor, from about 0.5 to about 15% by weight of the gastrointestinal enzyme inhibitor, from about 2 to about 5% by weight of the disintegrant, from about 1 to about 50% by weight of the filler, from about 0.1 to about 2% by weight of the lubricant, and from about 0.1 to about 2% by weight of the glidant.

In yet another embodiment, provided herein is an oral pharmaceutical composition comprising from about 0.5 to about 5% by weight of the therapeutic polypeptide, from about 10 to about 50% by weight of the bile acid compound, from about 10 to about 40% by weight of the absorption enhancer, from about 10 to about 40% by weight of the CYP450 inhibitor, from about 1 to about 15% by weight of the gastrointestinal enzyme inhibitor, from about 2 to about 5% by weight of the disintegrant, from about 1 to about 40% by weight of the filler, from about 0.2 to about 1% by weight of the lubricant, and from about 0.2 to about 1% by weight of the glidant.

In still another embodiment, provided herein is an oral pharmaceutical composition comprising from about 1 to about 2.5% by weight of the therapeutic polypeptide, from about 15 to about 50% by weight of the bile acid compound, from about 15 to about 30% by weight of the absorption enhancer, from about 15 to about 30% by weight of the CYP450 inhibitor, from about 1 to about 10% by weight of the gastrointestinal enzyme inhibitor, from about 2 to about 5% by weight of the disintegrant, from about 1 to about 35% by weight of the filler, from about 0.5 to about 1% by weight of the lubricant, and from about 0.5 to about 1% by weight of the glidant.

In one embodiment, provided herein is an oral pharmaceutical composition comprising from about 0.2 to about 5% by weight of semaglutide, from about 10 to about 65% by weight of CA, from about 10 to about 50% by weight of PG, from about 0.5 to about 15% by weight of SBTI, from about 2 to about 5% by weight of crospovidone, from about 1 to about 50% by weight of mannitol, from about 0.1 to about 2% by weight of magnesium stearate, and from about 0.1 to about 2% by weight of magnesium stearate.

In another embodiment, provided herein is an oral pharmaceutical composition comprising from about 0.5 to about 5% by weight of semaglutide, from about 10 to about 50% by weight of CA, from about 10 to about 40% by weight of PG, from about 1 to about 15% by weight of SBTI, from about 2 to about 5% by weight of crospovidone, from about 1 to about 40% by weight of mannitol, from about 0.2 to about 1% by weight of magnesium stearate, and from about 0.2 to about 1% by weight of magnesium stearate.

In yet another embodiment, provided herein is an oral pharmaceutical composition comprising from about 1 to about 2.5% by weight of semaglutide, from about 15 to about 50% by weight of CA, from about 15 to about 35% by weight of PG, from about 1 to about 10% by weight of SBTI, from about 2 to about 5% by weight of crospovidone, from about 1 to about 35% by weight of mannitol, from about 0.5 to about 1% by weight of magnesium stearate, and from about 0.5 to about 1% by weight of magnesium stearate.

In one embodiment, provided herein is an oral pharmaceutical composition comprising about 2.5% by weight of semaglutide, about 28% by weight of CA, about 34% by weight of PG, about 5% by weight of SBTI, about 3.5% by weight of crospovidone, about 25% by weight of mannitol, about 0.8% by weight of magnesium stearate, and about 0.8% by weight of magnesium stearate.

In one embodiment, provided herein is an oral pharmaceutical composition comprising from about 0.2 to about 5% by weight of semaglutide, from about 10 to about 65% by weight of DCA, from about 10 to about 50% by weight of PG, from about 0.5 to about 15% by weight of SBTI, from about 2 to about 5% by weight of crospovidone, from about 1 to about 50% by weight of mannitol, from about 0.1 to about 2% by weight of magnesium stearate, and from about 0.1 to about 2% by weight of magnesium stearate.

In another embodiment, provided herein is an oral pharmaceutical composition comprising from about 0.5 to about 5% by weight of semaglutide, from about 10 to about 50% by weight of DCA, from about 10 to about 40% by weight of PG, from about 1 to about 15% by weight of SBTI, from about 2 to about 5% by weight of crospovidone, from about 1 to about 40% by weight of mannitol, from about 0.2 to about 1% by weight of magnesium stearate, and from about 0.2 to about 1% by weight of magnesium stearate.

In yet another embodiment, provided herein is an oral pharmaceutical composition comprising from about 1 to about 2.5% by weight of semaglutide, from about 15 to about 50% by weight of DCA, from about 15 to about 35% by weight of PG, from about 1 to about 10% by weight of SBTI, from about 2 to about 5% by weight of crospovidone, from about 1 to about 35% by weight of mannitol, from about 0.5 to about 1% by weight of magnesium stearate, and from about 0.5 to about 1% by weight of magnesium stearate.

In one embodiment, provided herein is an oral pharmaceutical composition comprising about 2.5% by weight of semaglutide, about 29% by weight of DCA, about 34% by weight of PG, about 1.7% by weight of SBTI, about 3.5% by weight of crospovidone, about 28% by weight of mannitol, about 0.8% by weight of magnesium stearate, and about 0.8% by weight of magnesium stearate.

In another embodiment, provided herein is an oral pharmaceutical composition comprising about 2.5% by weight of semaglutide, about 43% by weight of DCA, about 34% by weight of PG, about 3.5% by weight of SBTI, about 3.5% by weight of crospovidone, about 12.5% by weight of mannitol, about 0.8% by weight of magnesium stearate, and about 0.8% by weight of magnesium stearate.

In one embodiment, provided herein is an oral pharmaceutical composition comprising from about 0.2 to about 5% by weight of semaglutide, from about 10 to about 65% by weight of GCA, from about 10 to about 50% by weight of PG, from about 0.5 to about 15% by weight of SBTI, from about 2 to about 5% by weight of crospovidone, from about 1 to about 50% by weight of mannitol, from about 0.1 to about 2% by weight of magnesium stearate, and from about 0.1 to about 2% by weight of magnesium stearate.

In another embodiment, provided herein is an oral pharmaceutical composition comprising from about 0.5 to about 5% by weight of semaglutide, from about 10 to about 50% by weight of GCA, from about 10 to about 40% by weight of PG, from about 1 to about 15% by weight of SBTI, from about 2 to about 5% by weight of crospovidone, from about 1 to about 40% by weight of mannitol, from about 0.2 to about 1% by weight of magnesium stearate, and from about 0.2 to about 1% by weight of magnesium stearate.

In yet another embodiment, provided herein is an oral pharmaceutical composition comprising from about 1 to about 2.5% by weight of semaglutide, from about 15 to about 50% by weight of GCA, from about 15 to about 35% by weight of PG, from about 1 to about 10% by weight of SBTI, from about 2 to about 5% by weight of crospovidone, from about 1 to about 35% by weight of mannitol, from about 0.5 to about 1% by weight of magnesium stearate, and from about 0.5 to about 1% by weight of magnesium stearate.

In one embodiment, provided herein is an oral pharmaceutical composition comprising about 2.5% by weight of semaglutide, about 17% by weight of GCA, about 34% by weight of PG, about 8.5% by weight of SBTI, about 3.5% by weight of crospovidone, about 33% by weight of mannitol, about 0.8% by weight of magnesium stearate, and about 0.8% by weight of magnesium stearate.

In another embodiment, provided herein is an oral pharmaceutical composition comprising about 2.5% by weight of semaglutide, about 17% by weight of GCA, about 34% by weight of PG, about 13.5% by weight of SBTI, about 3.5% by weight of crospovidone, about 28% by weight of mannitol, about 0.8% by weight of magnesium stearate, and about 0.8% by weight of magnesium stearate.

In yet another embodiment, provided herein is an oral pharmaceutical composition comprising about 2.5% by weight of semaglutide, about 51% by weight of GCA, about 34% by weight of PG, about 6% by weight of SBTI, about 3.5% by weight of crospovidone, about 2% by weight of mannitol, about 0.8% by weight of magnesium stearate, and about 0.8% by weight of magnesium stearate.

In yet another embodiment, provided herein is an oral pharmaceutical composition comprising about 1.5% by weight of semaglutide, about 33% by weight of GCA, about 22% by weight of PG, about 13.5% by weight of SBTI, about 3.5% by weight of crospovidone, about 25% by weight of mannitol, about 1% by weight of magnesium stearate, and about 1% by weight of magnesium stearate.

In still another embodiment, provided herein is an oral pharmaceutical composition comprising about 1.5% by weight of semaglutide, about 45% by weight of GCA, about 18% by weight of PG, about 3.2% by weight of SBTI, about 3.2% by weight of crospovidone, about 27% by weight of mannitol, about 1% by weight of magnesium stearate, and about 1% by weight of magnesium stearate.

In one embodiment, provided herein is an oral pharmaceutical composition comprising from about 0.2 to about 5% by weight of semaglutide, from about 10 to about 50% by weight of GCA, from about 10 to about 50% by weight of C10, from about 10 to about 50% by weight of PG, from about 0.5 to about 15% by weight of SBTI, from about 2 to about 5% by weight of crospovidone, from about 1 to about 50% by weight of mannitol, from about 0.1 to about 2% by weight of magnesium stearate, and from about 0.1 to about 2% by weight of magnesium stearate.

In another embodiment, provided herein is an oral pharmaceutical composition comprising from about 0.2 to about 5% by weight of semaglutide, from about 10 to about 50% by weight of GCA, from about 10 to about 40% by weight of C10, from about 10 to about 40% by weight of PG, from about 1 to about 15% by weight of SBTI, from about 2 to about 5% by weight of crospovidone, from about 1 to about 40% by weight of mannitol, from about 0.2 to about 1% by weight of magnesium stearate, and from about 0.2 to about 1% by weight of magnesium stearate.

In yet another embodiment, provided herein is an oral pharmaceutical composition comprising from about 1 to about 2.5% by weight of semaglutide, from about 15 to about 50% by weight of GCA, from about 15 to about 30% by weight of C10, from about 10 to about 30% by weight of PG, from about 1 to about 10% by weight of SBTI, from about 2 to about 5% by weight of crospovidone, from about 1 to about 35% by weight of mannitol, from about 0.5 to about 1% by weight of magnesium stearate, and from about 0.5 to about 1% by weight of magnesium stearate.

In one embodiment, provided herein is an oral pharmaceutical composition comprising about 1.5% by weight of semaglutide, about 45% by weight of GCA, about 25% by weight of C10, about 18% by weight of PG, about 3.2% by weight of SBTI, about 3.2% by weight of crospovidone, about 1.3% by weight of mannitol, about 1% by weight of magnesium stearate, and about 1% by weight of magnesium stearate.

In one embodiment, provided herein is an oral pharmaceutical composition comprising from about 0.2 to about 5% by weight of semaglutide, from about 10 to about 65% by weight of KGCA, from about 10 to about 50% by weight of PG, from about 0.5 to about 15% by weight of SBTI, from about 2 to about 5% by weight of crospovidone, from about 1 to about 50% by weight of mannitol, from about 0.1 to about 2% by weight of magnesium stearate, and from about 0.1 to about 2% by weight of magnesium stearate.

In another embodiment, provided herein is an oral pharmaceutical composition comprising from about 0.2 to about 5% by weight of semaglutide, from about 30 to about 65% by weight of KGCA, from about 20 to about 40% by weight of PG, from about 0.5 to about 10% by weight of SBTI, from about 2 to about 5% by weight of crospovidone, from about 2 to about 30% by weight of mannitol, from about 0.2 to about 1% by weight of magnesium stearate, and from about 0.2 to about 1% by weight of magnesium stearate.

In yet another embodiment, provided herein is an oral pharmaceutical composition comprising from about 1 to about 2.5% by weight of semaglutide, from about 40 to about 65% by weight of KGCA, from about 20 to about 35% by weight of PG, from about 0.5 to about 5% by weight of SBTI, from about 2 to about 5% by weight of crospovidone, from about 5 to about 15% by weight of mannitol, from about 0.5 to about 1% by weight of magnesium stearate, and from about 0.5 to about 1% by weight of magnesium stearate.

In one embodiment, provided herein is an oral pharmaceutical composition comprising about 2.5% by weight of semaglutide, about 40% by weight of KGCA, about 35% by weight of PG, about 5% by weight of SBTI, about 3.5% by weight of crospovidone, about 13% by weight of mannitol, about 0.8% by weight of magnesium stearate, and about 0.8% by weight of magnesium stearate.

In another embodiment, provided herein is an oral pharmaceutical composition comprising about 2% by weight of semaglutide, about 52% by weight of KGCA, about 29% by weight of PG, about 1.5% by weight of SBTI, about 3% by weight of crospovidone, about 12% by weight of mannitol, about 0.7% by weight of magnesium stearate, and about 0.7% by weight of magnesium stearate.

In yet another embodiment, provided herein is an oral pharmaceutical composition comprising about 2.5% by weight of semaglutide, about 31% by weight of KGCA, about 34% by weight of PG, about 0.8% by weight of SBTI, about 3.5% by weight of crospovidone, about 27% by weight of mannitol, about 0.8% by weight of magnesium stearate, and about 0.8% by weight of magnesium stearate.

In yet another embodiment, provided herein is an oral pharmaceutical composition comprising about 2.5% by weight of semaglutide, about 31% by weight of KGCA, about 34% by weight of PG, about 1.7% by weight of SBTI, about 3.5% by weight of crospovidone, about 26% by weight of mannitol, about 0.8% by weight of magnesium stearate, and about 0.8% by weight of magnesium stearate.

In yet another embodiment, provided herein is an oral pharmaceutical composition comprising about 2.5% by weight of semaglutide, about 31% by weight of KGCA, about 34% by weight of PG, about 5% by weight of SBTI, about 3.5% by weight of crospovidone, about 23% by weight of mannitol, about 0.8% by weight of magnesium stearate, and about 0.8% by weight of magnesium stearate.

In yet another embodiment, provided herein is an oral pharmaceutical composition comprising about 2% by weight of semaglutide, about 52% by weight of KGCA, about 29% by weight of PG, about 0.7% by weight of SBTI, about 3% by weight of crospovidone, about 13% by weight of mannitol, about 0.7% by weight of magnesium stearate, and about 0.7% by weight of magnesium stearate.

In yet another embodiment, provided herein is an oral pharmaceutical composition comprising about 1.6% by weight of semaglutide, about 61% by weight of KGCA, about 23% by weight of PG, about 0.6% by weight of SBTI, about 3.5% by weight of crospovidone, about 9% by weight of mannitol, about 0.8% by weight of magnesium stearate, and about 0.8% by weight of magnesium stearate.

In yet another embodiment, provided herein is an oral pharmaceutical composition comprising about 1.6% by weight of semaglutide, about 61% by weight of KGCA, about 23% by weight of PG, about 1.1% by weight of SBTI, about 3.5% by weight of crospovidone, about 8.5% by weight of mannitol, about 0.8% by weight of magnesium stearate, and about 0.8% by weight of magnesium stearate.

In still another embodiment, provided herein is an oral pharmaceutical composition comprising about 1.6% by weight of semaglutide, about 61% by weight of KGCA, about 23% by weight of PG, about 3.5% by weight of SBTI, about 3.5% by weight of crospovidone, about 6% by weight of mannitol, about 0.8% by weight of magnesium stearate, and about 0.8% by weight of magnesium stearate.

In one embodiment, provided herein is an oral pharmaceutical composition comprising from about 0.2 to about 5% by weight of tirzepatide, from about 10 to about 65% by weight of CDCA, from about 10 to about 50% by weight of PG, from about 0.5 to about 15% by weight of SBTI, from about 2 to about 5% by weight of crospovidone, from about 1 to about 50% by weight of mannitol, from about 0.1 to about 2% by weight of magnesium stearate, and from about 0.1 to about 2% by weight of magnesium stearate.

In another embodiment, provided herein is an oral pharmaceutical composition comprising from about 0.5 to about 5% by weight of tirzepatide, from about 10 to about 50% by weight of CDCA, from about 10 to about 40% by weight of PG, from about 1 to about 15% by weight of SBTI, from about 2 to about 5% by weight of crospovidone, from about 1 to about 50% by weight of mannitol, from about 0.2 to about 1% by weight of magnesium stearate, and from about 0.2 to about 1% by weight of magnesium stearate.

In yet another embodiment, provided herein is an oral pharmaceutical composition comprising from about 1 to about 2.5% by weight of tirzepatide, from about 15 to about 50% by weight of CDCA, from about 15 to about 35% by weight of PG, from about 1 to about 10% by weight of SBTI, from about 2 to about 5% by weight of crospovidone, from about 1 to about 40% by weight of mannitol, from about 0.5 to about 1% by weight of magnesium stearate, and from about 0.5 to about 1% by weight of magnesium stearate.

In one embodiment, provided herein is an oral pharmaceutical composition comprising about 2.5% by weight of tirzepatide, about 14% by weight of CDCA, about 34% by weight of PG, about 3.5% by weight of SBTI, about 3.5% by weight of crospovidone, about 41% by weight of mannitol, about 0.8% by weight of magnesium stearate, and about 0.8% by weight of magnesium stearate.

In one embodiment, provided herein is an oral pharmaceutical composition comprising about 2.5% by weight of tirzepatide, about 43% by weight of CDCA, about 34% by weight of PG, about 5% by weight of SBTI, about 3.5% by weight of crospovidone, about 11% by weight of mannitol, about 0.8% by weight of magnesium stearate, and about 0.8% by weight of magnesium stearate.

In one embodiment, provided herein is an oral pharmaceutical composition comprising from about 0.2 to about 5% by weight of tirzepatide, from about 10 to about 65% by weight of GCA, from about 10 to about 50% by weight of PG, from about 0.5 to about 15% by weight of SBTI, from about 2 to about 5% by weight of crospovidone, from about 1 to about 50% by weight of mannitol, from about 0.1 to about 2% by weight of magnesium stearate, and from about 0.1 to about 2% by weight of magnesium stearate.

In another embodiment, provided herein is an oral pharmaceutical composition comprising from about 0.5 to about 5% by weight of tirzepatide, from about 10 to about 50% by weight of GCA, from about 10 to about 40% by weight of PG, from about 1 to about 15% by weight of SBTI, from about 2 to about 5% by weight of crospovidone, from about 1 to about 50% by weight of mannitol, from about 0.2 to about 1% by weight of magnesium stearate, and from about 0.2 to about 1% by weight of magnesium stearate.

In yet another embodiment, provided herein is an oral pharmaceutical composition comprising from about 1 to about 2.5% by weight of tirzepatide, from about 15 to about 50% by weight of GCA, from about 15 to about 35% by weight of PG, from about 1 to about 10% by weight of SBTI, from about 2 to about 5% by weight of crospovidone, from about 1 to about 40% by weight of mannitol, from about 0.5 to about 1% by weight of magnesium stearate, and from about 0.5 to about 1% by weight of magnesium stearate.

In one embodiment, provided herein is an oral pharmaceutical composition comprising about 2.5% by weight of tirzepatide, about 17% by weight of GCA, about 34% by weight of PG, about 8.5% by weight of SBTI, about 3.5% by weight of crospovidone, about 33% by weight of mannitol, about 0.8% by weight of magnesium stearate, and about 0.8% by weight of magnesium stearate.

In another embodiment, provided herein is an oral pharmaceutical composition comprising about 2.5% by weight of tirzepatide, about 51% by weight of GCA, about 34% by weight of PG, about 1.7% by weight of SBTI, about 3.5% by weight of crospovidone, about 6% by weight of mannitol, about 0.8% by weight of magnesium stearate, and about 0.8% by weight of magnesium stearate.

In one embodiment, provided herein is an oral pharmaceutical composition comprising from about 0.2 to about 5% by weight of tirzepatide, from about 10 to about 65% by weight of KGCA, from about 10 to about 50% by weight of PG, from about 0.5 to about 15% by weight of SBTI, from about 2 to about 5% by weight of crospovidone, from about 1 to about 50% by weight of mannitol, from about 0.1 to about 2% by weight of magnesium stearate, and from about 0.1 to about 2% by weight of magnesium stearate.

In another embodiment, provided herein is an oral pharmaceutical composition comprising from about 0.5 to about 5% by weight of tirzepatide, from about 10 to about 50% by weight of KGCA, from about 10 to about 40% by weight of PG, from about 1 to about 15% by weight of SBTI, from about 2 to about 5% by weight of crospovidone, from about 1 to about 50% by weight of mannitol, from about 0.2 to about 1% by weight of magnesium stearate, and from about 0.2 to about 1% by weight of magnesium stearate.

In yet another embodiment, provided herein is an oral pharmaceutical composition comprising from about 1 to about 2.5% by weight of tirzepatide, from about 15 to about 50% by weight of KGCA, from about 15 to about 35% by weight of PG, from about 1 to about 10% by weight of SBTI, from about 2 to about 5% by weight of crospovidone, from about 1 to about 40% by weight of mannitol, from about 0.5 to about 1% by weight of magnesium stearate, and from about 0.5 to about 1% by weight of magnesium stearate.

In one embodiment, provided herein is an oral pharmaceutical composition comprising about 2.5% by weight of tirzepatide, about 20% by weight of KGCA, about 34% by weight of PG, about 3.5% by weight of SBTI, about 3.5% by weight of crospovidone, about 35% by weight of mannitol, about 0.8% by weight of magnesium stearate, and about 0.8% by weight of magnesium stearate.

In certain embodiments, a pharmaceutical composition provided herein is solid. In certain embodiments, a pharmaceutical composition provided herein is formulated as a capsule. In certain embodiments, a pharmaceutical composition provided herein is formulated as a tablet.

A pharmaceutical composition provided herein can be formulated as modified release dosage forms, including delayed-, extended-, prolonged-, sustained-, pulsatile-, controlled-, accelerated-, fast-, targeted-, programmed-release, and gastric retention dosage forms.

In certain embodiments, a pharmaceutical composition provided herein is enteric coated, thus comprising an enteric coating.

In certain embodiments, provided herein is an enteric coated composition comprising (i) a core comprising a pharmaceutical composition provided herein and (ii) an enteric coating.

In certain embodiments, the enteric coating is a single-layer coating. In certain embodiments, the enteric coating is a dual-layer coating. In certain embodiments, the enteric coating is a triple-layer coating.

In certain embodiments, the enteric coating of an enteric coated pharmaceutical composition provided herein is in an amount ranging from about 0.1 to about 10%, from about 0.2 to about 8%, or from about 0.5 to about 5% of the weight of the core. In certain embodiments, the enteric coating of an enteric coated pharmaceutical composition provided herein is in an amount ranging from about 0.1 to about 10% of the weight of the core. In certain embodiments, the enteric coating of an enteric coated pharmaceutical composition provided herein is in an amount ranging from about 0.2 to about 8% of the weight of the core. In certain embodiments, the enteric coating of an enteric coated pharmaceutical composition provided herein is in an amount ranging from about 0.5 to about 5% of the weight of the core.

In certain embodiments, the enteric coating comprises cellulose acetate phthalate (CAP), cellulose acetate succinate, cellulose acetate trimellitate, diethyl phthalate, hydroxypropyl methylcellulose (HPMC), hydroxypropyl methylcellulose acetate succinate (HPMCAS), hydroxypropyl methylcellulose phthalate (HPMCP), a polyethylene glycol, polyvinyl acetate phthalate (PVAP), a polyacrylic acid, a polymethacrylate, shellac, or sodium alginate, or a mixture of two or more thereof. In certain embodiments, the enteric coating comprises CAP, cellulose acetate succinate, cellulose acetate trimellitate, diethyl phthalate, EUDRAGIT L100, EUDRAGIT S100, EUDRAGIT L30D-55, HPMC, HPMCAS, HPMCP, PEG 400, PVAP, a polyacrylic acid, shellac, or sodium alginate, or a mixture of two or more thereof. In certain embodiments, the enteric coating comprises EUDRAGIT S100, HPMC, and PEG 400.

In certain embodiments, a pharmaceutical composition provided herein has a bioavailability of no less than about 1%, no less than about 2%, no less than about 5%, no less than about 10%, no less than about 15%, no less than about 20%, or no less than about 25%. In certain embodiments, a pharmaceutical composition provided herein has a bioavailability of no less than about 1%. In certain embodiments, a pharmaceutical composition provided herein has a bioavailability of no less than about 2%. In certain embodiments, a pharmaceutical composition provided herein has a bioavailability of no less than about 5%. In certain embodiments, a pharmaceutical composition provided herein has a bioavailability of no less than about 10%. In certain embodiments, a pharmaceutical composition provided herein has a bioavailability of no less than about 15%. In certain embodiments, a pharmaceutical composition provided herein has a bioavailability of no less than about 20%. In certain embodiments, a pharmaceutical composition provided herein has a bioavailability of no less than about 25%.

### Methods of Use

In one embodiment, provided herein is a method of treating diabetes and/or obesity in a subject, comprising administering to the subject in need thereof a therapeutically effective amount of an oral pharmaceutical composition provided herein.

In another embodiment, provided herein is a method of treating diabetes in a subject, comprising administering to the subject in need thereof a therapeutically effective amount of an oral pharmaceutical composition provided herein.

In certain embodiments, the diabetes is type 2 diabetes mellitus.

In yet another embodiment, provided herein is a method of treating obesity in a subject, comprising administering to the subject in need thereof a therapeutically effective amount of an oral pharmaceutical composition provided herein.

In certain embodiments, the subject is a mammal. In certain embodiments, the subject is a human.

In certain embodiments, the therapeutically effective amount of the therapeutic peptide *(e.g.,* a GLP-1RA) in an oral pharmaceutical composition provided herein is ranging from about 0.1 to about 100 mg per day, from about 0.2 to about 50 mg per day, from about 0.5 to about 25 mg per day, or from about 1 to about 10 mg per day. In certain embodiments, the therapeutically effective amount of the therapeutic peptide *(e.g.,* a GLP-1RA) in an oral pharmaceutical composition provided herein is ranging from about 0.1 to about 100 mg per day. In certain embodiments, the therapeutically effective amount of the therapeutic peptide *(e.g.,* a GLP-1RA) in an oral pharmaceutical composition provided herein is ranging from about 0.2 to about 50 mg per day. In certain embodiments, the therapeutically effective amount of the therapeutic peptide (*e.g.,* a GLP-1RA) in an oral pharmaceutical composition provided herein is ranging from about 0.5 to about 25 mg per day. In certain embodiments, the therapeutically effective amount of the therapeutic peptide (*e.g.,* a GLP-1RA) in an oral pharmaceutical composition provided herein is ranging from about 1 to about 10 mg per day.

In certain embodiments, an oral pharmaceutical composition provided herein is administered once daily (QD), twice daily (BID), three times daily (TID), or four times daily (QID). In certain embodiments, an oral pharmaceutical composition provided herein is administered once daily (QD). In certain embodiments, an oral pharmaceutical composition provided herein is administered twice daily (BID). In certain embodiments, an oral pharmaceutical composition provided herein is administered three times daily (TID). In certain embodiments, an oral pharmaceutical composition provided herein is administered four times daily (QID).

The disclosure will be further understood by the following non-limiting examples.

### EXAMPLES

As used herein, the symbols and conventions used herein, regardless of whether a particular abbreviation is specifically defined, are consistent with those used in the contemporary scientific literature, for example, the Journal of the American Chemical Society, the Journal of Medicinal Chemistry, or the Journal of Biological Chemistry. Specifically, but without limitation, the following abbreviations may be used in the examples and throughout the specification: g (grams); mg (milligrams); mL (milliliters); µL (microliters); mM (millimolar); µM (micromolar); mmol (millimoles); min (minute or minutes); h (hour or hours); DIPEA (*N,N-*diisopropylethylamine); DMF (dimethylformamide); EtOAc (ethyl acetate); HATU (hexafluorophosphate azabenzotriazole tetramethyl uranium); and prep-HPLC (preparative high performance liquid chromatography).

For all of the following examples, standard work-up and purification methods known to those skilled in the art can be utilized. Unless otherwise indicated, all temperatures are expressed in °C (degrees Centigrade). All experiments are conducted at room temperature unless otherwise specified. Synthetic methodologies illustrated herein are intended to exemplify the applicable chemistry through the use of specific examples and are not indicative of the scope of the disclosure.

### Example 1

### Preparation of N⁶-(((R)-4-((3R,5S,7R,8R,9S,10S,12S,13R,14S,17R)-3,7,12-trihydroxy-10,13-dimethylhexadecahydro-1H-cyclopenta[a]phenanthren-17-yl)pentanoyl)glycyl)-L-lysine (KGCA)

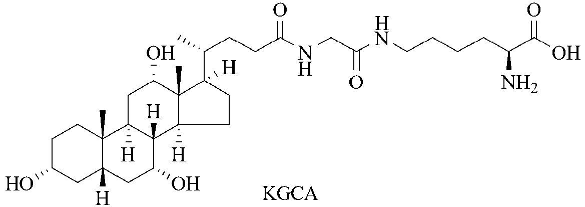

Bile acid compound KGCA was prepared as shown in Scheme 1.

Preparation of protected KGCA (*tert*-butyl *N*²-(*tert*-butoxycarbonyl)-*N*⁶-(((*R*)-4-((3*R*,5*S*,7*R*,8*R*,9*S*,10*S*,12*S*,13*R*,14*S*,17*R*)-3,7,12-trihydroxy-10,13-dimethylhexadecahydro-1*H-*cyclopenta[*a*]phenanthren-17-yl)pentanoyl)glycyl)-L-lysinate). To a solution of glycocholic acid (GCA) (30 g) in DMF (300 mL) at 0 °C were added DIPEA (25 g), HATU (24.5 g), and *tert-*butyl (tert-butoxycarbonyl)-*L*-lysinate (19.5 g). After stirring 25 °C for 2 h, the reaction mixture was added dropwise to a mixed solvent of 0.5M HCl and EtOAc and the organic layer was concentrated to afford protected KGCA (48 g).

Preparation of *N*⁶-(((*R*)-4-((3*R*,5*S*,7*R*,8*R*,9*S*,10*S*,12*S*,13*R*,14*S*,17*R*)-3,7,12-trihydroxy-10,13-dimethylhexadecahydro-1*H*-cyclopenta[*a*]phenanthren-17-yl)pentanoyl)glycyl)-L-lysine (KGCA). To 4M HCl/dioxane (400 mL) at 15 °C was added slowly protected KGCA (48 g). After stirring for 2 h, the reaction mixture was concentrated and purified by reverse-phase prep-HPLC to afford KGCA (20 g).

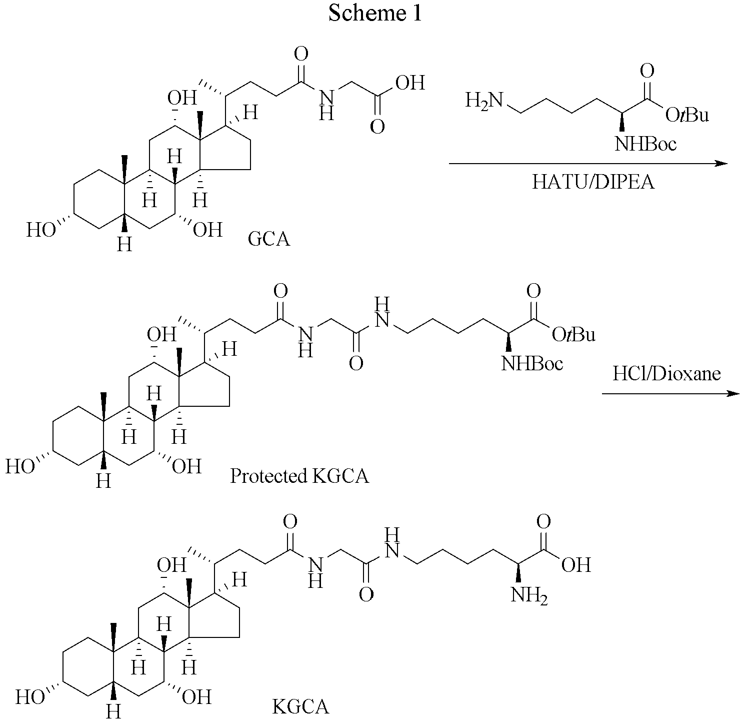

The following bile acid compounds are prepared similarly according to the synthetic procedures or methodologies exemplified herein.

*N*⁶-((*R*)-4-((3*R*,5*S*,7*R*,8*R*,9*S*,10*S*,13*R*,14*S*,17*R*)-3,7-Dihydroxy-10,13-dimethyl-hexadecahydro-1*H*-cyclopenta[a]phenanthren-17-yl)pentanoyl)-L-lysine (KCDCA).

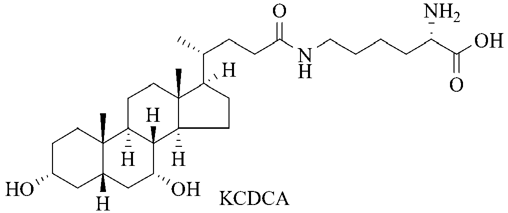

*N*⁶-((*R*)-4-((3*R*,5*S*,7*S*,8*R*,9*S*,10*S*,13*R*,14*S*,17*R*)-3,7-Dihydroxy-10,13-dimethyl-hexadecahydro-1*H*-cyclopenta[a]phenanthren-17-yl)pentanoyl)-L-lysine (KUDCA).

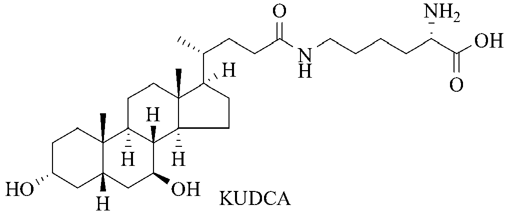

*N*⁶-((*R*)-4-((3*R*,5*R*,8*R*,9*S,*10*S*,12*S*,13*R*,14*S*,17*R*)-3,12-Dihydroxy-10,13-dimethyl-hexadecahydro-1*H*-cyclopenta[a]phenanthren-17-yl)pentanoyl)-L-lysine (KDCA).

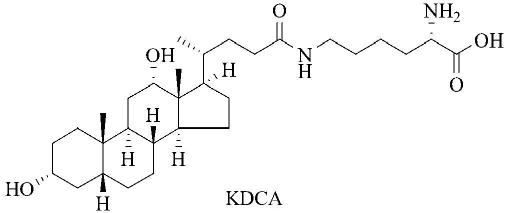

*N*⁶-((*R*)-4-((3*R*,5*S*,7*R*,8*R*,9*S*,10*S*,12*S*,13*R*,14*S*,17*R*)-3,7,12-Trihydroxy-10,13-dimethylhexadecahydro-1*H*-cyclopenta[*a*]phenanthren-17-yl)pentanoyl)-L-lysine (KCA).

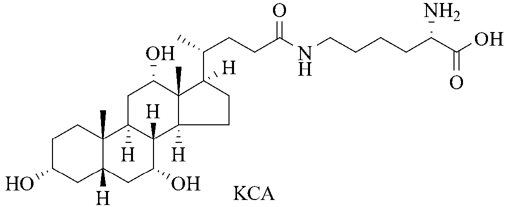

*N*⁶-(((*R*)-4-((3*R*,5*S*,7*R*,8*R*,9*S*,10*S*,12*S*,13*R*,14*S*,17*R*)-3,7,12-Trihydroxy-10,13-dimethylhexadecahydro-1*H*-cyclopenta[*a*]phenanthren-17-yl)pentanoyl)glycyl)-L-arginine (RGCA).

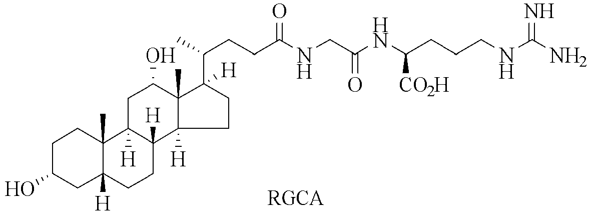

*N*⁶-(((*R*)-4-((3*R*,5*S*,7*R*,8*R*,9*S*,10*S*,12*S*,13*R*,14*S*,17*R*)-3,7,12-Trihydroxy-10,13-dimethylhexadecahydro-1*H*-cyclopenta[*a*]phenanthren-17-yl)pentanoyl)glycyl)-L-histidine (HGCA).

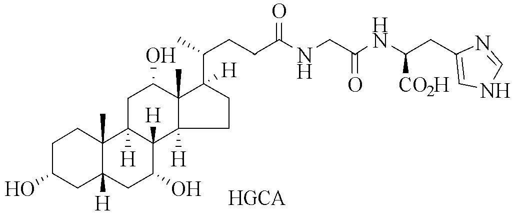

*N*⁶-(((*R*)-4-((3*R*,5*S*,7*R*,8*R*,9*S*,10*S*,12*S*,13*R*,14*S*,17*R*)-3,7,12-Trihydroxy-10,13-dimethylhexadecahydro-1*H*-cyclopenta[*a*]phenanthren-17-yl)pentanoyl)glycyl)-L-aspartic acid (DGCA).

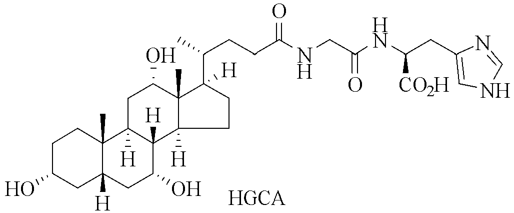

### Example 2

### Preparation of Oral GLP-1RA Formulations

For the preparation of oral semaglutide formulation **F1** in Table 1, a mixture of a semaglutide (a GLP-1RA), GCA, propyl gallate (PG), a soybean trypsin inhibitor (SBTI), and mannitol was grounded in a mortar. The mixture was then dry granulated and sized through a sieve with a pore size of 850 µm to obtain dry granules. After the addition of magnesium stearate and silicon dioxide, the dry granules were tableted using a tablet press to form tablet cores.

The tablet cores were coated with a 4% hydroxypropyl methylcellulose aqueous solution using a coating machine with an outlet air temperature at ≤ 35 °C and an atomization pressure of 0.12 MPa. The liquid spraying was continued until the weight gain rate reached 3%. After drying, the tablets were further coated with a coating solution containing EUDRAGIT S100 (8% by weight), PEG 400 (1.6% by weight), and EtOH (90.4% by weight) using a coating machine with an outlet air temperature at ≤ 30 °C and an atomization pressure of 0.12 MPa. The solution was sprayed continuously until the weight gain rate reached 2.5-3%. The tablets were then dried to obtain dual-layer enteric-coated tablets.

Oral semaglutide formulations **F2** to **F27** in Tables 1 to 6 and oral tirzepatide formulations **F31** to **F37** in Tables 7 and 8 were prepared similarly.

**TABLE 1. Oral Semaglutide Formulations**

| Ingredient | **Formulation (per tablet)** | | | | | | | | | | | |
|---|---|---|---|---|---|---|---|---|---|---|---|---|
| | **F1** (9-001) | | **F2** (9-002) | | **F3** (9-003) | | **F4** (9-004) | | **F5** (9-005) | | **F6** (15-01) | |
| | mg | % | mg | % | mg | % | mg | % | mg | % | mg | % |
| Semaglutide | 7 | 2.4 | 7 | 2.4 | 7 | 2.4 | 7 | 1.6 | 7 | 1.3 | 7 | 1.3 |
| GCA | 50 | 16.9 | 50 | 16.9 | 150 | 50.8 | 150 | 33.3 | 250 | 45.5 | 250 | 45.5 |
| C10 | | | | | | | | | | | 140 | 25.5 |
| PG | 100 | 33.9 | 100 | 33.9 | 100 | 33.9 | 100 | 22.2 | 100 | 18.2 | 100 | 18.2 |
| SBTI | 25 | 8.5 | 40 | 13.6 | 17.5 | 5.9 | 60 | 13.3 | 17.5 | 3.2 | 17.5 | 3.2 |
| Mannitol | 98 | 33.2 | 83 | 28.1 | 5.5 | 1.9 | 109 | 24.2 | 147 | 26.7 | 7 | 1.3 |
| Crospovidone | 10 | 3.4 | 10 | 3.4 | 10 | 3.4 | 15 | 3.3 | 17.5 | 3.2 | 17.5 | 3.2 |
| Mg Stearate | 2.5 | 0.8 | 2.5 | 0.8 | 2.5 | 0.8 | 4.5 | 1 | 5.5 | 1 | 5.5 | 1 |
| Silicon dioxide | 2.5 | 0.8 | 2.5 | 0.8 | 2.5 | 0.8 | 4.5 | 1 | 5.5 | 1 | 5.5 | 1 |

**TABLE 2. Oral Semaglutide Formulations**

| Ingredient | **Formulation (per tablet)** | | | | | | | | | | | |
|---|---|---|---|---|---|---|---|---|---|---|---|---|
| | **F7** (22-001) | | **F8** (22-002) | | **F9** (22-004) | | **F10** (22-006) | | **F11** (22-007) | | **F12** (22-008) | |
| | mg | % | mg | % | mg | % | mg | % | mg | % | mg | % |
| Semaglutide | 7 | 2.4 | 7 | 2.4 | 7 | 2.4 | 7 | 2.4 | 7 | 2.4 | 7 | 2.4 |
| Bile Acid | CDCA | | CDCA | | UDCA | | UDCA | | GCA | | GCA | |
| | 42 | 14.2 | 84 | 28.5 | 40 | 13.6 | 119.5 | 40.5 | 50 | 16.9 | 100 | 33.9 |
| PG | 100 | 33.9 | 100 | 33.9 | 100 | 33.9 | 100 | 33.9 | 100 | 33.9 | 100 | 33.9 |
| SBTI | 5 | 1.7 | 10 | 3.4 | 5 | 1.7 | 15 | 5.1 | 10 | 3.4 | 15 | 5.1 |
| Mannitol | 126 | 42.7 | 79 | 26.8 | 128 | 43.4 | 38.5 | 13.1 | 113 | 38.3 | 58 | 19.7 |
| Crospovidone | 10 | 3.4 | 10 | 3.4 | 10 | 3.4 | 10 | 3.4 | 10 | 3.4 | 10 | 3.4 |
| Mg Stearate | 2.5 | 0.8 | 2.5 | 0.8 | 2.5 | 0.8 | 2.5 | 0.8 | 2.5 | 0.8 | 2.5 | 0.8 |
| Silicon dioxide | 2.5 | 0.8 | 2.5 | 0.8 | 2.5 | 0.8 | 2.5 | 0.8 | 2.5 | 0.8 | 2.5 | 0.8 |

**TABLE 3. Oral Semaglutide Formulations**

| Ingredient | **Formulation (per tablet)** | | | | | | | | | | | |
|---|---|---|---|---|---|---|---|---|---|---|---|---|
| | **F13** (22-011) | | **F14** (22-012) | | **F15** (22-014) | | **F16** (22-015) | | **F17** (22-017) | | **F18** (22-018) | |
| | mg | % | mg | % | mg | % | mg | % | mg | % | mg | % |
| Semaglutide | 7 | 2.4 | 7 | 2.4 | 7 | 2.4 | 7 | 2.4 | 7 | 2.4 | 7 | 2 |
| Bile Acid | DCA | | DCA | | CA | | CA | | KGCA | | KGCA | |
| | 84 | 28.5 | 126 | 42.7 | 83 | 28.1 | 124.5 | 42.2 | 120.5 | 40.8 | 180.5 | 51.6 |
| PG | 100 | 33.9 | 100 | 33.9 | 100 | 33.9 | 100 | 33.9 | 100 | 33.9 | 100 | 28.6 |
| SBTI | 5 | 1.7 | 10 | 3.4 | 15 | 5.1 | 5 | 1.7 | 15 | 5.1 | 5 | 1.4 |
| Mannitol | 84 | 28.5 | 37 | 12.5 | 75 | 25.4 | 43.5 | 14.7 | 37.5 | 12.7 | 42.5 | 12.1 |
| Crospovidone | 10 | 3.4 | 10 | 3.4 | 10 | 3.4 | 10 | 3.4 | 10 | 3.4 | 10 | 2.9 |
| Mg Stearate | 2.5 | 0.8 | 2.5 | 0.8 | 2.5 | 0.8 | 2.5 | 0.8 | 2.5 | 0.8 | 2.5 | 0.7 |
| Silicon dioxide | 2.5 | 0.8 | 2.5 | 0.8 | 2.5 | 0.8 | 2.5 | 0.8 | 2.5 | 0.8 | 2.5 | 0.7 |

**TABLE 4. Oral Semaglutide Formulations**

| Ingredient | **Formulation (per tablet)** | | | | | | | |
|---|---|---|---|---|---|---|---|---|
| | **F19** (24-001) | | **F20** (24-002) | | **F21** (24-003) | | **F22** (24-004) | |
| | mg | % | mg | % | mg | % | mg | % |
| Semaglutide | 7 | 2.4 | 7 | 2.4 | 7 | 2.4 | 7 | 2.0 |
| KGCA | 90.5 | 30.7 | 90.5 | 30.7 | 90.5 | 30.7 | 180.5 | 51.6 |
| PG | 100 | 33.9 | 100 | 33.9 | 100 | 33.9 | 100 | 28.6 |
| SBTI | 2.5 | 0.8 | 5 | 1.7 | 15 | 5.1 | 2.5 | 0.7 |
| Mannitol | 80 | 27.1 | 77.5 | 26.3 | 67.5 | 22.9 | 45 | 12.9 |
| Crospovidone | 10 | 3.4 | 10 | 3.4 | 10 | 3.4 | 10 | 2.9 |
| Mg Stearate | 2.5 | 0.8 | 2.5 | 0.8 | 2.5 | 0.8 | 2.5 | 0.7 |
| Silicon dioxide | 2.5 | 0.8 | 2.5 | 0.8 | 2.5 | 0.8 | 2.5 | 0.7 |

**TABLE 5. Oral Semaglutide Formulations**

| Ingredient | **Formulation (per tablet)** | | | | | |
|---|---|---|---|---|---|---|
| | **F23** (24-007) | | **F24** (24-008) | | **F25** (24-009) | |
| | mg | % | mg | % | mg | % |
| Semaglutide | 4.7 | 1.6 | 4.7 | 1.6 | 4.7 | 1.6 |
| KGCA | 180.7 | 61.2 | 180.7 | 61.2 | 180.7 | 61.2 |
| PG | 66.7 | 22.6 | 66.7 | 22.6 | 66.7 | 22.6 |
| SBTI | 1.7 | 0.6 | 3.3 | 1.1 | 10 | 3.4 |
| Mannitol | 26.3 | 8.9 | 24.7 | 8.4 | 18 | 6.1 |
| Crospovidone | 10 | 3.4 | 10 | 3.4 | 10 | 3.4 |
| Mg Stearate | 2.5 | 0.8 | 2.5 | 0.8 | 2.5 | 0.8 |
| Silicon dioxide | 2.5 | 0.8 | 2.5 | 0.8 | 2.5 | 0.8 |

**TABLE 6. Oral Semaglutide Formulations**

| Ingredient | **Formulation (per tablet)** | | | |
|---|---|---|---|---|
| | **F26** (24-010) | | **F27** (24-011) | |
| | mg | % | mg | % |
| Semaglutide | 7 | 2.4 | 4.67 | 1.3 |
| CDCA | 50 | 16.9 | 100 | 28.6 |
| UDCA | | | 100 | 28.6 |
| PG | 100 | 33.9 | 100 | 28.6 |
| SBTI | 0 | 0 | 20 | 5.7 |
| Mannitol | 123 | 41.7 | 10.3 | 3.0 |
| Crospovidone | 10 | 3.4 | 10 | 2.9 |
| Mg Stearate | 2.5 | 0.8 | 2.5 | 0.7 |
| Silicon dioxide | 2.5 | 0.8 | 2.5 | 0.7 |

**TABLE 7. Oral Tirzepatide Formulations**

| Ingredient | **Formulation (per tablet)** | | | | | | | |
|---|---|---|---|---|---|---|---|---|
| | **F31** (14-001) | | **F32** (22-003) | | **F33** (22-005) | | **F34** (22-009) | |
| | mg | % | mg | % | mg | % | mg | % |
| Tirzepatide | 7 | 2.4 | 7 | 2.4 | 7 | 2.4 | 7 | 2.4 |
| Bile Acid | GCA | | CDCA | | UDCA | | GCA | |
| | 50 | 16.9 | 126 | 42.7 | 79.5 | 26.9 | 150 | 50.8 |
| PG | 100 | 33.9 | 100 | 33.9 | 100 | 33.9 | 100 | 33.9 |
| SBTI | 25 | 8.5 | 15 | 5.1 | 10 | 3.4 | 5 | 1.7 |
| Mannitol | 98 | 33.2 | 32 | 10.8 | 83.5 | 28.3 | 18 | 6.1 |
| Crospovidone | 10 | 3.4 | 10 | 3.4 | 10 | 3.4 | 10 | 3.4 |
| Mg Stearate | 2.5 | 0.8 | 2.5 | 0.8 | 2.5 | 0.8 | 2.5 | 0.8 |
| Silicon dioxide | 2.5 | 0.8 | 2.5 | 0.8 | 2.5 | 0.8 | 2.5 | 0.8 |

**TABLE 8. Oral Tirzepatide Formulations**

| Ingredient | **Formulation (per tablet)** | | | | | |
|---|---|---|---|---|---|---|
| | **F35** (22-010) | | **F36** (22-013) | | **F37** (22-016) | |
| | mg | % | mg | % | mg | % |
| Tirzepatide | 7 | 2.4 | 7 | 2.4 | 7 | 2.4 |
| Bile Acid | DCA | | CA | | KGCA | |
| | 42 | 14.2 | 41.5 | 14.1 | 60 | 20.3 |
| PG | 100 | 33.9 | 100 | 33.9 | 100 | 33.9 |
| SBTI | 15 | 5.1 | 10 | 3.4 | 10 | 3.4 |
| Mannitol | 116 | 39.3 | 121.5 | 41.2 | 103 | 34.9 |
| Crospovidone | 10 | 3.4 | 10 | 3.4 | 10 | 3.4 |
| Mg Stearate | 2.5 | 0.8 | 2.5 | 0.8 | 2.5 | 0.8 |
| Silicon dioxide | 2.5 | 0.8 | 2.5 | 0.8 | 2.5 | 0.8 |

### Example 3

### Bioavailability Determination of Oral GLP-1RA Formulations

The pharmaceutical properties of oral semaglutide formulation **F1** and RYBELSUS were evaluated under both fasting and non-fasting conditions. After adaptation period, 20 male washed-out beagle dogs were randomized into 4 groups. The animals of Groups 1 and 2 were fasted for 16 h and deprived of water for 12 h before administration; and the animals of Groups 3 and 4 were free to eat and drink water before administration. After administration, all the animals were free to move, but fasted and deprived of water for 0 to 4 h. After 4 h, they resumed feeding and were free to drink water (30 mL). Each animal of Groups 1 and 3 was given once a RYBELSUS tablet containing 14 mg semaglutide, whereas each animal of Groups 2 and 4 was given once two enteric-coated tablets of oral semaglutide formulation **F1**, each tablet containing 7 mg semaglutide. Blood samples were collected from the animals before administration and 0.25, 0.5, 1, 2, 4, 6, 8, 10, 24, 48, 72, 96, and 120 h after administration. The blood samples were centrifuged within 1.5 h after collection at 4 °C and 3,000 g for 10 min to obtain plasma samples, which were stored at -80 °C until analysis. The plasma samples were analyzed by LC-MS/MS for semaglutide. Pharmacokinetic parameters were calculated using WINNONLIN.

Similarly, oral semaglutide formulations **F2** to **F27** were evaluated under fasting conditions. The results are summarized in Tables 9 and 10, where the CV of each group was calculated based on their Cₘₐₓ values. The bioavailability (BA) of each oral semaglutide formulation is calculated as follows: BA (%) = AUC _{0-inf} of oral semaglutide formulation/AUC_{0-inf} of RYBELSUS x BA of RYBELSUS, where BA of RYBELSUS is 1%.

**TABLE 9. Pharmacokinetics of Oral Semaglutide Formulations**

| **Group** | **Number of Animals** | **Drug** | **Cₘₐₓ (ng/mL)** | **AUC₀₋ₕ (h·ng/mL)** | **AUC _{0-inf} (h·ng/mL)** | **BA (%)** | **CV (%)** |
|---|---|---|---|---|---|---|---|
| 1 | 4 | RYBELSUS | 105 | 4,740 | 6,370 | 1 | 66 |
| 2 | 4 | **F1** | 1,110 | 43,700 | 53,200 | 8.4 | 141 |
| 3 | 6 | RYBELSUS | 37 | 1,660 | 3350 | 0.5 | 147 |
| 4 | 6 | **F1** | 176 | 9,880 | 19,600 | 3.1 | 118 |

**TABLE 10. Pharmacokinetics of Oral Semaglutide Formulations**

| **Formulation** | **Number of Animals** | **Cₘₐₓ (ng/mL)** | **AUC₀₋ₕ (h·ng/mL)** | **AUC _{0-inf} (h·ng/mL)** | **BA (%)** | **CV (%)** |
|---|---|---|---|---|---|---|
| **F2** | 4 | 730 | 32,000 | 41,600 | 6.5 | 84 |
| **F3** | 4 | 1,070 | 54,600 | 74,200 | 12 | 88 |
| **F4** | 4 | 655 | 27,300 | 34,500 | 5.4 | 57 |
| **F5** | 4 | 1,240 | 56,800 | 74,400 | 12 | 38 |
| **F6** | 4 | 1,340 | 60,100 | 78,800 | 12 | 46 |
| **F7** | 4 | 19 | 620 | 2,480 | 0.39 | 78 |
| **F8** | 4 | 208 | 7,763 | 12,819 | 2 | 147 |
| **F9** | 4 | 49 | 1,468 | 1,955 | 0.3 | 63 |
| **F10** | 4 | 453 | 15,049 | 16,005 | 2.5 | 142 |
| **F11** | 4 | 94 | 2,687 | 3,119 | 0.49 | 140 |
| **F12** | 4 | 165 | 5,291 | 6,070 | 0.95 | 64 |
| **F13** | 4 | 1,038 | 31,602 | 34,766 | 5.5 | 80 |
| **F14** | 4 | 1,007 | 32,487 | 36,661 | 5.8 | 16 |
| **F15** | 4 | 1,035 | 46,736 | 58,629 | 9.2 | 96 |
| **F16** | 4 | 102 | 5,434 | 7,397 | 1.2 | 50 |
| **F17** | 4 | 1,000 | 46,341 | 58,659 | 9.2 | 116 |
| **F18** | 4 | 1,950 | 84,379 | 105,086 | 17 | 45 |
| **F19** | 4 | 350 | 16,073 | 20,233 | 3.2 | 64 |
| **F20** | 4 | 553 | 26,834 | 33,533 | 5.3 | 178 |
| **F21** | 4 | 748 | 36,531 | 50,138 | 7.9 | 82 |
| **F22** | 4 | 1,391 | 64,149 | 81,924 | 13 | 30 |
| **F23** | 4 | 923 | 43,053 | 51,958 | 8.2 | 104 |
| **F24** | 4 | 1,076 | 55,766 | 70,070 | 11 | 73 |
| **F25** | 4 | 1,179 | 54,558 | 71,121 | 11 | 57 |
| **F26** | 4 | 239 | 10,299 | 12,804 | 2 | 102 |
| **F27** | 4 | 331 | 14,674 | 19,707 | 3.1 | 79 |

Oral tirzepatide formulations **F31** to **F27** were each evaluated similarly under fasting conditions. A tirzepatide solution for subcutaneous injection was also employed. The results are summarized in Table 11, where the bioavailability of each oral tirzepatide formulation was calculated as follows: BA (%) = AUC _{0-inf} of oral tirzepatide formulation/AUC _{0-inf} of injected tirzepatide x 100%.

**TABLE 11. Pharmacokinetics of Oral Tirzepatide Formulations**

| **Formulation** | **Number of Animals** | **Cₘₐₓ (ng/mL)** | **AUC₀₋ₕ (h·ng/mL)** | **AUC _{0-inf} (h·ng/mL)** | **BA (%)** | **CV (%)** |
|---|---|---|---|---|---|---|
| **Injected Tirzepatide** | 4 | 486 | 66,375 | 70,913 | / | 4.6 |
| **F31** | 4 | 408 | 17,987 | 30,259 | 3.1 | 105 |
| **F32** | 4 | 288 | 11,228 | 14,070 | 1.4 | 149 |
| **F33** | 4 | 19 | 605 | 781 | 0.1 | 91 |
| **F34** | 4 | 461 | 17,866 | 21,618 | 2.2 | 82 |
| **F35** | 4 | 135 | 4,752 | 5,851 | 0.6 | 70 |
| **F36** | 4 | 234 | 11,399 | 17,068 | 1.7 | 99 |
| **F37** | 4 | 228 | 11,120 | 16,960 | 1.7 | 53 |

### Example 4

### Effect of Oral GLP-1RA Formulations on Weight Loss in Duodenal Intubation Model

After duodenal intubation model operation, 6 C57BL/6J mice assigned to Group 1 (G1) as a control; and 36 diet-induced obese (DIO) mice were randomized into 6 groups according to body weight with 6 mice per group: model group (G2), positive control group (G3), GCA formulation group (G4 ), KGCA formulation group (G5), and KGCA compound group (G6).

Each mouse in G2 (model group) was given pure water (10 mL/kg) once a day for 4 weeks. Each mouse in G3 (positive control group) was given semaglutide (10 µg/kg) as a solution by subcutaneous injection at a volume of 10 mL/kg once a day for 4 weeks. Each mouse in G4 (semaglutide/GCA treatment group) was given formulation **F3** (10 mg/L) as a solution at a volume of 10 mL/kg once a day for 4 weeks. Each mouse in G5 (semaglutide/KGCA treatment group) was given formulation **F22** (10 mg/L) as a solution at a volume of 10 mL/kg once a day for 4 weeks.

The mice were monitored for weight once a day, food intake twice a week, fasting blood sugar level once a week before and after administration, four blood lipids and liver blood biochemistry before administration and at the end of the administration period, fat and liver weight and organ coefficient at the end of the administration period, and liver pathology. Formulations **F3** and **F22** were found to be comparable to subcutaneous semaglutide in terms of body weight, food intake, weekly fasting blood glucose, four blood lipids after administration, liver blood biochemistry, fat and liver weight and its organ coefficient, and liver pathology.

The examples set forth above are provided to give those of ordinary skill in the art with a complete disclosure and description of how to make and use the claimed embodiments and are not intended to limit the scope of what is disclosed herein. Modifications that are obvious to persons of skill in the art are intended to be within the scope of the following claims. All publications, patents, and patent applications cited in this specification are incorporated herein by reference as if each such publication, patent or patent application were specifically and individually indicated to be incorporated herein by reference.

### Further embodiments

1. An oral pharmaceutical composition comprising a therapeutic polypeptide, a bile acid compound, a CYP450 inhibitor, and one or more pharmaceutically acceptable excipients; wherein the bile acid compound is a compound having the structure of Formula (I): 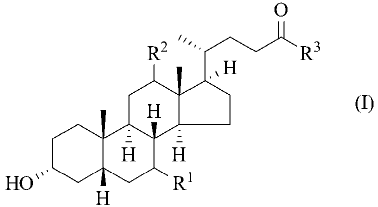 or an enantiomer, a mixture of enantiomers, a diastereomer, a mixture of two or more diastereomers, a tautomer, a mixture of two or more tautomers thereof; or a pharmaceutically acceptable salt, solvate, or hydrate thereof; wherein:
   R¹ and R² are each independently hydrogen or -OH;
   R³ is -N(H)-X-Z-R⁴, heterocyclyl, or -OH;
   R⁴ is C₁₋₆ alkyl, C₁₋₆ heteroalkyl, C₇₋₁₅ aralkyl, heterocyclyl, -OH, or -N(H)R^{4a};
   R^{4a} is C₁₋₆ alkyl, C₁₋₆ heteroalkyl, or C₇₋₁₅ aralkyl;
   X is a bond, C₁₋₆ alkylene, or C₁₋₆ heteroalkylene; and
   Z is a bond, -C(O)-, or -S(O₂)-;
   wherein each alkyl, alkylene, heteroalkyl, heteroalkylene, aralkyl, and heterocyclyl is optionally substituted with one or more, in one embodiment, one, two, three, or four, substituents Q, wherein each Q is independently selected from: (a) deuterium, cyano, halo, imino, nitro, and oxo; (b) C₁₋₆ alkyl, C₁₋₆ heteroalkyl, C₂₋₆ alkenyl, C₂₋₆ alkynyl, C₃₋₁₀ cycloalkyl, C₆₋₁₄ aryl, C₇₋₁₅ aralkyl, heteroaryl, and heterocyclyl, each of which is further optionally substituted with one or more, in one embodiment, one, two, three, or four, substituents Q^{a}; and (c) -C(O)R^{a}, -C(O)OR^{a}, -C(O)NR^{b}R^{c}, -C(O)SR^{a}, -C(NR^{a})NR^{b}R^{c}, -C(S)R^{a}, -C(S)OR^{a}, -C(S)NR^{b}R^{c}, -OR^{a}, -OC(O)R^{a}, -OC(O)OR^{a}, -OC(O)NR^{b}R^{c}, -OC(O)SR^{a}, -OC(NR^{a})NR^{b}R^{c}, -OC(S)R^{a}, -OC(S)OR^{a}, -OC(S)NR^{b}R^{c}, -OS(O)R^{a}, -OS(O)₂R^{a}, -OS(O)NR^{b}R^{c}, -OS(O)₂NR^{b}R^{c}, -NR^{b}R^{c}, -NR^{a}C(O)R^{d}, -NR^{a}C(O)OR^{d}, -NR^{a}C(O)NR^{b}R^{c}, -NR^{a}C(O)SR^{d}, -NR^{a}C(NR^{d})NR^{b}R^{c}, -NR^{a}C(S)R^{d}, -NR^{a}C(S)OR^{d}, -NR^{a}C(S)NR^{b}R^{c}, -NR^{a}S(O)R^{d}, -NR^{a}S(O)₂R^{d}, -NR^{a}S(O)NR^{b}R^{c}, -NR^{a}S(O)₂NR^{b}R^{c}, -SR^{a}, -S(O)R^{a}, -S(O)₂R^{a}, -S(O)NR^{b}R^{c}, and -S(O)₂NR^{b}R^{c}, wherein each R^{a}, R^{b}, R^{c}, and R^{d} is independently (i) hydrogen or deuterium; (ii) C₁₋₆ alkyl, C₁₋₆ heteroalkyl, C₂₋₆ alkenyl, C₂₋₆ alkynyl, C₃₋₁₀ cycloalkyl, C₆₋₁₄ aryl, C₇₋₁₅ aralkyl, heteroaryl, or heterocyclyl, each of which is optionally substituted with one or more, in one embodiment, one, two, three, or four, substituents Q^{a}; or (iii) R^{b} and R^{c} together with the N atom to which they are attached form heterocyclyl, optionally substituted with one or more, in one embodiment, one, two, three, or four, substituents Q^{a};
   wherein each Q^{a} is independently selected from: (a) deuterium, cyano, halo, nitro, imino, and oxo; (b) C₁₋₆ alkyl, C₂₋₆ alkenyl, C₂₋₆ alkynyl, C₃₋₁₀ cycloalkyl, C₆₋₁₄ aryl, C₇₋₁₅ aralkyl, heteroaryl, and heterocyclyl; and (c) -C(O)R^{e}, -C(O)OR^{e}, -C(O)NR^{f}R^{g}, -C(O)SR^{e}, -C(NR^{e})NR^{f}R^{g}, -C(S)R^{e}, -C(S)OR^{e}, -C(S)NR^{f}R^{g}, -OR^{e}, -OC(O)R^{e}, -OC(O)OR^{e}, -OC(O)NR^{f}R^{g}, -OC(O)SR^{e}, -OC(NR^{e})NR^{f}R^{g}, -OC(S)R^{e}, -OC(S)OR^{e}, -OC(S)NR^{f}R^{g}, -OS(O)R^{e}, -OS(O)₂R^{e}, -OS(O)NR^{f}R^{g}, -OS(O)₂NR^{f}R^{g}, -NR^{f}R^{g}, -NR^{e}C(O)R^{h}, -NR^{e}C(O)OR^{f}, -NR^{e}C(O)NR^{f}R^{g}, -NR^{e}C(O)SR^{f}, -NR^{e}C(NR^{h})NR^{f}R^{g}, -NR^{e}C(S)R^{h}, -NR^{e}C(S)OR^{f}, -NR^{e}C(S)NR^{f}R^{g}, -NR^{e}S(O)R^{h}, -NR^{e}S(O)₂R^{h}, -NR^{e}S(O)NR^{f}R^{g}, -NR^{e}S(O)₂NR^{f}R^{g}, -SR^{e}, -S(O)R^{e}, -S(O)₂R^{e}, -S(O)NR^{f}R^{g}, and -S(O)₂NR^{f}R^{g}; wherein each R^{e}, R^{f}, R^{g}, and R^{h} is independently (i) hydrogen or deuterium; (ii) C₁₋₆ alkyl, C₂₋₆ alkenyl, C₂₋₆ alkynyl, C₃₋₁₀ cycloalkyl, C₆₋₁₄ aryl, C₇₋₁₅ aralkyl, heteroaryl, or heterocyclyl; or (iii) R^{f} and R^{g} together with the N atom to which they are attached form heterocyclyl; or
   wherein the bile acid compound is chenodeoxycholic acid, cholic acid, dehydrocholic acid, deoxycholic acid, glycochenodeoxycholic acid, glycocholic acid, lithocholic acid, taurocholic acid, taurodeoxycholic acid, or ursodeoxycholic acid.
2. The oral pharmaceutical composition of embodiment 1, comprising from about 5 to about 65% by weight of the bile acid compound.
3. The oral pharmaceutical composition of embodiment 1 or 2, comprising from about 15 to about 50% or from about 40 to about 65% by weight of the bile acid compound.
4. The oral pharmaceutical composition of any one of embodiments 1 to 3, wherein R³ is -N(H)-X-Z-R⁴.
5. The oral pharmaceutical composition of any one of embodiments 1 to 4, wherein X is a bond.
6. The oral pharmaceutical composition of any one of embodiments 1 to 4, wherein X is C₁₋₆ alkylene, optionally substituted with one or more substituents Q.
7. The oral pharmaceutical composition of any one of embodiments 1 to 4 and 6, wherein X is methanediyl, ethane-1,1-diyl, propane-1,1-diyl, butane-1,1-diyl, or pentane-1,1-diyl, each optionally substituted with methyl, phenyl, 4-hydroxyphenyl, imidazol-4-yl, indol-3-yl, carboxy, aminocarbonyl, hydroxyl, amino, guanidino, mercapto, or methylthio.
8. The oral pharmaceutical composition of any one of embodiments 1 to 4, 6, and 7, wherein X is methanediyl, ethane-1,1-diyl, 2-phenylethane-1,1-diyl, 2-(4-hydroxyphenyl)ethane-1,1-diyl, 2-imidazol-4-ylethane-1,1-diyl, 2-(indol-3-yl)ethane-1,1-diyl, 2-carboxyethane-1,1-diyl, 2-hydroxyethane-1,1-diyl, 2-aminocarbonylethane-1,1-diyl, 2-mercaptoethane-1,1-diyl, 2-methylpropane-1,1-diyl, 2-carboxypropane-1,1-diyl, 2-hydroxypropane-1,1-diyl, 3-amino-carbonylpropane-1,1-diyl, 3-methylthiopropane-1,1-diyl, 2-methylbutane-1,1-diyl, 3-methylbutane-1,1-diyl, 4-aminobutane-1,1-diyl, 4-guanidinobutane-1,1-diyl, or 5-aminopentyane-1,1-diyl.
9. The pharmaceutical composition of any one of embodiments 1 to 8, wherein Z is a bond.
10. The pharmaceutical composition of any one of embodiments 1 to 8, wherein Z is -C(O)-.
11. The pharmaceutical composition of any one of embodiments 1 to 8, wherein Z is -S(O₂)-.
12. The pharmaceutical composition of any one of embodiments 1 to 11, wherein R⁴ is C₁₋₆ alkyl or C₇₋₁₅ aralkyl, each optionally substituted with one or more substituents Q.
13. The pharmaceutical composition of any one of embodiments 1 to 5, 9, and 12, wherein the bile acid compound is a compound having the structure of Formula (VI): 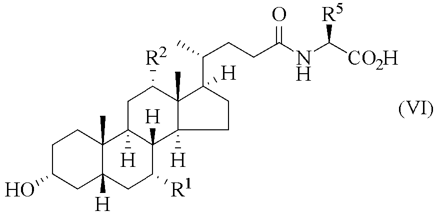 or a diastereomer, a mixture of two or more diastereomers, a tautomer, a mixture of two or more tautomers thereof; or a pharmaceutically acceptable salt, solvate, or hydrate thereof; wherein R⁵ is (i) hydrogen; or (ii) C₁₋₆ alkyl, C₁₋₆ heteroalkyl, or C₇₋₁₅ aralkyl, each optionally substituted with one or more substituents Q.
14. The pharmaceutical composition of any one of embodiments 1 to 5, 9, and 12, wherein the bile acid compound is a compound having the structure of Formula (VIII): 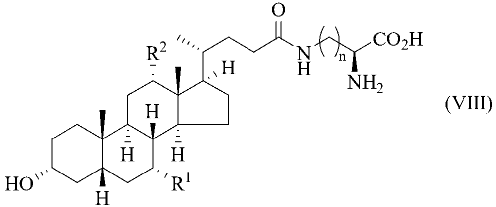 or a diastereomer, a mixture of two or more diastereomers, a tautomer, a mixture of two or more tautomers thereof; or a pharmaceutically acceptable salt, solvate, or hydrate thereof; wherein n is an integer of 1, 2, 3, 4, or 5.
15. The pharmaceutical composition of any one of embodiments 1 to 11, wherein R⁴ is

   -N(H)R^{4a}.
16. The pharmaceutical composition of embodiment 15, wherein R^{4a} is C₁₋₆ alkyl or C₇₋₁₅ aralkyl, each optionally substituted with one or more substituents Q.
17. The pharmaceutical composition of any one of embodiments 1 to 4, 6, 10, 15, and 16, wherein the bile acid compound is a compound having the structure of Formula (X): 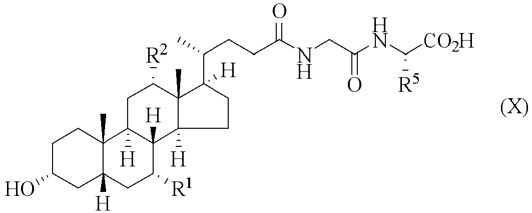 or a diastereomer, a mixture of two or more diastereomers, a tautomer, a mixture of two or more tautomers thereof; or a pharmaceutically acceptable salt, solvate, or hydrate thereof; wherein R⁵ is (i) hydrogen; or (ii) C₁₋₆ alkyl, C₁₋₆ heteroalkyl, C₁₋₆ heteroalkyl, or C₇₋₁₅ aralkyl, each optionally substituted with one or more substituents Q.
18. The pharmaceutical composition of any one of embodiments 1 to 4, 6, 10, 15, and 16, wherein the bile acid compound is a compound having the structure of Formula (XII): 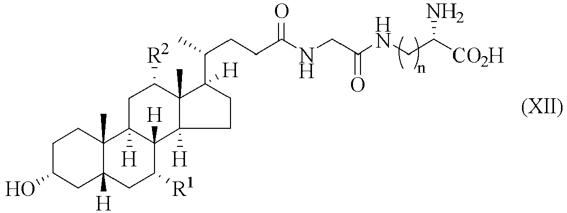 or a diastereomer, a mixture of two or more diastereomers, a tautomer, a mixture of two or more tautomers thereof; or a pharmaceutically acceptable salt, solvate, or hydrate thereof; wherein n is an integer of 1, 2, 3, 4, or 5.
19. The pharmaceutical composition of any one of embodiments 1 to 18, wherein R¹ is hydrogen.
20. The pharmaceutical composition of any one of embodiments 1 to 18, wherein R¹ is -OH.
21. The pharmaceutical composition of any one of embodiments 1 to 20, wherein R² is hydrogen.
22. The pharmaceutical composition of any one of embodiments 1 to 20, wherein R² is -OH.
23. The pharmaceutical composition of any one of embodiments 13, 17, and 19 to 22, wherein R⁵ is (i) hydrogen; or (ii) C₁₋₆ alkyl or C₇₋₁₅ aralkyl, each optionally substituted with heteroaryl, -C(O)OR^{a}, -C(O)NR^{b}R^{c}, -OR^{a}, -NR^{b}R^{c}, -NR^{a}C(NR^{d})NR^{b}R^{c}, or -SR^{a}.
24. The pharmaceutical composition of any one of embodiments 13, 17, and 19 to 23, wherein R⁵ is (i) hydrogen; or (ii) methyl, ethyl, propyl, butyl, or benzyl, each optionally substituted with imidazolyl, indolyl, carboxy, aminocarbonyl, hydroxyl, amino, guanidino, mercapto, or methylthio.
25. The pharmaceutical composition of any one of embodiments 13, 17, and 19 to 24, wherein R⁵ is hydrogen, methyl, isopropyl, isobutyl, but-2-yl, benzyl, 4-hydroxybenzyl, imidazol-4-ylmethyl, indol-3-ylmethyl, carboxymethyl, 2-carboxyethyl, aminocarbonylmethyl, 2-aminocarbonylethyl, hydroxymethyl, 1-hydroxyethyl, 3-aminopropyl, 4-aminobutyl, 3-guanidinopropyl, mercaptomethyl, or 2-methylthioethyl.
26. The pharmaceutical composition of any one of embodiments 14 and 18 to 22, wherein n is an integer of 3, 4, or 5.
27. The pharmaceutical composition of any one of embodiments 14, 18 to 22, and 26, wherein n is an integer of 4.
28. The pharmaceutical composition of any one of embodiments 1 to 3, wherein the bile acid compound is:
   N⁶-(((*R*)-4-((3*R*,5*S*,7*R*,8*R*,9*S*,10*S*,12*S*,13*R*,14*S*,17*R*)-3,7,12-trihydroxy-10,13-dimethyl-hexadecahydro-1*H*-cyclopenta[a]phenanthren-17-yl)pentanoyl)glycyl)-L-lysine;
   *N*⁶-((*R*)-4-((3R*,*5*S*,7*R*,8*R*,9*S*,10*S*,13*R*,14*S*,17*R*)-3,7-dihydroxy-10,13-dimethylhexadecahydro-1*H*-cyclopenta[*a*]phenanthren-17-yl)pentanoyl)-L-lysine;
   *N*⁶-((*R*)-4-((3*R*,5*S*,7*S*,8*R*,9*S*,10*S*,13*R*,14*S*,17*R*)-3,7-dihydroxy-10,13-dimethylhexadecahydro-1*H*-cyclopenta[a]phenanthren-17-yl)pentanoyl)-L-lysine;
   *N*⁶-((*R*)-4-((3*R*,5*R*,8*R*,9*S*,10*S*,12*S*,13*R*,14*S*,17*R*)-3,12-dihydroxy-10,13-dimethylhexadecahydro-1*H*-cyclopenta[a]phenanthren-17-yl)pentanoyl)-L-lysine;
   *N*⁶-((*R*)-4-((3*R*,5*S*,7*R*,8*R*,9*S*,10*S*,12*S*,13*R*,14*S*,17*R*)-3,7,12-trihydroxy-10,13-dimethylhexadecahydro-1*H*-cyclopenta[a]phenanthren-17-yl)pentanoyl)-L-lysine;
   *N*⁶-(((*R*)-4-((3*R*,5*S*,7*R*,8*R*,9*S*,10*S*,12*S*,13*R*,14*S*,17*R*)-3,7,12-trihydroxy-10,13-dimethylhexadecahydro-1*H*-cyclopenta[*a*]phenanthren-17-yl)pentanoyl)glycyl)-L-arginine;
   *N*⁶-(((*R*)-4-((3*R*,5*S*,7*R*,8*R*,9*S*,10*S*,12*S*,13*R*,14*S*,17*R*)-3,7,12-trihydroxy-10,13-dimethylhexadecahydro-1*H*-cyclopenta[*a*]phenanthren-17-yl)pentanoyl)glycyl)-L-histidine; or
   *N*⁶-(((*R*)-4-((3*R*,5*S*,7*R*,8*R*,9*S*,10*S*,12*S*,13*R*,14*S*,17*R*)-3,7,12-trihydroxy-10,13-dimethylhexadecahydro-1*H*-cyclopenta[a]phenanthren-17-yl)pentanoyl)glycyl)-L-aspartic acid;
   or a diastereomer, a mixture of two or more diastereomers, a tautomer, a mixture of two or more tautomers thereof; or a pharmaceutically acceptable salt, solvate, or hydrate thereof.
29. The pharmaceutical composition of any one of embodiments 1 to 4, 6, 10, 15, 16, 18, 20, 22, and 26 to 28, wherein the bile acid compound is:
   *N*⁶-(((*R*)-4-((3*R*,5*S*,7*R*,8*R*,9*S*,10*S*,12*S*,13*R*,14*S*,17*R*)-3,7,12-trihydroxy-10,13-dimethylhexadecahydro-1*H*-cyclopenta[*a*]phenanthren-17-yl)pentanoyl)glycyl)-*L*-lysine;
   or a diastereomer, a mixture of two or more diastereomers, a tautomer, a mixture of two or more tautomers thereof; or a pharmaceutically acceptable salt, solvate, or hydrate thereof.
30. The pharmaceutical composition of any one of embodiments 1 to 3, wherein the bile acid compound is chenodeoxycholic acid, cholic acid, dehydrocholic acid, deoxycholic acid, glycol-chenodeoxycholic acid, glycocholic acid, lithocholic acid, taurocholic acid, taurodeoxycholic acid, or ursodeoxycholic acid.
31. The pharmaceutical composition of any one of embodiments 1 to 3 and 30,
   wherein the bile acid compound is deoxycholic acid.
32. The pharmaceutical composition of any one of embodiments 1 to 3 and 30,
   wherein the bile acid compound is glycocholic acid.
33. The pharmaceutical composition of any one of embodiments 1 to 32, comprising from about 0.1 to about 10% by weight of the therapeutic polypeptide.
34. The pharmaceutical composition of any one of embodiments 1 to 32, comprising from about 1 to about 2.5% by weight of the therapeutic polypeptide.
35. The pharmaceutical composition of any one of embodiments 1 to 34, wherein the therapeutic peptide is a glucagon-like peptide-1 receptor agonist, a κ-opioid receptor agonist, or an insulin receptor agonist.
36. The pharmaceutical composition of any one of embodiments 1 to 35, wherein the therapeutic peptide is a glucagon-like peptide-1 receptor agonist.
37. The pharmaceutical composition of any one of embodiments 1 to 36, wherein the therapeutic peptide is beinaglutide, exenatide, liraglutide, lixisenatide, mazdutide, PEG-loxenatide, PEGylated exenatide, retatrutide, semaglutide, or tirzepatide.
38. The pharmaceutical composition of any one of embodiments 1 to 37, wherein the therapeutic peptide is a lipidated peptide.
39. The pharmaceutical composition of any one of embodiments 1 to 38, wherein the therapeutic peptide is semaglutide.
40. The pharmaceutical composition of any one of embodiments 1 to 38, wherein the therapeutic peptide is tirzepatide.
41. The pharmaceutical composition of any one of embodiments 1 to 40, comprising from about 10 to about 50% by weight of the CYP450 inhibitor.
42. The pharmaceutical composition of any one of embodiments 1 to 41, comprising from about 15 to about 35% or from about 20 to about 35% by weight of the CYP450 inhibitor.
43. The pharmaceutical composition of any one of embodiments 1 to 42, wherein the CYP450 inhibitor is a gallate ester.
44. The pharmaceutical composition of any one of embodiments 1 to 43, wherein the CYP450 inhibitor is propyl gallate.
45. The pharmaceutical composition of any one of embodiments 1 to 44, wherein the pharmaceutical composition has a weight ratio of the bile acid compound over the CYP450 inhibitor in a range from about 0.1 to about 50.
46. The pharmaceutical composition of any one of embodiments 1 to 44, wherein the pharmaceutical composition has a weight ratio of the bile acid compound over the CYP450 inhibitor in a range from about 0.5 to about 5.
47. The pharmaceutical composition of any one of embodiments 1 to 46, wherein the one or more pharmaceutically acceptable excipients comprise a disintegrant, filler, lubricant, or glidant, or a mixture of two or more thereof.
48. The pharmaceutical composition of any one of embodiments 1 to 47, comprising a therapeutic polypeptide, a bile acid compound, a CYP450 inhibitor, a disintegrant, a filler, a lubricant, and a glidant.
49. The pharmaceutical composition of embodiment 47 or 48, wherein the pharmaceutical composition comprises from about 1 to about 5% by weight of the disintegrant.
50. The pharmaceutical composition of any one of embodiments 47 to 49, comprising from about 2 to about 5% by weight of the disintegrant.
51. The pharmaceutical composition of any one of embodiments 47 to 50, wherein the disintegrant is crospovidone.
52. The pharmaceutical composition of any one of embodiments 47 to 51, wherein the pharmaceutical composition comprises from about 1 to about 50% by weight of the filler.
53. The pharmaceutical composition of any one of embodiments 47 to 52, wherein the pharmaceutical composition comprises from about 1 to about 35% or from about 5 to about 15% by weight of the filler.
54. The pharmaceutical composition of any one of embodiments 47 to 53, wherein the filler is mannitol.
55. The pharmaceutical composition of any one of embodiments 47 to 54, comprising from about 0.1 to about 5% by weight of the lubricant.
56. The pharmaceutical composition of any one of embodiments 47 to 55, comprising from about 0.5 to about 1% by weight of the lubricant.
57. The pharmaceutical composition of any one of embodiments 47 to 56, wherein the lubricant is magnesium stearate.
58. The pharmaceutical composition of any one of embodiments 47 to 57, comprising from about 0.1 to about 5% by weight of the glidant.
59. The pharmaceutical composition of any one of embodiments 47 to 58, comprising about 0.5 to about 1% by weight of the glidant.
60. The pharmaceutical composition of any one of embodiments 47 to 59, wherein the glidant is silicon dioxide.
61. The pharmaceutical composition of any one of embodiments 1 to 60, further comprising a gastrointestinal enzyme inhibitor.
62. The pharmaceutical composition of embodiment 61, comprising from about 0.2 to about 15% by weight of the gastrointestinal enzyme inhibitor.
63. The pharmaceutical composition of embodiment 61 or 62, comprising from about 1 to about 10% by weight of the gastrointestinal enzyme inhibitor.
64. The pharmaceutical composition of any one of embodiments 61 to 63, wherein the gastrointestinal enzyme inhibitor is a trypsin inhibitor.
65. The pharmaceutical composition of any one of embodiments 61 to 64, wherein the gastrointestinal enzyme inhibitor is soybean trypsin inhibitor.
66. The pharmaceutical composition of any one of embodiments 1 to 65, further comprising an absorption enhancer.
67. The pharmaceutical composition of embodiment 66, comprising from about 5 to about 50% by weight of the absorption enhancer.
68. The pharmaceutical composition of embodiment 66 or 67, comprising from about 15 to about 30% by weight of the absorption enhancer.
69. The pharmaceutical composition of any one of embodiments 66 to 68, wherein the absorption enhancer is sodium caprylate, sodium caprate, sodium *N*-(4-chlorosalicyloyl)-4-aminobutyrate, sodium *N*-(8-[2-hydroxybenzoyl]amino)caprylate, sodium 8-(*N*-2-hydroxy-5-chlorobenzoyl)-aminocaprylate, or salcaprozate sodium.
70. The pharmaceutical composition of any one of embodiments 66 to 69, wherein the absorption enhancer is sodium caprate.
71. The pharmaceutical composition of any one of embodiments 66 to 69, wherein the absorption enhancer is sodium N-(8-[2-hydroxybenzoyl]amino)caprylate.
72. The pharmaceutical composition of any one of embodiments 1, 2, 4 to 33, 35 to 41, 43 to 49, 51, 52, 54, 55, 57, 58, 60 to 62, 64, and 65, comprising from about 0.1 to about 10% by weight of the therapeutic polypeptide, from about 5 to about 65% by weight of the bile acid compound, from about 10 to about 50% by weight of the CYP450 inhibitor, from about 0.2 to about 15% by weight of the gastrointestinal enzyme inhibitor, from about 1 to about 5% by weight of the disintegrant, from about 1 to about 50% by weight of the filler, from about 0.1 to about 5% by weight of the lubricant, and from about 0.1 to about 5% by weight of the glidant.
73. The pharmaceutical composition of any one of embodiments 1, 2, 4 to 33, 35 to 41, 43 to 49, 51, 52, 54, 55, 57, 58, 60 to 62, 64, 65, and 72, comprising from about 0.2 to about 5% by weight of the therapeutic polypeptide, from about 10 to about 65% by weight of the bile acid compound, from about 10 to about 50% by weight of the CYP450 inhibitor, from about 0.5 to about 15% by weight of the gastrointestinal enzyme inhibitor, from about 2 to about 5% by weight of the disintegrant, from about 1 to about 50% by weight of the filler, from about 0.1 to about 2% by weight of the lubricant, and from about 0.1 to about 2% by weight of the glidant.
74. The pharmaceutical composition of any one of embodiments 1, 2, 4 to 33, 35 to 41, 43 to 49, 51, 52, 54, 55, 57, 58, 60 to 62, 64, 65, and 72, comprising from about 0.5 to about 5% by weight of the therapeutic polypeptide, from about 10 to about 50% by weight of the bile acid compound, from about 10 to about 40% by weight of the CYP450 inhibitor, from about 1 to about 15% by weight of the gastrointestinal enzyme inhibitor, from about 2 to about 5% by weight of the disintegrant, from about 1 to about 40% by weight of the filler, from about 0.2 to about 1% by weight of the lubricant, and from about 0.2 to about 1% by weight of the glidant.
75. The pharmaceutical composition of any one of embodiments 1, 2, 4 to 33, 35 to 41, 43 to 49, 51, 52, 54, 55, 57, 58, 60 to 62, 64, 65, and 72, comprising from about 1 to about 2.5% by weight of the therapeutic polypeptide, from about 15 to about 50% by weight of the bile acid compound, from about 15 to about 35% by weight of the CYP450 inhibitor, from about 1 to about 10% by weight of the gastrointestinal enzyme inhibitor, from about 2 to about 5% by weight of the disintegrant, from about 1 to about 35% by weight of the filler, from about 0.5 to about 1% by weight of the lubricant, and from about 0.5 to about 1% by weight of the glidant.
76. The pharmaceutical composition of any one of embodiments 1, 2, 4 to 33, 35 to 41, 43 to 49, 51, 52, 54, 55, 57, 58, 60 to 62, 64, 65, and 72, comprising from about 0.5 to about 5% by weight of the therapeutic polypeptide, from about 30 to about 65% by weight of the bile acid compound, from about 20 to about 40% by weight of the CYP450 inhibitor, from about 0.5 to about 10% by weight of the gastrointestinal enzyme inhibitor, from about 2 to about 5% by weight of the disintegrant, from about 2 to about 30% by weight of the filler, from about 0.2 to about 1% by weight of the lubricant, and from about 0.2 to about 1% by weight of the glidant.
77. The pharmaceutical composition of any one of embodiments 1, 2, 4 to 33, 35 to 41, 43 to 49, 51, 52, 54, 55, 57, 58, 60 to 62, 64, 65, and 72, comprising from about 1 to about 2.5% by weight of the therapeutic polypeptide, from about 40 to about 65% by weight of the bile acid compound, from about 20 to about 35% by weight of the CYP450 inhibitor, from about 0.5 to about 5% by weight of the gastrointestinal enzyme inhibitor, from about 2 to about 5% by weight of the disintegrant, from about 5 to about 15% by weight of the filler, from about 0.5 to about 1% by weight of the lubricant, and from about 0.5 to about 1% by weight of the glidant.
78. The pharmaceutical composition of any one of embodiments 1, 2, 4 to 33, 35 to 41, 43 to 49, 51, 52, 54, 55, 57, 58, 60 to 62, 64, 65, and 72, comprising from about 0.1 to about 10% by weight of the therapeutic polypeptide, from about 5 to about 65% by weight of the bile acid compound, from about 5 to about 50% by weight of the absorption enhancer, from about 10 to about 50% by weight of the CYP450 inhibitor, from about 0.2 to about 15% by weight of the gastrointestinal enzyme inhibitor, from about 1 to about 5% by weight of the disintegrant, from about 1 to about 50% by weight of the filler, from about 0.1 to about 5% by weight of the lubricant, and from about 0.1 to about 5% by weight of the glidant.
79. The pharmaceutical composition of any one of embodiments 1, 2, 4 to 33, 35 to 41, 43 to 49, 51, 52, 54, 55, 57, 58, 60 to 62, 64, 65, and 72, comprising from about 0.2 to about 5% by weight of the therapeutic polypeptide, from about 10 to about 50% by weight of the bile acid compound, from about 10 to about 50% by weight of the absorption enhancer, from about 10 to about 50% by weight of the CYP450 inhibitor, from about 0.5 to about 15% by weight of the gastrointestinal enzyme inhibitor, from about 2 to about 5% by weight of the disintegrant, from about 1 to about 50% by weight of the filler, from about 0.1 to about 2% by weight of the lubricant, and from about 0.1 to about 2% by weight of the glidant.
80. The pharmaceutical composition of any one of embodiments 1, 2, 4 to 33, 35 to 41, 43 to 49, 51, 52, 54, 55, 57, 58, 60 to 62, 64, 65, and 72, comprising from about 0.5 to about 5% by weight of the therapeutic polypeptide, from about 10 to about 50% by weight of the bile acid compound, from about 10 to about 40% by weight of the absorption enhancer, from about 10 to about 40% by weight of the CYP450 inhibitor, from about 1 to about 15% by weight of the gastrointestinal enzyme inhibitor, from about 2 to about 5% by weight of the disintegrant, from about 1 to about 40% by weight of the filler, from about 0.2 to about 1% by weight of the lubricant, and from about 0.2 to about 1% by weight of the glidant.
81. The pharmaceutical composition of any one of embodiments 1, 2, 4 to 33, 35 to 41, 43 to 49, 51, 52, 54, 55, 57, 58, 60 to 62, 64, 65, and 72, comprising from about 1 to about 2.5% by weight of the therapeutic polypeptide, from about 15 to about 50% by weight of the bile acid compound, from about 15 to about 30% by weight of the absorption enhancer, from about 15 to about 30% by weight of the CYP450 inhibitor, from about 1 to about 10% by weight of the gastrointestinal enzyme inhibitor, from about 2 to about 5% by weight of the disintegrant, from about 1 to about 35% by weight of the filler, from about 0.5 to about 1% by weight of the lubricant, and from about 0.5 to about 1% by weight of the glidant.
82. The pharmaceutical composition of embodiment 1, comprising:
   from about 0.2 to about 5% by weight of semaglutide, from about 10 to about 65% by weight of CA, from about 10 to about 50% by weight of PG, from about 0.5 to about 15% by weight of SBTI, from about 2 to about 5% by weight of crospovidone, from about 1 to about 50% by weight of mannitol, from about 0.1 to about 2% by weight of magnesium stearate, and from about 0.1 to about 2% by weight of magnesium stearate;
   from about 0.2 to about 5% by weight of semaglutide, from about 10 to about 65% by weight of DCA, from about 10 to about 50% by weight of PG, from about 0.5 to about 15% by weight of SBTI, from about 2 to about 5% by weight of crospovidone, from about 1 to about 50% by weight of mannitol, from about 0.1 to about 2% by weight of magnesium stearate, and from about 0.1 to about 2% by weight of magnesium stearate;
   from about 0.2 to about 5% by weight of semaglutide, from about 10 to about 65% by weight of GCA, from about 10 to about 50% by weight of PG, from about 0.5 to about 15% by weight of SBTI, from about 2 to about 5% by weight of crospovidone, from about 1 to about 50% by weight of mannitol, from about 0.1 to about 2% by weight of magnesium stearate, and from about 0.1 to about 2% by weight of magnesium stearate;
   from about 0.2 to about 5% by weight of semaglutide, from about 10 to about 50% by weight of GCA, from about 10 to about 50% by weight of C10, from about 10 to about 50% by weight of PG, from about 0.5 to about 15% by weight of SBTI, from about 2 to about 5% by weight of crospovidone, from about 1 to about 50% by weight of mannitol, from about 0.1 to about 2% by weight of magnesium stearate, and from about 0.1 to about 2% by weight of magnesium stearate;
   from about 0.2 to about 5% by weight of semaglutide, from about 10 to about 65% by weight of KGCA, from about 10 to about 50% by weight of PG, from about 0.5 to about 15% by weight of SBTI, from about 2 to about 5% by weight of crospovidone, from about 1 to about 50% by weight of mannitol, from about 0.1 to about 2% by weight of magnesium stearate, and from about 0.1 to about 2% by weight of magnesium stearate;
   from about 0.2 to about 5% by weight of tirzepatide, from about 10 to about 65% by weight of CDCA, from about 10 to about 50% by weight of PG, from about 0.5 to about 15% by weight of SBTI, from about 2 to about 5% by weight of crospovidone, from about 1 to about 50% by weight of mannitol, from about 0.1 to about 2% by weight of magnesium stearate, and from about 0.1 to about 2% by weight of magnesium stearate;
   from about 0.2 to about 5% by weight of tirzepatide, from about 10 to about 65% by weight of GCA, from about 10 to about 50% by weight of PG, from about 0.5 to about 15% by weight of SBTI, from about 2 to about 5% by weight of crospovidone, from about 1 to about 50% by weight of mannitol, from about 0.1 to about 2% by weight of magnesium stearate, and from about 0.1 to about 2% by weight of magnesium stearate; or
   from about 0.2 to about 5% by weight of tirzepatide, from about 10 to about 65% by weight of KGCA, from about 10 to about 50% by weight of PG, from about 0.5 to about 15% by weight of SBTI, from about 2 to about 5% by weight of crospovidone, from about 1 to about 50% by weight of mannitol, from about 0.1 to about 2% by weight of magnesium stearate, and from about 0.1 to about 2% by weight of magnesium stearate.
83. The pharmaceutical composition of any one of embodiments 1 to 82, wherein the pharmaceutical composition provided herein is formulated as a capsule or a tablet.
84. The pharmaceutical composition of any one of embodiments 1 to 83, further comprising an enteric coating.
85. The pharmaceutical composition of any one of embodiments 1 to 84, wherein the pharmaceutical composition has a bioavailability of no less than about 1%.
86. The pharmaceutical composition of any one of embodiments 1 to 85, wherein the pharmaceutical composition has a bioavailability of no less than about 5%.
87. The pharmaceutical composition of any one of embodiments 1 to 86, wherein the pharmaceutical composition has a bioavailability of no less than about 10%.
88. A method of treating diabetes and/or obesity in a subject, comprising administering to the subject in need thereof a therapeutically effective amount of the pharmaceutical composition of any one of embodiments 1 to 87.
89. The method of embodiment 88, wherein the diabetes is type 2 diabetes mellitus.

## Claims

1. An oral pharmaceutical composition comprising a therapeutic polypeptide, a bile acid compound, a CYP450 inhibitor, and one or more pharmaceutically acceptable excipients; wherein the bile acid compound is a compound having the structure of Formula (I): 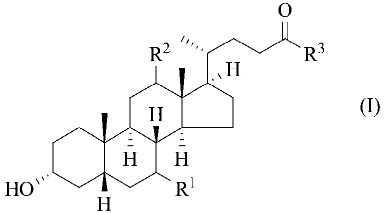 or an enantiomer, a mixture of enantiomers, a diastereomer, a mixture of two or more diastereomers, a tautomer, a mixture of two or more tautomers thereof; or a pharmaceutically acceptable salt, solvate, or hydrate thereof; wherein:
R¹ and R² are each independently hydrogen or -OH;
R³ is -N(H)-X-Z-R⁴, heterocyclyl, or -OH;
R⁴ is C₁₋₆ alkyl, C₁₋₆ heteroalkyl, C₇₋₁₅ aralkyl, heterocyclyl, -OH, or -N(H)R^{4a};
R^{4a} is C₁₋₆ alkyl, C₁₋₆ heteroalkyl, or C₇₋₁₅ aralkyl;
X is a bond, C₁₋₆ alkylene, or C₁₋₆ heteroalkylene; and
Z is a bond, -C(O)-, or -S(O₂)-;
wherein each alkyl, alkylene, heteroalkyl, heteroalkylene, aralkyl, and heterocyclyl is optionally substituted with one or more, in one embodiment, one, two, three, or four, substituents Q, wherein each Q is independently selected from: (a) deuterium, cyano, halo, imino, nitro, and oxo; (b) C₁₋₆ alkyl, C₁₋₆ heteroalkyl, C₂₋₆ alkenyl, C₂₋₆ alkynyl, C₃₋₁₀ cycloalkyl, C₆₋₁₄ aryl, C₇₋₁₅ aralkyl, heteroaryl, and heterocyclyl, each of which is further optionally substituted with one or more, in one embodiment, one, two, three, or four, substituents Q^{a}; and (c) -C(O)R^{a}, -C(O)OR^{a}, -C(O)NR^{b}R^{c}, -C(O)SR^{a}, -C(NR^{a})NR^{b}R^{c}, - C(S)R^{a}, -C(S)OR^{a}, -C(S)NR^{b}R^{c}, -OR^{a}, -OC(O)R^{a}, -OC(O)OR^{a}, -OC(O)NR^{b}R^{c}, -OC(O)SR^{a}, -OC(NR^{a})NR^{b}R^{c}, -OC(S)R^{a}, -OC(S)OR^{a}, -OC(S)NR^{b}R^{c}, -OS(O)R^{a}, -OS(O)₂R^{a}, -OS(O)NR^{b}R^{c}, -OS(O)₂NR^{b}R^{c}, -NR^{b}R^{c}, - NR^{a}C(O)R^{d}, -NR^{a}C(O)OR^{d}, -NR^{a}C(O)NR^{b}R^{c}, -NR^{a}C(O)SR^{d}, -NR^{a}C(NR^{d})NR^{b}R^{c}, -NR^{a}C(S)R^{d}, -NR^{a}C(S)OR^{d}, -NR^{a}C(S)NR^{b}R^{c}, -NR^{a}S(O)R^{d}, -NR^{a}S(O)₂R^{d}, -NR^{a}S(O)NR^{b}R^{c}, -NR^{a}S(O)₂NR^{b}R^{c}, -SR^{a}, -S(O)R^{a}, -S(O)₂R^{a}, -S(O)NR^{b}R^{c}, and -S(O)₂NR^{b}R^{c}, wherein each R^{a}, R^{b}, R^{c}, and R^{d} is independently (i) hydrogen or deuterium; (ii) C₁₋₆ alkyl, C₁₋₆ heteroalkyl, C₂₋₆ alkenyl, C₂₋₆ alkynyl, C₃₋₁₀ cycloalkyl, C₆₋₁₄ aryl, C₇₋₁₅ aralkyl, heteroaryl, or heterocyclyl, each of which is optionally substituted with one or more, in one embodiment, one, two, three, or four, substituents Q^{a}; or (iii) R^{b} and R^{c} together with the N atom to which they are attached form heterocyclyl, optionally substituted with one or more, in one embodiment, one, two, three, or four, substituents Q^{a};
wherein each Q^{a} is independently selected from: (a) deuterium, cyano, halo, nitro, imino, and oxo; (b) C₁₋₆ alkyl, C₂₋₆ alkenyl, C₂₋₆ alkynyl, C₃₋₁₀ cycloalkyl, C₆₋₁₄ aryl, C₇₋₁₅ aralkyl, heteroaryl, and heterocyclyl; and (c) -C(O)R^{e}, -C(O)OR^{e}, -C(O)NR^{f}R^{g}, -C(O)SR^{e}, -C(NR^{e})NR^{f}R^{g}, -C(S)R^{e}, -C(S)OR^{e}, -C(S)NR^{f}R^{g}, -OR^{e}, -OC(O)R^{e}, -OC(O)OR^{e}, -OC(O)NR^{f}R^{g}, -OC(O)SR^{e}, -OC(NR^{e})NR^{f}R^{g}, -OC(S)R^{e}, -OC(S)OR^{e}, -OC(S)NR^{f}R^{g}, -OS(O)R^{e}, -OS(O)₂R^{e}, -OS(O)NR^{f}R^{g}, -OS(O)₂NR^{f}R^{g}, -NR^{f}R^{g}, -NR^{e}C(O)R^{h}, - NR^{e}C(O)OR^{f}, -NR^{e}C(O)NR^{f}R^{g}, -NR^{e}C(O)SR^{f}, -NR^{e}C(NR^{h})NR^{f}R^{g}, -NR^{e}C(S)R^{h}, -NR^{e}C(S)OR^{f}, -NR^{e}C(S)NR^{f}R^{g}, -NR^{e}S(O)R^{h}, -NR^{e}S(O)₂R^{h}, -NR^{e}S(O)NR^{f}R^{g}, -NR^{e}S(O)₂NR^{f}R^{g}, -SR^{e}, -S(O)R^{e}, -S(O)₂R^{e}, -S(O)NR^{f}R^{g}, and -S(O)₂NR^{f}R^{g}; wherein each R^{e}, Rf, R^{g}, and R^{h} is independently (i) hydrogen or deuterium; (ii) C₁₋₆ alkyl, C₂₋₆ alkenyl, C₂₋₆ alkynyl, C₃₋₁₀ cycloalkyl, C₆₋₁₄ aryl, C₇₋₁₅ aralkyl, heteroaryl, or heterocyclyl; or (iii) R^{f} and R^{g} together with the N atom to which they are attached form heterocyclyl; or
wherein the bile acid compound is chenodeoxycholic acid, cholic acid, dehydrocholic acid, deoxycholic acid, glycochenodeoxycholic acid, glycocholic acid, lithocholic acid, taurocholic acid, taurodeoxycholic acid, or ursodeoxycholic acid.

2. The oral pharmaceutical composition of claim 1, wherein
(1) wherein said composition comprises from about 5 to about 65% by weight of the bile acid compound; optionally wherein said composition comprises from about 15 to about 50% or from about 40 to about 65% by weight of the bile acid compound; and/or
(2) R³ is -N(H)-X-Z-R⁴; and/or
(3) X is a bond; or X is C₁₋₆ alkylene, optionally substituted with one or more substituents Q; wherein X is, optionally, methanediyl, ethane-1,1-diyl, propane-1,1-diyl, butane-1,1-diyl, or pentane-1,1-diyl, each optionally substituted with methyl, phenyl, 4-hydroxyphenyl, imidazol-4-yl, indol-3-yl, carboxy, aminocarbonyl, hydroxyl, amino, guanidino, mercapto, or methylthio; or
X is, optionally, methanediyl, ethane-1,1-diyl, 2-phenylethane-1,1-diyl, 2-(4-hydroxyphenyl)ethane-1,1-diyl, 2-imidazol-4-ylethane-1,1-diyl, 2-(indol-3-yl)ethane-1,1-diyl, 2-carboxyethane-1,1-diyl, 2-hydroxyethane-1,1-diyl, 2-aminocarbonylethane-1,1-diyl, 2-mercaptoethane-1,1-diyl, 2-methylpropane-1,1-diyl, 2-carboxypropane-1,1-diyl, 2-hydroxypropane-1,1-diyl, 3-amino-carbonylpropane-1,1-diyl, 3-methylthiopropane-1,1-diyl, 2-methylbutane-1,1-diyl, 3-methyl-butane-1,1-diyl, 4-aminobutane-1,1-diyl, 4-guanidinobutane-1,1-diyl, or 5-aminopentyane-1,1-diyl; and/or
(4) Z is a bond; or Z is -C(O)-; or Z is -S(O₂)-; and/or
(5) R⁴ is C₁₋₆ alkyl or C₇₋₁₅ aralkyl, each optionally substituted with one or more substituents Q.

3. The pharmaceutical composition of claims 1 or 2, wherein
the bile acid compound is a compound having the structure of Formula (VI): 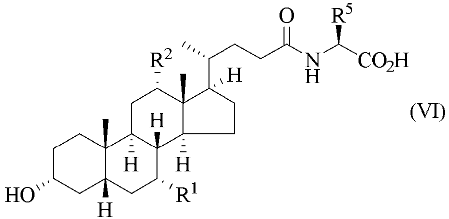 or a diastereomer, a mixture of two or more diastereomers, a tautomer, a mixture of two or more tautomers thereof; or a pharmaceutically acceptable salt, solvate, or hydrate thereof; wherein R⁵ is (i) hydrogen; or (ii) C₁₋₆ alkyl, C₁₋₆ heteroalkyl, or C₇₋₁₅ aralkyl, each optionally substituted with one or more substituents Q; or
the bile acid compound is a compound having the structure of Formula (VIII): 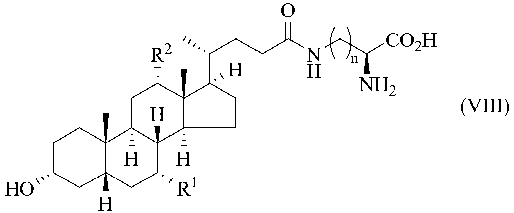 or a diastereomer, a mixture of two or more diastereomers, a tautomer, a mixture of two or more tautomers thereof; or a pharmaceutically acceptable salt, solvate, or hydrate thereof; wherein n is an integer of 1, 2, 3, 4, or 5.

4. The pharmaceutical composition of claim 1 or 2, wherein R⁴ is -N(H)R^{4a}, wherein, optionally, R^{4a} is C₁₋₆ alkyl or C₇₋₁₅ aralkyl, each optionally substituted with one or more substituents Q.

5. The pharmaceutical composition of any one of claims 1, 2 and 4, wherein
the bile acid compound is a compound having the structure of Formula (X): 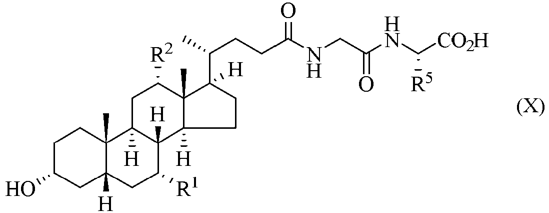 or a diastereomer, a mixture of two or more diastereomers, a tautomer, a mixture of two or more tautomers thereof; or a pharmaceutically acceptable salt, solvate, or hydrate thereof; wherein R⁵ is (i) hydrogen; or (ii) C₁₋₆ alkyl, C₁₋₆ heteroalkyl, C₁₋₆ heteroalkyl, or C₇₋₁₅ aralkyl, each optionally substituted with one or more substituents Q; or
the bile acid compound is a compound having the structure of Formula (XII): 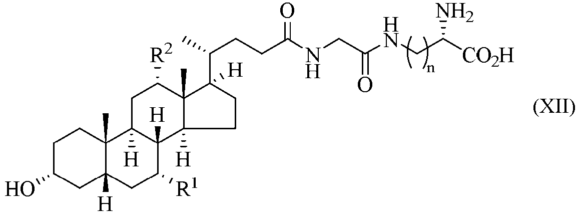 or a diastereomer, a mixture of two or more diastereomers, a tautomer, a mixture of two or more tautomers thereof; or a pharmaceutically acceptable salt, solvate, or hydrate thereof;
wherein n is an integer of 1, 2, 3, 4, or 5.

6. The pharmaceutical composition of any one of claims 1 to 5, wherein
(1) R¹ is hydrogen or -OH; and/or
(2) R² is hydrogen or -OH.

7. The pharmaceutical composition of any one of claims 1 to 6, wherein
(1) R⁵ is (i) hydrogen; or (ii) C₁₋₆ alkyl or C₇₋₁₅ aralkyl, each optionally substituted with heteroaryl, -C(O)OR^{a}, -C(O)NR^{b}R^{c}, -OR^{a}, -NR^{b}R^{c}, -NR^{a}C(NR^{d})NR^{b}R^{c}, or -SR^{a}; or
wherein R⁵ is (i) hydrogen; or (ii) methyl, ethyl, propyl, butyl, or benzyl, each optionally substituted with imidazolyl, indolyl, carboxy, aminocarbonyl, hydroxyl, amino, guanidino, mercapto, or methylthio; or
wherein R⁵ is hydrogen, methyl, isopropyl, isobutyl, but-2-yl, benzyl, 4-hydroxybenzyl, imidazol-4-ylmethyl, indol-3-ylmethyl, carboxymethyl, 2-carboxyethyl, aminocarbonylmethyl, 2-aminocarbonylethyl, hydroxymethyl, 1-hydroxyethyl, 3-aminopropyl, 4-aminobutyl, 3-guanidinopropyl, mercaptomethyl, or 2-methylthioethyl; or
(2) n is an integer of 3, 4, or 5, optionally, n is an integer of 4.

8. The pharmaceutical composition of any one of claims 1 to 7, wherein
(1) the bile acid compound is:
*N*⁶-(((*R*)-4-((3*R*,5*S*,7*R*,8*R*,9*S*,10*S*,12*S*,13*R*,14*S*,17*R*)-3,7,12-trihydroxy-10,13-dimethyl-hexadecahydro-1*H*-cyclopenta[*a*]phenanthren-17-yl)pentanoyl)glycyl)-L-lysine;
*N*⁶-((*R*)-4-((3*R*,5*S*,7*R*,8*R*,9*S*,10*S*,13*R*,14*S*,17*R*)-3,7-dihydroxy-10,13-dimethylhexadeca-hydro-1*H*-cyclopenta[a]phenanthren-17-yl)pentanoyl)-L-lysine;
*N*⁶*-*((*R*)-4-((3*R*,5*S*,7*S*,8*R*,9*S*,10*S*,13*R*,14*S*,17*R*)-3,7-dihydroxy-10,13-dimethylhexadeca-hydro-1*H*-cyclopenta[a]phenanthren-17-yl)pentanoyl)-L-lysine;
*N*⁶-((*R*)-4-((3*R*,5*R*,8*R*,9*S*,10*S*,12*S*,13*R*,14*S*,17*R*)-3,12-dihydroxy-10,13-dimethylhexadecahydro-1*H*-cyclopenta[*a*]phenanthren-17-yl)pentanoyl)-L-lysine;
*N*⁶-((*R*)-4-((3*R*,5*S*,7*R*,8*R*,9*S*,10*S*,12*S*,13*R*,14*S*,17*R*)-3,7,12-trihydroxy-10,13-dimethylhexadecahydro-1*H*-cyclopenta[a]phenanthren-17-yl)pentanoyl)-L-lysine;
*N*⁶-(((*R*)-4-((3*R*,5*S*,7*R*,8*R*,9*S*,10*S*,12*S*,13*R*,14*S*,17*R*)-3,7,12-trihydroxy-10,13-dimethyl-hexadecahydro-1*H*-cyclopenta[a]phenanthren-17-yl)pentanoyl)glycyl)-L-arginine;
*N*⁶-(((*R*)-4-((3*R*,5*S*,7*R*,8*R*,9*S*,10*S*,12*S*,13*R*,14*S*,17*R*)-3,7,12-trihydroxy-10,13-dimethyl-hexadecahydro-1*H*-cyclopenta[a]phenanthren-17-yl)pentanoyl)glycyl)-L-histidine; or
*N*⁶-(((*R*)-4-((3*R*,5*S*,7*R*,8*R*,9*S*,10*S*,12*S*,13*R*,14*S*,17*R*)-3,7,12-trihydroxy-10,13-dimethyl-hexadecahydro-1*H*-cyclopenta[a]phenanthren-17-yl)pentanoyl)glycyl)-L-aspartic acid;
or a diastereomer, a mixture of two or more diastereomers, a tautomer, a mixture of two or more tautomers thereof; or a pharmaceutically acceptable salt, solvate, or hydrate thereof, optionally, the bile acid compound is:
*N*⁶-(((*R*)-4-((3*R*,5*S*,7*R*,8*R*,9*S*,10*S*,12*S*,13*R*,14*S*,17*R*)-3,7,12-trihydroxy-10,13-dimethyl-hexadecahydro-1*H*-cyclopenta[a]phenanthren-17-yl)pentanoyl)glycyl)-L-lysine;
or a diastereomer, a mixture of two or more diastereomers, a tautomer, a mixture of two or more tautomers thereof; or a pharmaceutically acceptable salt, solvate, or hydrate thereof, or
(2) the bile acid compound is chenodeoxycholic acid, cholic acid, dehydrocholic acid, deoxycholic acid, glycol-chenodeoxycholic acid, glycocholic acid, lithocholic acid, taurocholic acid, taurodeoxycholic acid, or ursodeoxycholic acid, optionally,
the bile acid compound is deoxycholic acid; or the bile acid compound is glycocholic acid.

9. The pharmaceutical composition of any one of claims 1 to 8, comprising from about 0.1 to about 10% by weight of the therapeutic polypeptide, optionally, comprising from about 1 to about 2.5% by weight of the therapeutic polypeptide.

10. The pharmaceutical composition of any one of claims 1 to 9, wherein
(1) the therapeutic peptide is a glucagon-like peptide-1 receptor agonist, a κ-opioid receptor agonist, or an insulin receptor agonist, and/or
(2) the therapeutic peptide is a glucagon-like peptide-1 receptor agonist; and/or
(3) the therapeutic peptide is beinaglutide, exenatide, liraglutide, lixisenatide, mazdutide, PEG-loxenatide, PEGylated exenatide, retatrutide, semaglutide, or tirzepatide; and/or
(4) the therapeutic peptide is a lipidated peptide; and/or
(5) the therapeutic peptide is semaglutide or tirzepatide.

11. The pharmaceutical composition of any one of claims 1 to 10, comprising from about 10 to about 50% by weight of the CYP450 inhibitor, optionally, comprising from about 15 to about 35% or from about 20 to about 35% by weight of the CYP450 inhibitor.

12. The pharmaceutical composition of any one of claims 1 to 11, wherein
(1) the CYP450 inhibitor is a gallate ester; and/or
(2) the CYP450 inhibitor is propyl gallate; and/or
(3) the pharmaceutical composition has a weight ratio of the bile acid compound over the CYP450 inhibitor in a range from about 0.1 to about 50, optionally,
the pharmaceutical composition has a weight ratio of the bile acid compound over the CYP450 inhibitor in a range from about 0.5 to about 5; and/or
(4) the one or more pharmaceutically acceptable excipients comprise a disintegrant, filler, lubricant, or glidant, or a mixture of two or more thereof; and/or
(5) the pharmaceutical composition comprises a therapeutic polypeptide, a bile acid compound, a CYP450 inhibitor, a disintegrant, a filler, a lubricant, and a glidant.

13. The pharmaceutical composition of claim 12(4) or (5), wherein
(1) the pharmaceutical composition comprises from about 1 to about 5% by weight of the disintegrant, optionally, from about 2 to about 5% by weight of the disintegrant; and/or
(2) the disintegrant is crospovidone; and/or
(3) the pharmaceutical composition comprises from about 1 to about 50% by weight of the filler, optionally, the pharmaceutical composition comprises from about 1 to about 35% or from about 5 to about 15% by weight of the filler; and/or
(4) the filler is mannitol; and/or
(5) the pharmaceutical composition comprises from about 0.1 to about 5% by weight of the lubricant; and/or
(6) the pharmaceutical composition comprises from about 0.5 to about 1% by weight of the lubricant; and/or
(7) the lubricant is magnesium stearate; and/or
(8) the pharmaceutical composition comprises from about 0.1 to about 5% by weight of the glidant, optionally, about 0.5 to about 1% by weight of the glidant; and/or
(9) the glidant is silicon dioxide.

14. The pharmaceutical composition of any one of claims 1 to 13, further comprising
(1) a gastrointestinal enzyme inhibitor, optionally, comprising from about 0.2 to about 15% by weight of the gastrointestinal enzyme inhibitor, or comprising from about 1 to about 10% by weight of the gastrointestinal enzyme inhibitor; wherein the gastrointestinal enzyme inhibitor is, optionally, a trypsin inhibitor or soybean trypsin inhibitor; and/or
(2) an absorption enhancer, optionally, comprising from about 5 to about 50% by weight of the absorption enhancer, or comprising from about 15 to about 30% by weight of the absorption enhancer, wherein the absorption enhancer is, optionally, sodium caprylate, sodium caprate, sodium *N*-(4-chlorosalicyloyl)-4-aminobutyrate, sodium *N*-(8-[2-hydroxybenzoyl]amino)caprylate, sodium 8-(*N*-2-hydroxy-5-chlorobenzoyl)-aminocaprylate, or salcaprozate sodium, or the absorption enhancer is, optionally, sodium caprate, or the absorption enhancer is, optionally, sodium N-(8-[2-hydroxybenzoyl]amino)caprylate.

15. The pharmaceutical composition of any one of claims 1 to 14, comprising
(1) from about 0.1 to about 10% by weight of the therapeutic polypeptide, from about 5 to about 65% by weight of the bile acid compound, from about 10 to about 50% by weight of the CYP450 inhibitor, from about 0.2 to about 15% by weight of the gastrointestinal enzyme inhibitor, from about 1 to about 5% by weight of the disintegrant, from about 1 to about 50% by weight of the filler, from about 0.1 to about 5% by weight of the lubricant, and from about 0.1 to about 5% by weight of the glidant; or
(2) from about 0.2 to about 5% by weight of the therapeutic polypeptide, from about 10 to about 65% by weight of the bile acid compound, from about 10 to about 50% by weight of the CYP450 inhibitor, from about 0.5 to about 15% by weight of the gastrointestinal enzyme inhibitor, from about 2 to about 5% by weight of the disintegrant, from about 1 to about 50% by weight of the filler, from about 0.1 to about 2% by weight of the lubricant, and from about 0.1 to about 2% by weight of the glidant; or
(3) from about 0.5 to about 5% by weight of the therapeutic polypeptide, from about 10 to about 50% by weight of the bile acid compound, from about 10 to about 40% by weight of the CYP450 inhibitor, from about 1 to about 15% by weight of the gastrointestinal enzyme inhibitor, from about 2 to about 5% by weight of the disintegrant, from about 1 to about 40% by weight of the filler, from about 0.2 to about 1% by weight of the lubricant, and from about 0.2 to about 1% by weight of the glidant; or
(4) from about 1 to about 2.5% by weight of the therapeutic polypeptide, from about 15 to about 50% by weight of the bile acid compound, from about 15 to about 35% by weight of the CYP450 inhibitor, from about 1 to about 10% by weight of the gastrointestinal enzyme inhibitor, from about 2 to about 5% by weight of the disintegrant, from about 1 to about 35% by weight of the filler, from about 0.5 to about 1% by weight of the lubricant, and from about 0.5 to about 1% by weight of the glidant; or
(5) from about 0.5 to about 5% by weight of the therapeutic polypeptide, from about 30 to about 65% by weight of the bile acid compound, from about 20 to about 40% by weight of the CYP450 inhibitor, from about 0.5 to about 10% by weight of the gastrointestinal enzyme inhibitor, from about 2 to about 5% by weight of the disintegrant, from about 2 to about 30% by weight of the filler, from about 0.2 to about 1% by weight of the lubricant, and from about 0.2 to about 1% by weight of the glidant; or
(6) from about 1 to about 2.5% by weight of the therapeutic polypeptide, from about 40 to about 65% by weight of the bile acid compound, from about 20 to about 35% by weight of the CYP450 inhibitor, from about 0.5 to about 5% by weight of the gastrointestinal enzyme inhibitor, from about 2 to about 5% by weight of the disintegrant, from about 5 to about 15% by weight of the filler, from about 0.5 to about 1% by weight of the lubricant, and from about 0.5 to about 1% by weight of the glidant; or
(7) from about 0.1 to about 10% by weight of the therapeutic polypeptide, from about 5 to about 65% by weight of the bile acid compound, from about 5 to about 50% by weight of the absorption enhancer, from about 10 to about 50% by weight of the CYP450 inhibitor, from about 0.2 to about 15% by weight of the gastrointestinal enzyme inhibitor, from about 1 to about 5% by weight of the disintegrant, from about 1 to about 50% by weight of the filler, from about 0.1 to about 5% by weight of the lubricant, and from about 0.1 to about 5% by weight of the glidant; or
(8) from about 0.2 to about 5% by weight of the therapeutic polypeptide, from about 10 to about 50% by weight of the bile acid compound, from about 10 to about 50% by weight of the absorption enhancer, from about 10 to about 50% by weight of the CYP450 inhibitor, from about 0.5 to about 15% by weight of the gastrointestinal enzyme inhibitor, from about 2 to about 5% by weight of the disintegrant, from about 1 to about 50% by weight of the filler, from about 0.1 to about 2% by weight of the lubricant, and from about 0.1 to about 2% by weight of the glidant; or
(9) from about 0.5 to about 5% by weight of the therapeutic polypeptide, from about 10 to about 50% by weight of the bile acid compound, from about 10 to about 40% by weight of the absorption enhancer, from about 10 to about 40% by weight of the CYP450 inhibitor, from about 1 to about 15% by weight of the gastrointestinal enzyme inhibitor, from about 2 to about 5% by weight of the disintegrant, from about 1 to about 40% by weight of the filler, from about 0.2 to about 1% by weight of the lubricant, and from about 0.2 to about 1% by weight of the glidant; or
(10) from about 1 to about 2.5% by weight of the therapeutic polypeptide, from about 15 to about 50% by weight of the bile acid compound, from about 15 to about 30% by weight of the absorption enhancer, from about 15 to about 30% by weight of the CYP450 inhibitor, from about 1 to about 10% by weight of the gastrointestinal enzyme inhibitor, from about 2 to about 5% by weight of the disintegrant, from about 1 to about 35% by weight of the filler, from about 0.5 to about 1% by weight of the lubricant, and from about 0.5 to about 1% by weight of the glidant; or
(11) from about 0.2 to about 5% by weight of semaglutide, from about 10 to about 65% by weight of CA, from about 10 to about 50% by weight of PG, from about 0.5 to about 15% by weight of SBTI, from about 2 to about 5% by weight of crospovidone, from about 1 to about 50% by weight of mannitol, from about 0.1 to about 2% by weight of magnesium stearate, and from about 0.1 to about 2% by weight of magnesium stearate; or
(12) from about 0.2 to about 5% by weight of semaglutide, from about 10 to about 65% by weight of DCA, from about 10 to about 50% by weight of PG, from about 0.5 to about 15% by weight of SBTI, from about 2 to about 5% by weight of crospovidone, from about 1 to about 50% by weight of mannitol, from about 0.1 to about 2% by weight of magnesium stearate, and from about 0.1 to about 2% by weight of magnesium stearate; or
(13) from about 0.2 to about 5% by weight of semaglutide, from about 10 to about 65% by weight of GCA, from about 10 to about 50% by weight of PG, from about 0.5 to about 15% by weight of SBTI, from about 2 to about 5% by weight of crospovidone, from about 1 to about 50% by weight of mannitol, from about 0.1 to about 2% by weight of magnesium stearate, and from about 0.1 to about 2% by weight of magnesium stearate; or
(14) from about 0.2 to about 5% by weight of semaglutide, from about 10 to about 50% by weight of GCA, from about 10 to about 50% by weight of C10, from about 10 to about 50% by weight of PG, from about 0.5 to about 15% by weight of SBTI, from about 2 to about 5% by weight of crospovidone, from about 1 to about 50% by weight of mannitol, from about 0.1 to about 2% by weight of magnesium stearate, and from about 0.1 to about 2% by weight of magnesium stearate; or
(15) from about 0.2 to about 5% by weight of semaglutide, from about 10 to about 65% by weight of KGCA, from about 10 to about 50% by weight of PG, from about 0.5 to about 15% by weight of SBTI, from about 2 to about 5% by weight of crospovidone, from about 1 to about 50% by weight of mannitol, from about 0.1 to about 2% by weight of magnesium stearate, and from about 0.1 to about 2% by weight of magnesium stearate; or
(16) from about 0.2 to about 5% by weight of tirzepatide, from about 10 to about 65% by weight of CDCA, from about 10 to about 50% by weight of PG, from about 0.5 to about 15% by weight of SBTI, from about 2 to about 5% by weight of crospovidone, from about 1 to about 50% by weight of mannitol, from about 0.1 to about 2% by weight of magnesium stearate, and from about 0.1 to about 2% by weight of magnesium stearate; or
(17) from about 0.2 to about 5% by weight of tirzepatide, from about 10 to about 65% by weight of GCA, from about 10 to about 50% by weight of PG, from about 0.5 to about 15% by weight of SBTI, from about 2 to about 5% by weight of crospovidone, from about 1 to about 50% by weight of mannitol, from about 0.1 to about 2% by weight of magnesium stearate, and from about 0.1 to about 2% by weight of magnesium stearate; or
(18) from about 0.2 to about 5% by weight of tirzepatide, from about 10 to about 65% by weight of KGCA, from about 10 to about 50% by weight of PG, from about 0.5 to about 15% by weight of SBTI, from about 2 to about 5% by weight of crospovidone, from about 1 to about 50% by weight of mannitol, from about 0.1 to about 2% by weight of magnesium stearate, and from about 0.1 to about 2% by weight of magnesium stearate.

16. The pharmaceutical composition of any one of claims 1 to 15, wherein
(1) the pharmaceutical composition provided herein is formulated as a capsule or a tablet; and/or
(2) the pharmaceutical composition further comprises an enteric coating; and/or
(3) the pharmaceutical composition has a bioavailability of no less than about 1%, or a bioavailability of no less than about 5% or a bioavailability of no less than about 10%.

17. The pharmaceutical composition of any one of claims 1 to 16 for use in a method of treating diabetes and/or obesity in a subject, said method comprising administering to the subject in need thereof a therapeutically effective amount of said pharmaceutical composition, wherein the diabetes is, optionally, type 2 diabetes mellitus.
